(19) Europäisches Patentamt
European Patent Office
Office européen des brevets

(11) Publication number : **0 562 832 A1**

# (12) EUROPEAN PATENT APPLICATION

(21) Application number : **93302220.4**

(22) Date of filing : **23.03.93**

(51) Int. Cl.$^5$ : **C07D 209/12,** C07D 409/14, C07D 401/06, C07D 231/56, A61K 31/445, A61K 31/40, A61K 31/41

(30) Priority : **23.03.92 JP 65323/92**
**22.01.93 JP 9156/93**

(43) Date of publication of application :
**29.09.93 Bulletin 93/39**

(84) Designated Contracting States :
**AT BE CH DE DK ES FR GB GR IE IT LI LU MC NL PT SE**

(71) Applicant : **SANKYO COMPANY LIMITED**
**5-1 Nihonbashi Honcho 3-chome**
**Chuo-ku, Tokyo 103 (JP)**

(72) Inventor : **Naruto, Shunji, c/o Sankyo Company Limited**
**2-58, Hiromachi 1-chome**
**Shinagawa-ku, Tokyo 140 (JP)**
Inventor : **Sugano, Yuichi, c/o Sankyo Company Limited**
**2-58, Hiromachi 1-chome**
**Shinagawa-ku, Tokyo 140 (JP)**
Inventor : **Matsui, Yoshiki, c/o Sankyo Company Limited**
**2-58, Hiromachi 1-chome**
**Shinagawa-ku, Tokyo 140 (JP)**
Inventor : **Tonohiro, Toshiyuki, c/o Sankyo Company Limited**
**2-58, Hiromachi 1-chome**
**Shinagawa-ku, Tokyo 140 (JP)**
Inventor : **Kaneko, Tsugio, c/o Sankyo Company Limited**
**2-58, Hiromachi 1-chome**
**Shinagawa-ku, Tokyo 140 (JP)**
Inventor : **Iwata, Nobuyoshi, c/o Sankyo Company Limited**
**2-58, Hiromachi 1-chome**
**Shinagawa-ku, Tokyo 140 (JP)**

(74) Representative : **Gibson, Christian John Robert et al**
**MARKS & CLERK 57/60 Lincoln's Inn Fields**
**London WC2A 3LS (GB)**

(54) **Indole and indazole derivatives, for the treatment and prophylaxis of cerebral disorders, their preparation and their use.**

(57) Compounds of formula (I) :

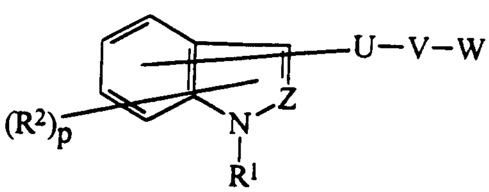

(I)

[in which $R^1$ and $R^2$ are each hydrogen or various organic groups, p is 0, 1, 2 or 3, U is -CO- or -CH($OR^3$)- where $R^3$ is hydrogen or a hydroxy-protecting group, V is an optionally unsaturated aliphatic hydrocarbon group and W is a nitrogen-containing group] are useful in the treatment and prophylaxis of dementia especially of the Alzheimer's type.

EP 0 562 832 A1

The present invention relates to a series of new indole and indazole derivatives which have the ability to inhibit the activity of acetylcholinesterase, and which may therefore be used to improve cerebral function, in particular for the treatment and prophylaxis of cerebral disorders, such as senile dementia or dementia of the Alzheimer type. The invention also provides methods and compositions using these compounds as well as processes for their preparation.

Dementia, both true senile dementia and syndromes resembling it, such as Alzheimer's disease and dementia resulting from cerebral injuries, is an increasing problem in the modern world. Alzheimer's disease is characterised by such morphological changes as senile plague and changes to nerve fibrils, and degeneration of many nervous cells and/or impairment of their function have been reported. In particular, degeneration and impairment of the function of the acetylcholine nervous system have much been studied, and this degeneration and impairment of function are considered to be the reason for the decreases in memory and learning ability experienced by Alzheimer's patients. Accordingly, it is clear that one approach to the development of a treatment or prophylaxis of dementia of Alzheimer type is to find a means of activating the impaired function of the acetylcholine nervous system. Potential methods of achieving this include:

1) administration of an acetylcholine precursor, thereby raising the level of acetylcholine in the patient;
2) administration of an inhibitor of acetylcholinesterase, which is an enzyme capable of decomposing acetylcholine, thereby preventing its decomposition and thus raising its level; and
3) administration of a receptor agonist which directly stimulates acetylcholine receptors.

Of these, the most promising currently appears to be the second, and therapeutic studies of the use of acetylcholinesterase inhibitors against Alzheimer's disease are being clinically carried out.

A number of compounds has been found to have the ability to inhibit the activity of acetylcholinesterase, although there does not appear to be any general theory of the structure/activity relationship, and the compounds proposed for this use have a variety of different structures. So far as we are presently aware, none of the prior art compounds proposed for use in the treatment of dementia of the senile dementia type are structurally related to the compounds of the present invention. Examples of such prior art include the tricyclic amines of European Patent Publication No. 441 517, and the cyclic amine derivatives of European Patent Publication No. 378 207, Japanese Patent Application Kokai No. Sho. 64-79151 and Japanese Patent Application Kokai No. Hei. No. 2-169569.

There is, however, still a need for more drugs for the treatment or prevention of these distressing disorders.

The compounds of the present invention are those compounds of formula (I):

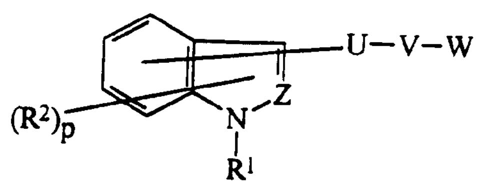

(I)

wherein:

Z represents a group of formula -N= or -CH= or a group of formula -CH= in which the hydrogen atom is replaced by the group -U-V-W shown in formula (I);

p is 0, 1, 2 or 3;

$R^1$ represents a hydrogen atom or any of the groups or atoms represented by $R^2$;

$R^2$ represents:

a hydrogen atom;

any of the groups and atom represented by $R^a$;

an aryl group, as defined below;

an aralkyl group, as defined below;

an aralkyloxycarbonyl group, in which the aralkyl part is as defined below;

an arylamino group, in which the aryl part is as defined below;

an arylaminoalkyl group, in which the aryl part is as defined below, and the alkyl part has from 1 to 6 carbon atoms;

a heterocyclic group having from 3 to 8 ring atoms of which from 1 to 4 are hetero-atoms selected from nitrogen, oxygen and sulphur hetero-atoms, said group being unsubstituted or being substituted by at least one substituent selected from the groups and atoms represented by $R^a$;

an alkyl group which has from 1 to 6 carbon atoms and which is substituted by a single heterocyclic group, said heterocyclic group having from 3 to 8 ring atoms of which from 1 to 4 are hetero-atoms selected from nitrogen, oxygen and sulphur hetero-atoms, and being unsubstituted or being substituted by at least one substituent selected from the groups and atoms represented by $R^a$; or

an alkylamino group which has from 1 to 6 carbon atoms and which is substituted by a single heterocyclic group, said heterocyclic group having from 3 to 8 ring atoms of which from 1 to 4 are hetero-atoms selected from nitrogen, oxygen and sulphur hetero-atoms, and being unsubstituted or being substituted by at least one substituent selected from the groups and atoms represented by $R^a$;

U represents a group of formula -CO- or $-CH(OR^3)-$, where $R^3$ represents a hydrogen atom or a hydroxy-protecting group;

V represents a group of formula

$$-(CR^e{=}CR^f)_m-(CR^gR^h)_n-$$

where $m$ is 0, 1 or 2, $n$ is 0 or an integer from 1 to 7, provided that $(m + n) > 0$ and $R^e$, $R^f$, $R^g$ and $R^h$ are independently selected from hydrogen atoms and alkyl groups having from 1 to 4 carbon atoms;

W represents:

a heterocyclic group having from 3 to 14 ring atoms, of which one is a nitrogen atom through which the heterocyclic group is attached to the group represented by V, from 1 to 3 are additional hetero-atoms selected from nitrogen, oxygen and sulphur hetero-atoms, and the remainder are carbon atoms, said group being unsubstituted or being substituted by at least one substituent selected from oxygen atoms and the groups and atoms represented by $R^a$;

a group of formula (II):

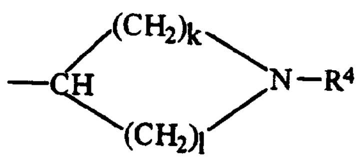

(II)

or

a group of formula (II) in which the ring is condensed with one or two benzene rings

or

a group of formula:

$$-(Ar)_j-(CH_2)_i-NR^4R^5 \qquad (III)$$

where

$k$ and $l$ are independently selected from 0 and integers from 1 to 4, provided that $(k + l)$ is at least 1;.

$i$ is 0 or an integer from 1 to 4;

$j$ is 0 or 1;

Ar represents an aryl group as defined below or an aromatic heterocyclic group which has from 5 to 7 ring atom, of which from 1 to 3 are hetero-atoms, from 0 to 3 of said hetero-atoms being nitrogen atoms and 0 or 1 of said hetero-atoms being selected from oxygen and sulphur atom, said aromatic heterocyclic group being unsubstituted
or being substituted by at least one substituent selected from the groups and atoms represented by $R^a$;

$R^4$ and $R^5$ are independently selected from:

hydrogen atoms;

the groups and atom represented by $R^a$;

aryl groups, as defined below;

aralkyl groups, as defined below;

arylcarbonyl groups, in which the aryl part is as defined below; and

heterocyclic groups having from 3 to 8 ring atom of which from 1 to 4 are hetero-atoms selected from nitrogen, oxygen and sulphur hetero-atoms, said group being unsubstituted or being substituted by at least one substituent selected from the groups and.atoms represented by $R^a$;

wherein aryl groups are carbocyclic aromatic groups having from 6 to 14 ring carbon atoms and being unsubstituted or being substituted by at least one substituent selected from the groups and atoms represented by $R^a$;

wherein aralkyl group is an alkyl group having from 1 to 6 carbon atoms which group is substituted by from 1 to 3 aryl groups as defined above;

$R^a$ represents:

an alkyl group having from 1 to 6 carbon atoms;

an aryl group which is a carbocyclic aromatic group having from 6 to 14 ring carbon atoms and being unsubstituted or being substituted by at least one substituent selected from the groups and atoms represented by $R^c$;

a cycloalkyl group having from 3 to 14 ring carbon atoms in one or more rings;

a cycloalkylalkyl group in which the cycloalkyl part has from 3 to 14 ring carbon atoms in one or more rings and the alkyl part has from 1 to 4 carbon atoms;

a heterocyclic group having from 3 to 14 ring atoms of which from 1 to 4 are hetero-atoms selected from nitrogen, oxygen and sulphur hetero-atoms, said group being unsubstituted or being substituted by at least one substituent selected from the groups and atoms represented by $R^b$;

an aralkyl group in which the alkyl part has from 1 to 6 carbon atoms and the aryl part is a carbocyclic aromatic group having from 6 to 14 ring carbon atoms and being unsubstituted or being substituted by at least one substituent selected from the groups and atoms represented by $R^c$;

a halogen atom; an amino group;

an alkylamino group in which the alkyl part has from 1 to 6 carbon atoms;

a dialkylamino group in which each alkyl part is independently selected from alkyl groups having from 1 to 6 carbon atoms;

a mono- or di- arylamino group in which the or each aryl part is a carbocyclic aromatic group having from 6 to 14 ring carbon atom and being unsubstituted or substituted by at least one substituent selected from the groups and atoms represented by $R^c$;

an aminoalkyl group in which the alkyl part has from 1 to 6 carbon atoms and in which the amino part is unsubstituted or is substituted by a single acyl group represented by $R^d$;

an alkylaminoalkyl group in which each alkyl part is independently selected from alkyl groups having from 1 to 6 carbon atoms;

an alkynylaminoalkyl group in which the alkyl part has from 1 to 6 carbon atoms and the alkynyl part has from 2 to 6 carbon atoms;

a nitro group;

a cyano group;

a sulphonyl group;

an alkylsulphonyl group in which the alkyl part has from 1 to 6 carbon atoms;

a haloalkylsulphonyl group in which the alkyl part has from 1 to 6 carbon atoms;

an alkanoyl group having from 1 to 6 carbon atoms;

an arylcarbonyl group in which the aryl part is a carbocyclic aromatic group having from 6 to 14 ring carbon atoms and being unsubstituted or substituted by at least one substituent selected from the groups and atom represented by $R^c$;

an arylalkanoyl group in which the aryl part is a carbocyclic aromatic group having from 6 to 14 ring carbon atom and being unsubstituted or substituted by at least one substituent selected from the groups and atoms represented by $R^c$ and the alkanoyl part has from 2 to 6 carbon atoms;

an alkoxy group having from 1 to 6 carbon atoms;

an alkoxycarbonyl group having from 2 to 7 carbon atoms;

a haloalkyl group having from 1 to 6 carbon atoms; and

in the case of groups attached to nitrogen atoms, a cyanoamino group;

$R^b$ represents:

an alkyl group having from 1 to 6 carbon atoms;

an aryl group which is a carbocyclic aromatic group having from 6 to 14 ring carbon atoms and being unsubstituted or being substituted by at least one substituent selected from the groups and atoms represented by $R^c$;

an aralkyl group in which the alkyl part has from 1 to 6 carbon atom and the aryl part is a carbocyclic aromatic group having from 6 to 14 ring carbon atoms and being unsubstituted or being substituted by at least one substituent selected from the groups and atoms represented by $R^c$;

a halogen atom;

an amino group;

an alkylamino group in which the alkyl part has from 1 to 6 carbon atoms;

a dialkylamino group in which each alkyl part is independently selected from alkyl groups having from 1 to 6 carbon atoms;

an arylamino group in which the aryl part is a carbocyclic aromatic group having from 6 to 14 ring carbon atoms and being unsubstituted or substituted by at least one substituent selected from the groups and atoms represented by $R^c$;

an aminoalkyl group in which the alkyl part has from 1 to 6 carbon atoms;

an alkylaminoalkyl group in which each alkyl part is independently selected from alkyl groups having from 1 to 6 carbon atoms;

a nitro group;

a cyano group;

an alkanoyl group having from 1 to 6 carbon atoms;

an arylcarbonyl group in which the aryl part is a carbocyclic aromatic group having from 6 to 14 ring carbon atoms and being unsubstituted or substituted by at least one substituent selected from the groups and atoms represented by $R^c$;

an arylalkanoyl group in which the aryl part is a carbocyclic aromatic group having from 6 to 14 ring carbon atom and being unsubstituted or substituted by at least one substituent selected from the groups and atom represented by $R^c$;

an alkoxy group having from 1 to 6 carbon atoms;

an alkoxycarbonyl group having from 2 to 7 carbon atoms;

a haloalkyl group having from 1 to 6 carbon atoms;

$R^c$ represents:

an alkyl group having from 1 to 6 carbon atoms;

an aryl group which is an unsubstituted carbocyclic aromatic group having from 6 to 10 ring carbon atoms;

an aralkyl group in which the alkyl part has from 1 to 6 carbon atoms and the aryl part is an unsubstituted carbocyclic aromatic group having from 6 to 10 ring carbon atoms;

a halogen atom;

an amino group;

an alkylamino group in which the alkyl part has from 1 to 6 carbon atoms;

a dialkylamino group in which each alkyl part is independently selected from alkyl groups having from 1 to 6 carbon atoms;

an arylamino group in which the aryl part is an unsubstituted carbocyclic aromatic group having from 6 to 10 ring carbon atoms;

an aminoalkyl group in which the alkyl part has from 1 to 6 carbon atoms;

an alkylaminoalkyl group in which each alkyl part is independently selected from alkyl groups having from 1 to 6 carbon atoms;

a nitro group;

a cyano group;

an alkanoyl group having from 1 to 6 carbon atoms;

an arylcarbonyl group in which the aryl part is an unsubstituted carbocyclic aromatic group having from 6 to 10 ring carbon atoms;

an alkoxy group having from 1 to 6 carbon atoms;

an alkoxycarbonyl group having from 2 to 7 carbon atoms;

a haloalkyl group having from 1 to 6 carbon atoms;

$R^d$ represents:

an alkanoyl group having from 1 to 6 carbon atoms;

an alkenoyl or alkynoyl group having from 3 to 6 carbon atoms;

an aromatic acyl group in which the aromatic part is a carbocyclic aromatic group which has from 6 to 14 ring carbon atom and which is unsubstituted or is substituted by at least one substituent selected from the groups and atoms represented by $R^c$;

a heterocyclic acyl group in which the heterocyclic part has from 3 to 8 ring atom, of which from 1 to 3 are hetero-atoms selected from nitrogen, oxygen and sulphur hetero-atoms, said heterocylic group being unsubstituted or being substituted by at least one substituent selected from the groups and atoms represented by $R^c$;

an alkoxycarbonyl group in which the alkoxy part has from 1 to 6 carbon atoms;

an aryloxycarbonyl group in which the aryl part has from 6 to 10 ring carbon atom and is unsubstituted or is substituted by at least one substituent selected from the groups and atom represented by $R^c$; or

an aralkyloxycarbonyl group in which the aralkyl part is an alkyl group having from 1 to 6 carbon atoms which is substituted by from 1 to 3 aryl groups having from 6 to 10 ring carbon atoms and being unsubstituted or substituted by at least one substituent selected from the groups and atoms represented by $R^c$;

and pharmaceutically acceptable salts thereof.

The invention also provides a pharmaceutical composition for the treatment or prophylaxis of cerebral impairment, which composition comprises an effective amount of an acetylcholinesterase inhibitor in admixture with a pharmaceutically acceptable carrier or diluent, wherein said acetylcholinesterase inhibitor is at least one

compound of formula (I) or a pharmaceutically acceptable salt thereof.

The invention also provides the use of a compound of formula (I) or a pharmaceutically acceptable salt thereof as a pharmaceutical and, particularly, as an acetylcholinesterase inhibitor.

The present invention also provides processes for preparing these compounds, which are described in greater detail hereafter.

In the compounds of the present invention, where $R^a$ (and, hence, $R^1$ and/or $R^2$) represents an alkyl group having from 1 to 6 carbon atoms, this may be a straight or branched chain group having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those straight or branched chain alkyl groups having from 1 to 4 carbon atoms, preferably the methyl, ethyl, propyl, isopropyl, butyl and isobutyl groups, and most preferably the methyl group.

Where $R^1$, $R^2$ or $R^a$ represents an aryl group, this is a carbocyclic aryl group having from 6 to 14 ring carbon atoms and may be substituted or unsubstituted. In the case of $R^1$ and $R^2$, the substituents are selected from the groups and atoms represented by $R^a$; in the case of $R^a$, they are selected from the groups and atoms represented by $R^c$. There is no particular restriction on the number of substituents, except such as may be imposed by the number of substitutable positions (e.g. 5 in the case of a phenyl group or 7 in the case of a naphthyl group) or possibly by steric constraints. However, in general, we prefer from 1 to 5, more preferably from 1 to 3, and most preferably 1, substituent. Examples of the substituents included in $R^a$ and $R^c$ are given elsewhere herein. The aryl groups preferably have from 6 to 10 ring carbon atoms and more preferably have 6 or 10 ring carbon atoms. Specific examples of the unsubstituted aryl groups include the phenyl, naphthyl (1- or 2- naphthyl), indenyl, phenanthrenyl, anthracenyl, pentalenyl, heptalenyl, acenaphthylenyl and fluorenyl groups, of which the phenyl, naphthyl, indenyl, phenanthrenyl and anthracenyl are preferred, the phenyl and naphthyl groups being more preferred and the phenyl group being most preferred. Any of these groups may be unsubstituted or substituted, as defined above.

Where $R^a$, and, hence, $R^1$, $R^2$, $R^4$ or $R^5$ represents a cycloalkyl group, this has from 3 to 14 ring carbon atoms, which may be present in one or more rings, which may be fused or otherwise attached to each other. Preferred groups are saturated cyclic hydrocarbon groups having from 3 to 10 carbon atoms in a ring, which may optionally be bridged. Specific examples of such groups include the cyclopropyl, cyclobutyl, cyclopentyl, cyclohexyl, cycloheptyl, cyclooctyl, norbornyl and adamantyl groups. Of these, we prefer those saturated cyclic hydrocarbon groups having from 5 to 10 ring carbon atoms, and more preferably the cyclohexyl and adamantyl groups.

Where $R^1$, $R^2$ or $R^a$ represents an aralkyl group, this is an alkyl group having from 1 to 6 carbon atoms which is substituted by at least one, and preferably from 1 to 3, aryl substituents. The alkyl groups have from 1 to 6 carbon atoms and may be as defined and exemplified above in relation to the alkyl groups which may be represented by $R^1$, $R^2$ or $R^a$. The aryl groups are carbocyclic aryl groups having from 6 to 14 ring carbon atom which may be substituted or unsubstituted. In the case of $R^1$ and $R^2$, the substituents are selected from the groups and atom represented by $R^a$; in the case of $R^a$, they are selected from the groups and atom represented by $R^c$. These aryl groups are as defined and exemplified above in relation to the aryl groups which may be represented by $R^1$, $R^2$ or $R^a$. Specific examples of the unsubstituted aralkyl groups include the benzyl, naphthylmethyl, indenylmethyl, phenanthrenylmethyl, anthracenylmethyl, pentalenylmethyl, heptalenylmethyl, acenaphthylenylmethyl, fluorenylmethyl, diphenylmethyl, triphenylmethyl, 1-phenylethyl, phenethyl (i.e. 2-phenylethyl), 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl, 6-naphthylhexyl, diphenylmethyl (i.e. benzhydryl) and triphenylmethyl (i.e. trityl) groups. Of these, we prefer those aralkyl groups in which an alkyl group having from 1 to 6 carbon atoms, more preferably from 1 to 4 carbon atoms, is bonded with an aryl group having from 6 to 10 ring carbon atoms, and more preferably 6 or 10 carbon atoms. Particularly preferred aralkyl groups are the benzyl, 2-phenylethyl, 3-propylphenyl and 4-butylphenyl groups, the benzyl group being most preferred. Any of these groups may be unsubstituted or substituted, as defined above.

Where $R^a$ (and, hence, $R^1$ and/or $R^2$) represents a heterocyclic group, this has from 3 to 14 ring atoms, which may be in a one or more ring systems fused or otherwise attached to each other. Of the ring atoms, from 1 to 4 are hetero-atoms selected from nitrogen, oxygen and sulphur atoms. However, where there are 4

ring hetero-atoms, we prefer that all 4 should be nitrogen atoms. Where there are 3 ring hetero-atoms, we prefer that 1, 2 or 3 should be nitrogen atoms, and, correspondingly, 2, 1 or 0 should be oxygen or sulphur atoms. Where there are 2 or 1 hetero-atoms, these may be selected freely from the aforementioned nitrogen, oxygen and sulphur atoms. The heterocyclic ring may be fully or partially saturated, or it may be completely unsaturated (i.e. aromatic). Examples of unsaturated heterocyclic groups include the furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl, indolyl, quinolyl, isoquinolyl, purinyl and benzothienyl groups; examples of saturated or partially saturated heterocyclic groups include the morpholinyl (especially morpholino), thiomorpholinyl (especially thiomorpholino), pyrrolidinyl, pyrrolinyl, imidazolidinyl, 4,5-dihydroimidazolyl, pyrazolidinyl, pyrazolinyl, piperidyl, piperazyl, dihydrobenzothienyl and indolinyl. Of these, we prefer the unsaturated or partially saturated heterocyclic groups having 5 to 7 ring atoms including at least one nitrogen or sulphur atom and optionally an oxygen atom. The more preferred groups are the thienyl, furyl, 4,5-dihydroimidazolyl, tetrahydroquinolyl, tetrahydroisoquinolyl and indolinyl groups. Any of these groups may be unsubstituted or substituted, as defined above, i.e. by a group or atom represented by $R^a$, in the case of $R^1$ or $R^2$, and by a group or atom represented by $R^b$, in the case of $R^a$.

Where $R^a$ (and, hence, $R^1$ and/or $R^2$) represents a halogen atom, this may be a fluorine, chlorine, bromine or iodine atom, preferably a fluorine or chlorine atom.

Where $R^a$ (and, hence, $R^1$ and/or $R^2$) represents an alkylamino or dialkylamino group, the or each alkyl group has from 1 to 6 carbon atom and may be any of those alkyl groups exemplified above. In the case of the dialkylamino groups, the two alkyl groups may be the same or different. Specific examples of such groups include the methylamino, ethylamino, propylamino, isopropylamino, butylamino, isobutylamino, sec-butylamino, t-butylamino, pentylamino, isopentylamino, 2-methylbutylamino, neopentylamino, 1-ethylpropylamino, hexylamino, 4-methylpentylamino, 3-methylpentylamino, 2-methylpentylamino, 1-methylpentylamino, 3,3-dimethylbutylamino, 2,2-dimethylbutylamino, 1,1-dimethylbutylamino, 1,2-dimethylbutylamino, 1,3-dimethylbutylamino, 2,3-dimethylbutylamino, 2-ethylbutylamino, dimethylamino, diethylamino, dipropylamino, diisopropylamino, dibutylamino, diisobutylamino, di-sec-butylamino, di-t-butylamino, dipentylamino, diisopentylamino, di-2-methylbutylamino, dineopentylamino, di-(1-ethylpropyl)amino, dihexylamino, di-(4-methylpentyl)amino, di-(3-methylpentyl)amino, di-(2-methylpentyl)amino, di-(1-methylpentyl)amino, di-(3,3-dimethylbutyl)amino, di-(2,2-dimethylbutyl)amino, di-(1,1-dimethylbutyl)-amino, di-(1,2-dimethylbutyl)amino, di-(1,3-dimethylbutyl)amino, di-(2,3-dimethylbutyl)amino and di-(2-ethylbutyl)amino groups. Of these, we prefer the straight or branched chain mono- and di- alkylamino groups having from 1 to 4 carbon atom in the or each alkyl group, and more prefer the diethylamino and dimethylamino groups.

Where $R^a$ (and, hence, $R^1$ and/or $R^2$) represents a mono- or di- arylamino group, the or each aryl part may be as defined and exemplified above. Specific examples of such arylamino groups include the phenylamino, indenylamino, naphthylamino, phenanthrenylamino, anthracenylamino, diphenylamino, diindenylamino, dinaphthylamino, diphenanthrenylamino and dianthracenylamino groups. Of these, we prefer the arylamino groups where the or each aryl part has from 6 to 10 carbon atoms, more preferably 6 or 10 ring carbon atom, and most prefer the phenylamino group. Any of these groups may be unsubstituted or substituted, as defined above, i.e. by a group or atom $R^a$ in the case of $R^1$ and $R^2$ or by a group or atom $R^c$ in the case of $R^a$.

Where $R^a$ (and, hence, $R^1$ and/or $R^2$) represents an aminoalkyl group, the alkyl part may be any of the alkyl groups exemplified above. Specific examples include the aminomethyl, 2-aminoethyl, 1-aminoethyl, 1-aminopropyl, 2-aminopropyl, 3-aminopropyl, 1-amino-1-methylethyl, 1-aminobutyl, 2-aminobutyl, 3-aminobutyl, 4-aminobutyl, 3-amino-2-methylpropyl, 1-aminopentyl, 2-aminopentyl, 3-aminopentyl, 4-aminopentyl, 5-aminopentyl, 4-amino-2-methylbutyl, 3-amino-1-ethylpropyl, 5-amino-4-methylpentyl, 5-amino-3-methylpentyl, 5-amino-2-methylpentyl, 5-amino-1-methylpentyl, 4-amino-3,3-dimethylbutyl, 4-amino-2,2-dimethylbutyl, 4-amino-1,1-dimethylbutyl, 4-amino-1,2-dimethylbutyl, 4-amino-1,3-dimethylbutyl, 4-amino-2,3-dimethylbutyl, 4-amino-2-ethylbutyl and 6-aminohexyl groups. Of these, we prefer those straight or branched chain aminoalkyl groups having from 1 to 4 carbon atoms, preferably the aminomethyl, 2-aminoethyl, 3-aminopropyl and 4-aminobutyl groups, and most preferably the aminomethyl group. The amino group may be unsubstituted or it may be substituted by one or two substituents selected from the groups represented by $R^d$, for example:

alkanoyl groups having from 1 to 6 carbon atoms, for example the formyl, acetyl, propionyl, valeryl, isovaleryl, pivaloyl or hexanoyl groups, of which the acetyl, propionyl and pivaloyl groups are preferred;

alkenoyl or alkynoyl groups having from 3 to 6 carbon atoms, for example the acryloyl, methacryloyl, propioloyl, crotonoyl or isocrotonoyl groups, of which the acryloyl and methacryloyl groups are preferred;

aromatic acyl groups in which the aromatic part is a carbocyclic aromatic group which may be as defined and exemplified above in relation to the aryl groups; specific examples are the benzoyl, naphthoyl and toluoyl groups, any of which may be unsubstituted or substituted, as defined above;

a heterocyclic acyl group in which the heterocyclic part has from 3 to 8 ring atoms, of which from 1 to

3 are hetero-atoms selected from nitrogen, oxygen and sulphur hetero-atoms, said heterocylic group being unsubstituted or being substituted by at least one substituent selected from the groups and atoms represented by $R^c$; examples include the furoyl, thenoyl, nicotinoyl and isonicotinoyl group, any of which may be unsubstituted or substituted, as defined above;

alkoxycarbonyl groups in which the alkoxy part has from 1 to 6 carbon atoms, for example such groups derived from any of the alkyl groups exemplified above; specific examples include the methoxycarbonyl, ethoxycarbonyl, propoxycarbonyl, isopropoxycarbonyl, butoxycarbonyl, isobutoxycarbonyl, sec-butoxycarbonyl, t-butoxycarbonyl, pentyloxycarbonyl, isopentyloxycarbonyl, 2-methylbutyloxycarbonyl, neopentyloxycarbonyl, hexyloxycarbonyl, 4-methylpentyloxycarbonyl, 3-methylpentyloxycarbonyl, 2-methylpentyloxycarbonyl, 3,3-dimethylbutyloxycarbonyl, 2,2-dimethylbutyloxycarbonyl, 1,1-dimethylbutyloxycarbonyl, 1,2-dimethylbutyloxycarbonyl, 1,3-dimethylbutyloxycarbonyl and 2,3-dimethylbutyloxycarbonyl; preferably a methoxycarbonyl group or an ethoxycarbonyl group;

an aryloxycarbonyl group in which the aryl part has from 6 to 10 ring carbon atoms and is unsubstituted or is substituted by at least one substituent selected from the groups and atoms represented by $R^c$, and the aryl part may be any of those aryl groups exemplified above; specific examples include the phenoxycarbonyl and

naphthyloxycarbonyl groups, either of which may be unsubstituted or substituted, as defined above; and

aralkyloxycarbonyl groups in which the aralkyl part may be any of those aralkyl groups defined and exemplified above, but preferably a benzyloxycarbonyl group, which may be substituted or unsubstituted.

Where $R^a$ (and, hence, $R^1$ and/or $R^2$) represents an alkylaminoalkyl group, each alkyl part has from 1 to 6 carbon atoms and may be independently selected from those alkyl groups exemplified above. Preferably each alkyl part has from 1 to 4 carbon atoms. Specific examples of such alkylaminoalkyl groups include the methylaminomethyl, ethylaminomethyl, propylaminomethyl, butylaminomethyl, isopropylaminomethyl, isobutylaminomethyl, sec-butylaminomethyl, t-butylaminomethyl, pentylaminomethyl, hexylaminomethyl, 2-methylaminoethyl, 2-ethylaminoethyl, 2-propylaminoethyl, 2-butylaminoethyl, 2-isopropylaminoethyl, 2-isobutylaminoethyl, 2-sec-butylaminoethyl, 2-t-butylaminoethyl, 2-pentylaminoethyl, 2-hexylaminoethyl, 3-methylaminopropyl, 3-ethylaminopropyl, 3-propylaminopropyl, 3-butylaminopropyl, 3-isopropylaminopropyl, 3-isobutylaminopropyl, 3-sec-butylaminopropyl, 3-t-butylaminopropyl, 3-pentylaminopropyl, 3-hexylaminopropyl, 4-methylaminobutyl, 4-ethylaminobutyl, 4-propylaminobutyl, 4-butylaminobutyl, 4-isopropylaminobutyl, 4-isobutylaminobutyl, 4-sec-butylaminobutyl, 4-t-butylaminobutyl, 4-pentylaminobutyl, 4-hexylaminobutyl, 5-methylaminopentyl, 5-ethylaminopentyl, 5-propylaminopentyl, 5-butylaminopentyl, 5-isopropylaminopentyl, 5-isobutylaminopentyl, 5-sec-butylaminopentyl, 5-t-butylaminopentyl, 5-pentylaminopentyl, 5-hexylaminopentyl, 6-methylaminohexyl, 6-ethylaminohexyl, 6-propylaminohexyl, 6-butylaminohexyl, 6-isopropylaminohexyl, 6-isobutylaminohexyl, 6-sec-butylaminohexyl, 6-t-butylaminohexyl, 6-pentylaminohexyl and 6-hexylaminohexyl groups, of which we prefer those having from 1 to 4 carbon atoms in each alkyl group, especially the methylaminomethyl and ethylaminomenthyl groups.

Where $R^a$ (and, hence, $R^1$ and/or $R^2$) represents an alkynylaminoalkyl group, the alkyl part has from 1 to 6 carbon atom and may be selected from those alkyl groups exemplified above. Preferably the alkyl part has from 1 to 4 carbon atoms. The alkynyl part has from 2 to 6 carbon atoms and may be a straight or branched chain group having from 2 to 6, preferably 3 or 4, carbon atoms; examples include the ethynyl, propargyl (2-propynyl), 1-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl groups, of which the propynyl and butynyl groups are preferred, the propargyl and 2-butynyl groups being most preferred. Specific examples of such alkynylaminoalkyl groups include the (ethynylamino)-methyl, (propargylamino)methyl, N-(2-butynyl)aminomethyl, 2-(ethynylamino)ethyl, 2-(propargylamino)ethyl, 2-[(2-butynyl)amino]ethyl, 3-(ethynylamino)propyl, 3-(propargylamino)propyl, 3-[(2-butynyl)amino]propyl, 4-(ethynylamino)butyl, 4-(propargylamino)butyl, 4-[(2-butynyl)amino]butyl, 5-(ethynylamino)pentyl, 5-(propargylamino)pentyl, 5-[(2-butynyl)amino]pentyl, 6-(ethynylamino)hexyl, 6-(propargylamino)hexyl and 6-[(2-butynyl)amino]hexyl, of which we prefer those having from 1 to 4 carbon atoms in the alkyl group and from 2 to 4 carbon atoms (especially 3 or 4 carbon atoms) in the alkynyl group, especially the propargylaminomethyl and (2-butynyl)aminomethyl groups.

Where $R^a$ (and, hence, $R^1$ and/or $R^2$) represents an alkylsulphonyl group, the alkyl part has from 1 to 6 carbon atoms and may be any of the alkyl groups exemplified above. Specific examples of such alkylsulphonyl groups include the methylsulphonyl, ethylsulphonyl, propylsulphonyl, isopropylsulphonyl, butylsulphonylsulphonylsulphonyl, isobutylsulphonyl, sec-butylsulphonyl, t-butylsulphonyl, pentylsulphonyl, isopentylsulphonyl, 2-methylbutylsulphonyl, neopentylsulphonyl, 1-ethylpropylsulphonyl, hexylsulphonyl, 4-methylpentylsulphonyl, 3-methylpentylsulphonyl, 2-methylpentylsulphonyl, 1-methylpentylsulphonyl, 3,3-dimethylbutylsulphonyl, 2,2-dimethylbutylsulphonyl, 1,1-dimethylbutylsulphonyl, 1,2-dimethylbutylsulphonyl, 1,3-dimethylbutylsulphonyl, 2,3-dimethylbutylsulphonyl and 2-ethylbutylsulphonyl groups. Of these, we prefer the straight or

branched chain alkylsulphonyl groups having from 1 to 4 carbon atoms, more preferably the methyl- sulphonyl group.

Where $R^a$ (and, hence, $R^1$ and/or $R^2$) represents a haloalkylsulphonyl group, the alkyl part has from 1 to 6 carbon atom and may be any of the alkyl groups exemplified above, and the halogen atom may likewise be any of those exemplified above. There is no particular restriction on the number of halogen atoms present, except such as may be imposed by the number of substitutable positions; however, subject to this restriction, from 1 to 5 halogen atom are preferred, from 1 to 3 being more preferred. Specific examples of such haloalkylsulphonyl groups include the trifluoromethylsulphonyl, trichloromethylsulphonyl, difluoromethylsulphonyl, dichloromethylsulphonyl, dibromomethylsulphonyl, fluoromethylsulphonyl, 2,2,2-trichloroethylsulphonyl, 2,2,2-trifluoroethylsulphonyl, 2-bromoethylsulphonyl, 2-chloroethylsulphonyl, 2-fluoroethylsulphonyl and 2,2-dibromoethylsulphonyl groups. Of these, we prefer those haloalkylsulphonyl groups having 1 or 2 carbon atoms, and more prefer the trifluoromethylsulphonyl group.

Where $R^a$ (and, hence, $R^1$ and/or $R^2$) represents an alkanoyl group, this may be a straight or branched chain group having from 1 to 6 carbon atoms, and examples of such groups include the formyl, acetyl, propionyl, butyryl, isobutyryl, valeryl, isovaleryl and hexanoyl groups. Of these, we prefer those straight or branched chain alkanoyl groups having from 1 to 4 carbon atoms, and more prefer the acetyl group.

Where $R^a$ (and, hence, $R^1$ and/or $R^2$) represents an arylcarbonyl group, the aryl part may be any of those aryl groups defined and exemplified above. Specific examples of such groups include the benzoyl, 1-naphthylcarbonyl, 2-naphthylcarbonyl, 1-phenanthrylcarbonyl, 1-anthracenylcarbonyl and 1-indenylcarbonyl groups. Of these, we prefer the benzoyl, 1-naphthylcarbonyl and 1-phenanthrylcarbonyl groups, and more prefer those arylcarbonyl groups in which the aryl part has from 6 to 10, still-more preferably 6 or 10, ring carbon atoms. The most preferred such group is the benzoyl group. Any of these groups may be unsubstituted or substituted, as defined above.

Where $R^a$ (and, hence, $R^1$ and/or $R^2$) represents an arylalkanoyl group, the aryl part is a carbocyclic aromatic group having from 6 to 14 ring carbon atom as defined and exemplified above, and the alkanoyl part has from 2 to 6 carbon atom, also as defined and exemplified above. There may be one or more aryl groups present as substituents on the alkanoyl group, preferably from 1 to 3 aryl groups, and more preferably 1 aryl goup. Specific examples of such arylalkanoyl groups include the phenylacetyl, 2-(1-naphthyl)acetyl, 2-phenylpropionyl, 3-phenylpropionyl, 2-phenylbutyryl, 3-phenylbutyryl, 4-phenylbutyryl, 2-phenylvaleryl, 3-phenylvaleryl, 4-phenylvaleryl, 5-phenylvaleryl, 2-phenylhexanoyl, 3-phenylhexanoyl, 4-phenylhexanoyl, 5-phenylhexanoyl and 6-phenylhexanoyl groups. Of these, we prefer those arylalkanoyl groups having from 6 to 10, more preferably 6 or 10, ring carbon atoms in the aryl part and from 2 to 4 carbon atoms in the alkanoyl part. In particular, we prefer that the aryl part should be a phenyl group, and the most preferred group is the phenylacetyl group. Any of these groups may be unsubstituted or substituted, as defined above.

Where $R^a$ (and, hence, $R^1$ and/or $R^2$) represents an alkoxy group having from 1 to 6 carbon atoms, this may be a straight or branched chain group having from 1 to 6, preferably from 1 to 4, carbon atoms, and examples include the methoxy, ethoxy, propoxy, isopropoxy, butoxy, isobutoxy, sec-butoxy, t-butoxy, pentyloxy, isopentyloxy, 2-methylbutyloxy, neopentyloxy, hexyloxy, 4-methylpentyloxy, 3-methylpentyloxy, 2-methylpentyloxy, 3,3-dimethylbutyloxy, 2,2-dimethylbutyloxy, 1,1-dimethylbutyloxy, 1,2-dimethylbutyloxy, 1,3-dimethylbutyloxy and 2,3-dimethylbutyloxy groups. Of these, we prefer those straight or branched chain alkoxy groups having from 1 to 4 carbon atom, more preferably the methoxy and ethoxy groups.

Where $R^a$ (and, hence, $R^1$ and/or $R^2$) represents an alkoxycarbonyl group, this has from 2 to 7 carbon atom, i.e. the alkoxy part has from 1 to 6 carbon atoms, and may be a straight or branched chain group. Specific examples of such groups include the methoxycarbonyl, ethoxycarbonyl, propoxycarbonyl, isopropoxycarbonyl, butoxycarbonyl, isobutoxycarbonyl, sec-butoxycarbonyl, t-butoxycarbonyl, pentyloxycarbonyl, isopentyloxycarbonyl, 2-methylbutyloxycarbonyl, neopentyloxycarbonyl, hexyloxycarbonyl, 4-methylpentyloxycarbonyl, 3-methylpentyloxycarbonyl, 2-methylpentyloxycarbonyl, 3,3-dimethylbutyloxycarbonyl, 2,2-dimethylbutyloxycarbonyl, 1,1-dimethylbutyloxycarbonyl, 1,2-dimethylbutyloxycarbonyl, 1,3-dimethylbutyloxycarbonyl and 2,3-dimethylbutyloxycarbonyl groups. Of these, we prefer those alkoxycarbonyl groups having from 2 to 5 carbon atoms, more preferably the methoxycarbonyl group or the ethoxycarbonyl group.

Where $R^a$ (and, hence, $R^1$ and/or $R^2$) represents a haloalkyl group, the halogen atom(s) and the alkyl group may each be as separately defined and exemplified above. There is no restriction on the number of halogen atoms, except such as may be imposed by the number of substitutable positions. However, in general, we prefer, subject to that restriction, from 1 to 5 halogen atoms, more preferably from 1 to 3 halogen atoms. Specific examples of such haloalkyl groups include the trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl and 2,2-dibromoethyl groups. Of these, we prefer those haloalkyl groups having from 1 to 4, more preferably 1 or 2, carbon atoms, and most prefer the trifluoromethyl group.

Where $R^1$ or $R^2$ represents an aralkyloxycarbonyl group, the aralkyl part is as defined and exemplified above. Specific examples include the benzyloxycarbonyl, naphthylmethoxycarbonyl, indenylmethoxycarbonyl, phenanthrenylmethoxycarbonyl, anthracenylmethoxycarbonyl, pentalenylmethoxycarbonyl, heptalenylmethoxycarbonyl, acenaphthylenylmethoxycarbonyl, fluorenylmethoxycarbonyl, diphenylmethoxycarbonyl, triphenylmethoxycarbonyl, 1-phenylethoxycarbonyl, 2-phenylethoxycarbonyl, 1-naphthylethoxycarbonyl, 2-naphthylethoxycarbonyl, 1-phenylpropoxycarbonyl, 2-phenylpropoxycarbonyl, 3-phenylpropoxycarbonyl, 1-naphthylpropoxycarbonyl, 2-naphthylpropoxycarbonyl, 3-naphthylpropoxycarbonyl, 1-phenylbutoxycarbonyl, 2-phenylbutoxycarbonyl, 3-phenylbutoxycarbonyl, 4-phenylbutoxycarbonyl, 1-naphthylbutoxycarbonyl, 2-naphthylbutoxycarbonyl, 3-naphthylbutoxycarbonyl, 4-naphthylbutoxycarbonyl, 1-phenylpentyloxycarbonyl, 2-phenylpentyloxycarbonyl, 3-phenylpentyloxycarbonyl, 4-phenylpentyloxycarbonyl, 5-phenylpentyloxycarbonyl, 1-naphthylpentyloxycarbonyl, 2-naphthylpentyloxycarbonyl, 3-naphthylpentyloxycarbonyl, 4-naphthylpentyloxycarbonyl, 5-naphthylpentyloxycarbonyl, 1-phenylhexyloxycarbonyl, 2-phenylhexyloxycarbonyl, 3-phenylhexyloxycarbonyl, 4-phenylhexyloxycarbonyl, 5-phenylhexyloxycarbonyl, 6-phenylhexyloxycarbonyl, 1-naphthylhexyloxycarbonyl, 2-naphthylhexyloxycarbonyl, 3-naphthylhexyloxycarbonyl, 4-naphthylhexyloxycarbonyl, 5-naphthylhexyloxycarbonyl, 6-naphthylhexyloxycarbonyl, diphenylmethoxycarbonyl and triphenylmethoxycarbonyl groups. Of these, we prefer those aralkyloxycarbonyl groups in which an alkyl group having from 1 to 6 carbon atoms, more preferably from 1 to 4 carbon atom, is bonded with an aryl group having from 6 to 10 ring carbon atom, and more preferably 6 or 10 carbon atom. Particularly preferred aralkyloxycarbonyl groups are the benzyloxycarbonyl, 2-phenylethoxycarbonyl, 3-propylphenyloxycarbonyl and 4-butylphenyloxycarbonyl groups, the benzyloxycarbonyl group being most preferred. Any of these groups may be unsubstituted or substituted, as defined above.

Where $R^1$ or $R^2$ represents an arylaminoalkyl group, this may be any one of the aminoalkyl groups defined above, but in which the amino group is substituted by 1 or 2, preferably 1, aryl groups. Specific examples of such groups include the N-phenylaminomethyl, 2-(N-phenylamino)ethyl, 2-(N-naphthylamino)ethyl, 1-(N-phenylamino)ethyl, 1-(N-phenylamino)-propyl, 2-(N-phenylamino)propyl, 3-(N-phenylamino)-propyl, 1-(N-phenylamino)-1-methylethyl, 1-(N-phenylamino)butyl, 2-(N-phenylamino)butyl, 3-(N-phenylamino)-butyl, 4-(N-phenylamino)butyl, 3-(N-phenylamino)-2-methylpropyl, 1-(N-phenylamino)pentyl, 2-(N-phenylamino)pentyl, 3-(N-phenylamino)pentyl, 4-(N-phenylamino)pentyl, 5-(N-phenylamino)pentyl, 4-(N-phenylamino)-2-methylbutyl, 3-(N-phenylamino)-1-ethylpropyl, 5-(N-phenylamino)-4-methylpentyl, 5-(N-phenylamino)-3-methylpentyl, 5-(N-phenylamino)-2-methylpentyl, 5-(N-phenylamino)-1-methylpentyl, 4-(N-phenylamino)-3,3-dimethylbutyl, 4-(N-phenylamino)-2,2-dimethylbutyl, 4-(N-phenylamino)-1,1-dimethylbutyl, 4-(N-phenylamino)-1,2-dimethylbutyl, 4-(N-phenylamino)-1,3-dimethylbutyl, 4-(N-phenylamino)-2,3-dimethylbutyl, 4-(N-phenylamino)-2-ethylbutyl and 6-(N-phenylamino)hexyl groups. Of these, we prefer those straight or branched chain arylaminoalkyl groups having from 1 to 4 carbon atoms, preferably the N-phenylaminomethyl, 2-(N-phenylamino)-ethyl, 3-(N-phenylamino)propyl and 4-(N-phenylamino)-butyl groups, and most preferably the (N-phenylamino)-methyl group. Any of these groups may be unsubstituted or substituted, as defined above.

Where $R^1$ or $R^2$ represents an alkyl group which has from 1 to 6 carbon atom and which is substituted by a single heterocyclic group, the heterocyclic group and the alkyl group may each be as separately defined and exemplified above. Specific examples of such groups include the 2-furylmethyl, 2-thenyl (i.e. 2-thienylmethyl), 2-(2-thienyl)ethyl, 2-pyrrolylmethyl, 3-pyrazolylmethyl, 2-imidazolylmethyl, 3-oxazolylmethyl, 2-isoxazolylmethyl, 2-thiazolylmethyl, 3-isothiazolylmethyl, 1,2,3-oxadiazol-4-ylmethyl, 1,2,3-triazol-2-ylmethyl, 1,2,4-triazol-1-ylmethyl, tetrazol-4-ylmethyl, 1,3,4-thiadiazol-2-ylmethyl, 2-pyranylmethyl, 2-pyridylmethyl, 2-pyridazinylmethyl, 2-pyrimidinylmethyl, 2-pyrazinylmethyl, 2-indolylmethyl, 4-quinolylmethyl, 4-isoquinolylmethyl, 8-purinylmethyl, 2-benzothienylmethyl, 2-morpholinylmethyl, 2-thiomorpholinylmethyl, 2-pyrrolidinylmethyl, 2-pyrrolinylmethyl, 2-imidazolidinylmethyl, 4,5-dihydroimidazol-2-ylmethyl, 3-pyrazolidinylmethyl, 3-pyrazolinylmethyl, 4-piperidylmethyl, 2-piperazylmethyl and 2-dihydrobenzothienylmethyl groups. Of these, we prefer those in which the heterocyclic group is an unsaturated or partially saturated heterocyclic group with from 5 to 7 ring atoms containing at least one nitrogen or sulphur atom and optionally an oxygen atom. The most preferred groups are the 2-thienylmethyl, 2-furylmethyl, 4,5-dihydroimidazol-2-ylmethyl, tetrahydroquinol-2-ylmethyl and tetrahydroquinol-2-ylmethyl groups.

Where $R^1$ or $R^2$ represents an alkylamino group which has from 1 to 6 carbon atoms and which is substituted by a single heterocyclic group, the heterocyclic group and the alkyl group may each be as separately defined and exemplified above. Specific examples of such groups include the 2-furylmethylamino, 2-thenylamino (i.e. 2-thienylmethylamino), 2-(2-thienyl)ethylamino, 2-pyrrolylmethylamino, 3-pyrazolylmethylamino, 2-imidazolylmethylamino, 3-oxazolylmethylamino, 2-isoxazolylmethylamino, 2-thiazolylmethylamino, 3-isothiazolylmethylamino, 1,2,3-oxadiazol-4-ylmethylamino, 1,2,3-triazol-2-ylmethylamino, 1,2,4-triazol-1-ylmethylamino, tetrazol-4-ylmethylamino, 1,3,4-thiadiazol-2-ylmethylamino, 2-pyranylmethylamino, 2-pyridylmethylamino, 2-pyridazinylmethylamino, 2-pyrimidinylmethylamino, 2-pyrazinylmethylamino, 2-indolylmethylamino, 4-quinolylmethylamino, 4-isoquinolylmethylamino, 8-purinylmethylamino, 2-benzothienylmethylamino, 2-morpholinylmethylamino, 2-thiomorpholinylmethylamino, 2-pyrrolidinylmethylamino, 2-pyrrolinylmethylamino, 2-imidazolidinylmethylamino, 4,5-dihydroimidazol-2-ylmethylamino, 3-pyrazolidinylmethylamino, 3-pyrazolinylmethylamino, 4-piperidylmethylamino, 2-piperazylmethylamino and 2-dihydrobenzothienylmethylamino groups. Of these, we prefer those in which the heterocyclic group is an unsaturated or partially saturated heterocyclic group with from 5 to 7 ring atoms containing at least one nitrogen or sulphur atom and optionally an oxygen atom. The most preferred groups are the 2-thienylmethylamino, 2-furylmethylamino, 4,5-dihydroimidazol-2-ylmethylamino, tetrahydroquinol-2-ylmethylamino and tetrahydroquinol-2-ylmethylamino groups.

Where U represents a group of formula -CO- or -CH(O$R^3$)-, where $R^3$ represents a hydrogen atom or a hydroxy-protecting group, the nature of the hydroxy-protecting group is not critical to the invention, and any such group known in the art may equally be used here. The only restriction is that, if the compound is to be used for therapeutic or prophylactic purposes, the group must be pharmaceutically acceptable. However, if the compound is to be used for other purposes, e.g. as an intermediate in the preparation of other, and perhaps more active compounds, even this restriction does not apply.

Preferably the protecting group is an ester-forming group. Examples of such groups include:

alkyl groups having from 1 to 6 carbon atom, such as the methyl, ethyl, propyl, isopropyl, butyl, isobutyl, sec-butyl, t-butyl, pentyl and hexyl groups;

alkanesulphonyl groups in which the alkane part has from 1 to 6 carbon atoms, such as the methanesulphonyl, ethanesulphonyl and 1-propanesulphonyl groups;

fluorinated alkanesulphonyl groups in which the alkane part has from 1 to 6 carbon atoms, such as the trifluoromethanesulphonyl and pentafluoroethanesulphonyl groups;

arylsulphonyl groups in which the aryl part is as defined and exemplified above, such as the benzenesulphonyl and p-toluenesulphonyl groups;

aliphatic acyl groups, preferably: alkanoyl groups having from 1 to 25 carbon atoms, more preferably from 1 to 20 carbon atoms, still more preferably from 1 to 6 carbon atoms, and most preferably from 1 to 4 carbon atoms, (such as the formyl, acetyl, propionyl, butyryl, isobutyryl, pivaloyl, valeryl, isovaleryl, hexanoyl, heptanoyl, octanoyl, lauroyl, myristoyl, tridecanoyl, palmitoyl and stearoyl groups, of which the acetyl group is most preferred); halogenated alkanoyl groups having from 2 to 6 carbon atoms, especially halogenated acetyl groups (such as the chloroacetyl, dichloroacetyl, trichloroacetyl and trifluoroacetyl groups); lower alkoxyalkanoyl groups in which the alkoxy part has from 1 to 5, preferably from 1 to 3, carbon atoms and the alkanoyl part has from 2 to 6 carbon atoms and is preferably an acetyl group (such as the methoxyacetyl group); and unsaturated analogs of such groups, especially alkenoyl or alkynoyl groups having from 3 to 6 carbon atoms [such as the acryloyl, methacryloyl, propioloyl, crotonoyl, isocrotonoyl and (E)-2-methyl-2-butenoyl groups];

aromatic acyl groups, preferably arylcarbonyl groups, in which the aryl part has from 6 to 14, more preferably from 6 to 10, still more preferably 6 or 10, and most preferably 6, ring carbon atoms and is a carbocyclic group, which is unsubstituted or has from 1 to 5, preferably from 1 to 3 substituents, selected from the groups and atoms represented by $R^c$, preferably: unsubstituted groups (such as the benzoyl, $\alpha$-naphthoyl and $\beta$-naphthoyl groups); halogenated arylcarbonyl groups (such as the 2-bromobenzoyl and 4-chlorobenzoyl groups); lower alkyl-substituted arylcarbonyl groups, in which the or each alkyl substituent has from 1 to 5, preferably from 1 to 4, carbon atoms (such as the 2,4,6-trimethylbenzoyl and 4-toluoyl groups); lower alkoxy-substituted arylcarbonyl groups, in which the or each alkoxy substituent preferably has from 1 to 5, preferably from 1 to 4, carbon atoms (such as the 4-anisoyl group); nitro-substituted arylcarbonyl groups (such as the 4-nitrobenzoyl and 2-nitrobenzoyl groups); lower alkoxycarbonyl-substituted arylcarbonyl groups, in which the or each alkoxycarbonyl substituent preferably has from 2 to 6 carbon atoms [such as the 2-(methoxycarbonyl)benzoyl group]; and aryl-substituted arylcarbonyl groups, in which the aryl substituent is as defined above, except that, if it is substituted by a further aryl group, that aryl group is not itself substituted by an aryl group (such as the 4-phenylbenzoyl group);

heterocyclic groups having 5 or 6 ring atoms, of which 1 or 2 are hetero-atoms selected from oxygen, sulphur and nitrogen atoms, preferably oxygen or sulphur atoms, which groups may be unsubstituted or may have at least one substituent selected from the groups and atoms represented by $R^b$; examples include: the tetrahydropyranyl groups, which may be substituted or unsubstituted, such as the tetrahydropyran-2-yl, 3-bromotetrahydropyran-2-yl and 4-methoxytetra- hydropyran-4-yl groups; tetrahydrothiopyranyl groups, which may be substituted or unsubstituted, such as the tetrahydrothiopyran-2-yl and 4-methoxytetrahydrothiopyran-4-yl groups; tetrahydrofuranyl groups, which may be substituted or unsubstituted, such as the tetrahydrofuran-2-yl group; and tetrahydrothienyl groups, which may be substituted or unsubstituted, such as the tetrahydrothien-2-yl group;

tri-substituted silyl groups, in which all three or two or one of the substituents are alkyl groups having from 1 to 5, preferably from 1 to 4, carbon atoms, and none, one or two of the substituents are aryl groups, as

defined above, but preferably phenyl or substituted phenyl groups, preferably: tri(lower alkyl)silyl groups (such as the trimethylsilyl, triethylsilyl, isopropyldimethylsilyl, t-butyldimethylsilyl, methyldiisopropylsilyl, methyldi-t-butylsilyl and triisopropylsilyl groups); and tri(lower alkyl)silyl groups in which one or two of the alkyl groups have been replaced by aryl groups (such as the diphenylmethylsilyl, diphenylbutylsilyl, diphenyl-t-butylsilyl, diphenylisopropylsilyl and phenyldiisopropylsilyl groups);

alkoxyalkyl groups, in which the alkoxy and alkyl parts each have from 1 to 5, preferably from 1 to 4, carbon atoms, especially alkoxymethyl groups, and such groups which have at least one, preferably from 1 to 5, more preferably from 1 to 3, and most preferably 1, substituents, preferably: lower alkoxymethyl groups and other alkoxyalkyl groups (such as the methoxymethyl, 1,1-dimethyl-1-methoxymethyl, ethoxymethyl, propoxymethyl, isopropoxymethyl, butoxymethyl and t-butoxymethyl groups); lower alkoxy-substituted lower alkoxymethyl groups (such as the 2-methoxyethoxymethyl group); halogenated lower alkoxymethyl groups [such as the 2,2,2-trichloroethoxymethyl and bis(2-chloroethoxy)-methyl groups] and lower alkoxy-substituted ethyl groups (such as the 1-ethoxyethyl, 1-methyl-1-methoxyethyl and 1-isopropoxyethyl groups);

other substituted ethyl groups, preferably: halogenated ethyl groups (such as the 2,2,2-trichloroethyl group); and arylselenyl-substituted ethyl groups, in which the aryl part is as defined above [such as the 2-(phenylselenyl)ethyl group];

aralkyl groups, preferably alkyl groups having from 1 to 4, more preferably from 1 to 3 and most preferably 1 or 2, carbon atoms which are substituted with from 1 to 3 aryl groups, as defined and exemplified above, which may be unsubstituted (such as the benzyl, phenethyl, 1-phenylethyl, 3-phenylpropyl, α-naphthylmethyl, p-naphthylmethyl, diphenylmethyl, triphenylmethyl, α-naphthyldiphenylmethyl and 9-anthrylmethyl groups) or substituted on the aryl part with a substituent, such as a lower alkyl group, a lower alkoxy group, a nitro group, a halogen atom, a cyano group, or an alkylenedioxy group having from 1 to 3 carbon atoms, preferably a methylenedioxy group, [such as the 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzoyl, 4-bromobenzyl, 4-cyanobenzyl, 4-cyanobenzyldiphenylmethyl, bis(2-nitrophenyl)-methyl and piperonyl groups);

alkoxycarbonyl groups, especially such groups having from 2 to 7, more preferably 2 to 5, carbon atoms and which may be unsubstituted (such as the methoxycarbonyl, ethoxycarbonyl, t-butoxycarbonyl and isobutoxycarbonyl groups) or substituted with a halogen atom or a tri-substituted silyl group, e.g. a tri(lower alkylsilyl) group (such as the 2,2,2-trichloroethoxycarbonyl and 2-trimethylsilylethoxycarbonyl groups);

alkenyloxycarbonyl groups in which the alkenyl part has from 2 to 6, preferably from 2 to 4, carbon atoms (such as the vinyloxycarbonyl and allyloxycarbonyl groups); and

sulpho groups;

aralkyloxycarbonyl groups, in which the aralkyl part is as defined and exemplified above, and in which the aryl ring, if substituted, preferably has one or two lower alkoxy or nitro substituents (such as the benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups);

alkanoyloxyalkoxycarbonyl and alkenoyloxyalkoxycarbonyl groups, in which the alkanoyl, alkenoyl and alkoxy parts may each be as separately defined and exemplified above, for example the acetoxymethoxycarbonyl, 2-acetoxyethoxycarbonyl, 3-acetoxypropoxycarbonyl, 4-acetoxybutoxycarbonyl, pivaloyloxymethoxycarbonyl, 2-pivaloyloxyethoxycarbonyl, 3-pivaloyloxypropoxycarbonyl, 4-pivaloyloxybutoxycarbonyl, propionyloxymethoxycarbonyl, 2-propionyloxyethoxycarbonyl, 3-propionyloxypropoxycarbonyl and 4-propionyloxybutoxycarbonyl groups, preferably the pivaloyloxymethoxycarbonyl group;

and amino acid residues, for example the glycyl or alanyl groups.

In particular, we especially prefer those groups which are cleaved from the hydroxy group in vivo, to give a compound containing a free hydroxy group, that is to form a pro-drug, and the aliphatic acyl groups.

V represents a group of formula

$$-(CR^e{=}CR^f)_m-(CR^gR^h)_n-$$

where m is 0, 1 or 2, n is 0 or an integer from 1 to 7, provided that (m + n) > 0 and $R^e$, $R^f$, $R^g$ and $R^h$ are independently selected from hydrogen atoms and alkyl groups having from 1 to 4 carbon atoms. Examples of the alkyl groups which may be represented by $R^e$, $R^f$, $R^g$ and $R^h$ include the methyl, ethyl, propyl, isopropyl, butyl, isobutyl, sec-butyl and t-butyl groups, of which the methyl group is preferred. It is preferred either that all of $R^e$, $R^f$, $R^g$ and $R^h$ represent hydrogen atoms or that one represents an alkyl group and the others represent hydrogen atoms.

Where W represents a heterocyclic group having from 3 to 14 ring atoms, one of these is a nitrogen atom through which the heterocyclic group is attached to the group represented by V, from 1 to 3 are additional hetero-atoms selected from nitrogen, oxygen and sulphur hetero-atoms, and the remainder are carbon atoms. When there are 3 such additional hetero-atoms, it is preferred that all three, two or one are nitrogen atoms and, correspondingly, none, one or two are selected from oxygen and sulphur atoms. Where there are two or

one such additional hetero-atoms, they may be chosen freely from nitrogen, oxygen and sulphur hetero-atoms. However, we prefer those groups containing from 1 to 3 nitrogen atoms and optionally one oxygen or sulphur atom. The heterocyclic group may be unsubstituted or it may have a substituent selected from the groups and atoms represented by $R^a$ and/or an oxygen substituent (to form an oxo group). Examples of such heterocyclic groups include: completely saturated nitrogen-containing heterocyclic groups, such as the morpholinyl, thiomorpholinyl, pyrrolidinyl, pyrazolidinyl, piperidyl and piperazinyl groups; partially saturated nitrogen-containing heterocyclic groups, such as the 3-oxoisoxazolyl, 2-oxopyridyl, 4,5-dihydroimidazolyl, tetrahydroisoquinolyl and tetrahydroquinolyl groups; and unsaturated nitrogen- containing heterocyclic groups, such as the uracil, imidazolyl, purinyl and benzimidazolyl groups. Of these, we prefer those completely saturated or partially saturated nitrogen-containing heterocyclic groups having from 6 to 10 ring atoms; and more preferably the 1-tetrahydroquinolyl, 2-tetrahydroquinolyl, 1-piperazinyl and 1-pyrrolidinyl groups; and most preferably the 2-tetrahydroisoquinolyl group.

Where W represents a group of formula (II), where k and l is each 0 or an integer from 1 to 4, we prefer that (k + l) is an integer from 1 to 5. Examples of groups represented by formula (II) include the aziridinyl, pyrrolidinyl, piperidinyl, azepinyl, azocinyl, azoninyl and azecinyl groups, of which we prefer the pyrrolidinyl and piperidinyl groups.

Where Ar represents an aryl group, this may be any of the aryl groups defined and exemplified above, preferably a phenyl group, and these may be unsubstituted or substituted as defined above. Where it represents an aromatic heterocyclic group, this may be any of those groups defined and exemplifeid above for W which are both aromatic and possess from 5 to 7 ring atoms. Preferred examples include the thienyl, furyl, pyridyl and azepinyl groups. These likewise may be unsubstituted or substituted as defined above.

Where $R^4$ or $R^5$ represents an aryl, aralkyl, arylcarbonyl or heterocylic group, these may be as defined and exemplified above in relation to the corresponding groups which may be represented by $R^1$ or $R^2$.

Examples of the groups and atoms represented by $R^b$ and $R^c$ are as given above in relation to the corresponding groups which may be represented by $R^a$.

Examples of the groups represented by $R^3$ and $R^d$ are as given in the corresponding groups included among the hydroxy-protecting groups.

The compounds of the present invention can form salts. Examples of such salts include: salts with an alkali metal, such as sodium, potassium or lithium; salts with an alkaline earth metal, such as barium or calcium; salts with another metal, such as magnesium or aluminium; ammonium salts; organic base salts, such as a salt with triethylamine, diisopropylamine, cyclohexylamine or dicyclohexylamine; and salts with a basic amino acid, such as lysine or arginine. Also, where since compounds of the present invention contain a basic group in their molecules, they can form acid addition salts. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethane- sulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with amino acids, such as glutamic acid or aspartic acid.

The compounds of the present invention may contain several asymmetric carbon atoms in their molecules, depending upon the nature of the groups represented by $R^1$, $R^2$, $R^3$, $R^4$, $R^5$, $R^a$, $R^b$, $R^c$ and $R^d$, and may thus be able to form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

Of the compounds of the present invention in which Z represents a -CH= group, preferred classes of compounds are those compounds of formula (I) in which:

(1) $R^1$ represents a hydrogen atom, an alkyl group having from 1 to 6 carbon atoms, a cycloalkyl group having from 3 to 10 ring carbon atoms, an aryl group as defined above, an aralkyl group as defined above, an alkanoyl group as defined above, an arylcarbonyl group as defined above, an alkoxy group having from 1 to 6 carbon atoms, an alkoxycarbonyl group having from 1 to 6 carbon atoms or a haloalkyl group having from 1 to 6 carbon atoms;

(2) $R^2$ represents a hydrogen atom, an alkyl group having from 1 to 6 carbon atoms, a cycloalkyl group having from 3 to 10 ring carbon atoms, an aryl group as defined above, an aralkyl group as defined above, a halogen atom, an amino group, a mono- or di- alkylamino group as defined above, alkylaminoalkyl groups in which each alkyl part has from 1 to 4 carbon atoms, alkynylaminoalkyl groups in which the alkynyl part

has from 2 to 4 carbon atoms and the alkyl part has from 1 to 4 carbon atoms, an arylamino group as defined above, a nitro group, a cyano group, a sulphonyl group, an alkylsulphonyl group as defined above, a haloalkylsulphonyl group as defined above, an alkanoyl group as defined above, an arylcarbonyl group as defined above, an alkoxy group having from 1 to 6 carbon atoms, an alkoxycarbonyl as defined above or a haloalkyl group having from 1 to 6 carbon atoms;

(3) U represents a group of general formula: -CO- or -CH($OR^3$)- (in which $R^3$ represents a hydrogen atom, an alkanoyl group as defined above, an arylcarbonyl group as defined above, an alkylsulphonyl group having from 1 to 6 carbon atoms or an arylsulphonyl group as defined above);

(4) $R^3$ represents a hydrogen atom or a hydroxy protecting group for a pro-drug;

(5) V represents a group of general formula: $-(CH=CH)_m-(CH_2)_n-$ (in which $\underline{m}$ is 0, 1 or 2, and $\underline{n}$ is 0 or an integer of from 1 to 7);

(6) W represents a nitrogen-containing heterocyclic group as defined above, or a group of formula (II) as defined above, in which $\underline{k}$ and $\underline{l}$ are the same or different and each is 0 or an integer of from 1 to 4; $R^4$ represents a hydrogen atom, an alkyl group having from 1 to 6 carbon atoms, a cycloalkyl group as defined above, an aryl group as defined above, an aralkyl group as defined above, an alkanoyl group as defined above, an arylcarbonyl group as defined above, an alkoxy group having from 1 to 6 carbon atoms, an alkoxycarbonyl group having from 2 to 7 carbon atoms or a haloalkyl group having from 1 to 6 carbon atoms;

(7) W represents a group of formula: $-NR^4R^5$ (in which $R^4$ and $R^5$ are the same or different and each independently represents a hydrogen atom, an alkyl group having from 1 to 6 carbon atoms, a cycloalkyl group as defined above, an aryl group as defined above, an aralkyl group as defined above, an alkanoyl group as defined above, an arylcarbonyl group as defined above, an alkoxy group having from 1 to 6 carbon atoms, an alkoxycarbonyl group having from 2 to 7 carbon atoms or a haloalkyl group having from 1 to 6 carbon atoms, or a saturated or unsaturated heterocyclic group containing a nitrogen atom or atoms as defined above.

Of the compounds of the present invention in which Z represents a -N= group, preferred classes of compounds are those compounds of formula (I) in which:

(1) $R^1$ and $R^2$ are the same or different from each other and each represents:

a hydrogen atom;

an alkyl group having from 1 to 4 carbon atoms;

a cycloalkyl group having from 3 to 10 ring carbon atoms;

a halogen atom;

a sulphonyl group;

an amino group;

a mono- or di- alkylamino group in which the or each aryl part has from 1 to 4 carbon atoms;

an alkylaminoalkyl group in which each alkyl part has from 1 to 4 carbon atoms;

an alkynylaminoalkyl group in which the alkynyl part has from 2 to 4 carbon atoms and the alkyl part has from 1 to 4 carbon atoms;

a nitro group, a cyano group;

an alkanoyl group having from 1 to 4 carbon atoms;

an alkoxy group having from 1 to 4 carbon atoms;

an alkoxycarbonyl group having from 2 to 5 carbon atoms;

a haloalkyl group having from 1 to 4 carbon atoms;

an aryl group having from 6 to 10 ring carbon atoms, which group may be unsubstituted or may have 1 or 2 substituents selected from substituents A:

substituents A are selected from alkyl groups having from 1 to 4 carbon atoms, cycloalkyl groups having from 3 to 6 ring carbon atoms, halogen atoms, mono- or di- alkylamino groups in which the or each alkyl part has from 1 to 4 carbon atoms, alkylsulphonyl groups having from 1 to 4 carbon atoms, haloalkylsulphonyl groups having from 1 to 4 carbon atoms, alkanoyl groups having from 2 to 4 carbon atoms, alkoxy groups having from 1 to 4 carbon atoms, alkoxycarbonyl groups having from 2 to 5 carbon atoms, haloalkyl groups having from 1 to 4 carbon atoms, halogen atoms, amino groups, nitro groups, cyano groups and sulphonyl groups;

an aralkyl group having from 1 to 3 carbon atoms in the alkyl part and from 6 to 10 ring carbon atoms in the aryl part, said group being unsubstituted or substituted by at least one substituent selected from substituents A;

a mono- or di- arylamino group, in which the or each aryl part has from 6 to 10 ring carbon atoms and is unsubstituted or substituted by at least one substituent selected from substituents A;

an arylcarbonyl group, in which the aryl part has from 6 to 10 ring carbon atoms and is unsubstituted or substituted by at least one substituent selected from substituents A; or

an arylalkanoyl group having from 2 to 4 carbon atoms in the alkanoyl part and from 6 to 10 ring carbon atoms in the aryl part, said group being unsubstituted or substituted by at least one substituent selected from substituents A;

(2) U represents a group of general formula: -CO- or -CH($OR^3$)-, in which $R^3$ represents:

a hydrogen atom;

an alkanoyl group having from 2 to 4 carbon atoms;

an alkylsulphonyl group having from 1 to 4 carbon atoms;

an arylcarbonyl group having from 6 to 10 ring carbon atoms in the aryl part, which group may be unsubstituted or may have 1 or 2 substituents selected from substituents B:

substituents B are selected from alkyl groups having from 1 to 4 carbon atoms, alkoxy groups having from 1 to 4 carbon atoms, halogen atoms and nitro groups;

an arylsulphonyl group, in which the aryl part has from 6 to 10 ring carbon atoms and is unsubstituted or substituted by at least one substituent selected from substituents B; or

a hydroxy-protecting group for a pro-drug;

(3) V represents a group of formula:

$$-(CH=CH)_m-(CH_2)_n-$$

in which $\underline{m}$ is 0, 1 or 2, and $\underline{n}$ is 0 or an integer of from 1 to 5, provided that ($\underline{m}$ + $\underline{n}$) > 0;

(4) W represents a partially or completely saturated nitrogen-containing heterocyclic group having from 5 to 10 ring atoms including a nitrogen atom through which it is attached to the group represented by V, preferably a 1-piperidinyl, 1-piperazinyl, 1-morpholinyl, 1-tetrahydroquinolyl or 2-tetrahydroisoquinolyl group;

(5) W represents a group of formula (II) as defined above, wherein $\underline{k}$ and $\underline{l}$ are the same or different from each other and each is an integer from 1 to 3, and the ring nitrogen atom is substituted with $R^4$ as defined above;

(6) W represents a group of formula (II) as defined above having a 5- or 6-membered ring, wherein an ethylene group in the 5- or 6-membered ring is condensed with 1 or 2 benzene rings, and the ring nitrogen atom is substituted with $R^4$ as defined above, preferably a tetrahydroquinolyl, tetrahydroisoquinolyl and acridinyl group;

(7) preferably $R^4$ in the above (5) and (6) represents:

a hydrogen atom;

an alkyl group having from 1 to 4 carbon atoms group;

a cycloalkyl group having from 3 to 10 ring carbon atoms;

an alkanoyl group having from 1 to 4 carbon atoms;

an alkoxy group having from 1 to 4 carbon atoms;

an alkoxycarbonyl group having from 2 to 5 carbon atoms;

an aryl group having from 6 to 10 ring carbon atoms and which is unsubstituted or may have 1 or 2 substituents selected from substituents C:

substituents C are selected from alkyl groups having from 1 to 4 carbon atoms, halogen atoms, haloalkyl groups having from 1 to 4 carbon atoms, alkanoyl groups having 2 or 3 carbon atoms, alkoxy groups having from 1 to 4 carbon atoms, mono- or di- alkylamino groups in which the or each alkyl part has from 1 to 4 carbon atoms, nitro groups and cyano groups;

an arylalkanoyl group having from 2 to 4 carbon atoms in the alkanoyl part and from 6 to 10 ring carbon atoms in the aryl part, said group being unsubstituted or substituted by at least one substituent selected from substituents C;

an arylcarbonyl group, in which the aryl part has from 6 to 10 ring carbon atoms and is unsubstituted or substituted by at least one substituent selected from substituents C;

an aralkyl group having from 1 to 3 carbon atoms in the alkyl part and from 6 to 10 ring carbon atoms in the aryl part, said group being unsubstituted or substituted by at least one substituent selected from substituents C;

a nitrogen-containing heterocyclic group having from 5 to 10 ring atoms, said group being unsubstituted or substituted by at least one substituent selected from substituents C;

and a nitrogen-containing heterocyclic alkyl group, in which the heterocyclic part has from 5 to 10 ring atoms, said group being unsubstituted or substituted by at least one substituent selected from substituents C, and the alkyl part has from 1 to 4 carbon atoms;

(8) W represents a group of formula $-NR^5R^6$, wherein $R^5$ and $R^6$ may be the same or different from each other and each represents:

a hydrogen atom;

an alkyl group having from 1 to 4 carbon atoms group;

a cycloalkyl group having from 3 to 10 ring carbon atoms;

an alkanoyl group having from 1 to 4 carbon atoms;
an alkoxy group having from 1 to 4 carbon atoms;
an aryl group having from 6 to 10 ring carbon atoms which is unsubstituted or may have 1 or 2 substituents selected from substituents D:

substituents D are selected from alkyl groups having from 1 to 4 carbon atoms, halogen atoms, haloalkyl groups having from 1 to 4 carbon atoms, alkanoyl groups having 2 or 3 carbon atoms, alkoxy groups having from 1 to 4 carbon atoms, alkoxylalkyl groups in which the alkyl and alkoxy parts each have from 1 to 4 carbon atoms, nitro groups and cyano groups;

an arylalkanoyl group having from 2 to 4 carbon atom in the alkanoyl part and from 6 to 10 ring carbon atoms in the aryl part, said group being unsubstituted or substituted by at least one substituent selected from substituents D;
an arylcarbonyl group, in which the aryl part has from 6 to 10 ring carbon atom and is unsubstituted or substituted by at least one substituent selected from substituents D;
an aralkyl group having from 1 to 3 carbon atoms in the alkyl part and from 6 to 10 ring carbon atoms in the aryl part, said group being unsubstituted or substituted by at least one substituent selected from substituents D;
a nitrogen-containing heterocyclic group having from 5 to 10 ring atoms, said group being unsubstituted or substituted by at least one substituent selected from substituents D;
and a nitrogen-containing heterocyclic alkyl group, in which the heterocyclic part has from 5 to 10 ring atoms, said group being unsubstituted or substituted by at least one substituent selected from substituents D, and the alkyl part has from 1 to 4 carbon atoms;
an aryloxy group, in which the aryl part has from 6 to 10 ring carbon atoms and is unsubstituted or substituted by at least one substituent selected from substituents D;
and a saturated or unsaturated heterocyclic group having from 5 to 10 ring atoms, such as a piperazinyl, quinolyl, thienyl or furyl group;

More preferred compounds of the present invention are those compounds of formula (I) and salts thereof, in which:

(1) $R^1$ represents:

a hydrogen atom;
a methyl, ethyl, isopropyl or isobutyl group;
a cyclopropyl, cyclobutyl, cyclopentyl or cyclohexyl group;
a trifluoromethyl or 2,2,2-trifluoroethyl group;
an acetyl or propionyl group;
methoxy, ethoxy or isopropyloxy group;
a methoxycarbonyl or ethoxycarbonyl group;
a phenyl group which is unsubstituted or is substituted by 1 or 2 substituents selected from substituents E:

substituents E are selected from methyl, ethyl, isopropyl, cyclopropyl, chlorine, fluorine, bromine, trifluoromethyl, methylamino, dimethylamino, acetyl, propionyl, methoxy, ethoxy, isopropoxy, nitro and cyano groups;

a benzyl group which is unsubstituted or is substituted by 1 or 2 substituents selected from substituents E;
a benzoyl group which is unsubstituted or is substituted by 1 or 2 substituents selected from substituents E;
or a phenylacetyl group which is unsubstituted or is substituted by 1 or 2 substituents selected from substituents E;

(2) $R^2$ represents:

a hydrogen atom;
a methyl, ethyl, isopropyl or isobutyl group;
a cyclopropyl, cyclobutyl, cyclopentyl or cyclohexyl group;
a fluorine, chlorine or bromine atom;
sulphonyl group;
a trifluoromethyl or 2,2,2-trifluoroethyl group;
a methylamino, ethylamino, isopropylamino, dimethylamino, diethylamino or diisopropylamino group;
an alkylaminoalkyl group in which each alkyl part has from 1 to 3 carbon atoms;
an alkynylaminoalkyl group in which the alkynyl part has 3 or 4 carbon atoms and the alkyl part has from 1 to 3 carbon atoms;

an acetyl or propionyl group;
a methoxy, ethoxy or isopropyloxy group;
a methoxycarbonyl or ethoxycarbonyl group;
an amino group;
a nitro group;
a cyano group;
a phenyl group which is unsubstituted or is substituted by 1 or 2 substituents selected from substituents F:

substituents F are selected from methyl, ethyl, isopropyl, cyclopropyl, chlorine, fluorine, bromine, trifluoromethyl, methylamino, dimethylamino, acetyl, propionyl, methoxy, ethoxy, isopropoxy, nitro and cyano groups;

or a benzyl group which is unsubstituted or is substituted by 1 or 2 substituents selected from substituents F;

(3) U represents a group of general formula: -CO- or $-CH(OR^3)-$, in which $R^3$ represents:

a hydrogen atom;
an acetyl, propionyl, methylsulphonyl, ethylsulphonyl, propylsulphonyl, trimethylsilyl, triethylsilyl, methoxycarbonyl or ethoxycarbonyl group;
a benzoyl group which is unsubstituted or is substituted by 1 or 2 substituents selected from substituents G:

substituents G are selected from methyl, ethyl, fluorine, chlorine, bromine, acetyl, methoxy, ethoxy, nitro and cyano groups;

or a phenylsulphonyl group which is unsubstituted or is substituted by 1 or 2 substituents selected from substituents G;

(4) V represents a group of formula:
$-(CH=CH)_m-(CH_2)_n-$ (in which $\underline{m}$ is 0 or 1, and $\underline{n}$ is 0 or an integer from 1 to 3, provided that $(\underline{m} + \underline{n}) > 0$;

(5) W represents a partially or completely saturated nitrogen-containing heterocyclic group having from 6 to 10 ring atom including a nitrogen atom through which it is attached to the group represented by V, such as a 2-tetrahydroisoquinolyl group;

(6) W represents a group of formula (II) as defined above, wherein $\underline{k}$ and $\underline{l}$ are the same or different from each other and each is 1 or 2, and $R^4$ is as defined above, such as a pyrrolidinyl or piperidyl which is unsubstituted or substituted by a group $R^4$;

(7) W represents a group of formula (II) having a 6-membered ring formed by a cyclic alkylene group and a nitrogen atom, wherein the ethylene group in the 6-membered ring is condensed with 1 or 2 benzene rings, such as a 4-(1-tetrahydroquinolyl) group which is unsubstituted or substituted by a group $R^4$;

(8) $R^4$ in the above (6) and (7) represents:

a hydrogen atom;
a methyl, ethyl or isopropyl group;
an adamantyl group;
an acetyl, propionyl or butyryl group;
a methoxy or ethoxy group;
a methoxycarbonyl or ethoxycarbonyl group;
a phenyl group which is unsubstituted or is substituted by 1 or 2 substituents selected from substituents H:

substituents H are selected from methyl, ethyl, fluorine, chlorine, bromine, acetyl, methoxy, ethoxy, dimethylamino, nitro and cyano groups;

an aralkyl group having from 1 to 3 carbon atoms in the alkyl part and from 6 to 10 ring carbon atoms in the aryl part, said group being unsubstituted or substituted by at least one substituent selected from substituents H;

a benzoyl group which is unsubstituted or is substituted by 1 or 2 substituents selected from substituents H;

a 2-thienyl, 3-thienyl, 2-furyl, 3-furyl, 2-indolinyl, 2-imidazolyl or 2-(4,5-dihydro)-imidazolyl group which is unsubstituted or is substituted by 1 or 2 substituents selected from substituents H;

a 2-thenyl, 3-thenyl, 2-furylmethyl, 3-furylmethyl, 2-imidazolylmethyl or 4,5-dihydroimidazol-2-ylmethyl group which is unsubstituted or is substituted by 1 or 2 substituents selected from substituents H;

(9) W represents a group of formula $-NR^5R^6$, wherein $R^5$ and $R^6$ may be the same or different from each other and each represents:

a hydrogen atom;
a methyl, ethyl or isopropyl group;

an adamantyl group;
an acetyl or propionyl group;
a methoxy or ethoxy group;
a methoxycarbonyl or ethoxycarbonyl group;
a phenyl group which is unsubstituted or is substituted by 1 or 2 substituents selected from substituents I:

substituents I are selected from methyl, ethyl, fluorine, chlorine, bromine, acetyl, methoxy, ethoxy, nitro, cyano and dimethylamino groups;

an aralkyl group having from 1 to 3 carbon atoms in the alkyl part and from 6 to 10 ring carbon atoms in the aryl part, said group being unsubstituted or substituted by at least one substituent selected from substituents I;
a benzoyl group which is unsubstituted or is substituted by 1 or 2 substituents selected from substituents I;
a 2-thienyl, 3-thienyl, 2-furyl, 3-furyl, 2-indolinyl, 2-imidazolyl or 2-(4,5-dihydro)-imidazolyl group which is unsubstituted or is substituted by 1 or 2 substituents selected from substituents I;
a 2-thenyl, 3-thenyl, 2-furylmethyl, 3-furylmethyl, 2-imidazolylmethyl or 4,5-dihydroimidazol-2-ylmethyl group which is unsubstituted or is substituted by 1 or 2 substituents selected from substituents I.

Still more preferred compounds of the present invention are those compounds of formula (I) and salts thereof, in which:

(1) $R^1$ represents:
a hydrogen atom;
a methyl, ethyl, isopropyl or isobutyl group;
adamantyl group;
a trifluoromethyl, or 2,2,2-trifluoroethyl group;
a phenyl group;
a phenoxy group;
or a benzyl group;

(2) $R^2$ represents:
a hydrogen atom;
a methyl, ethyl or isopropyl group;
a cyclopropyl group;
an acetyl group;
a methoxy or ethoxy group;
a fluorine, chlorine or bromine atom;
a trifluoromethyl or 2,2,2-trifluoroethyl group;
an amino group;
a methylamino or dimethylamino group;
a methylaminomethyl or ethylaminomethyl group;
a propargylaminomethyl group or a [(2-butynyl)-amino]methyl group;
a nitro group;
a cyano group;
a phenyl group or a benzyl group;

(3) U represents a group of formula: -CO- or -CH($OR^3$)-, wherein $R^3$ represents a hydrogen atom, an acetyl group, a propionyl group or a benzoyl group;

(4) V is a vinyl group, ethylene or a trimethylene group;

(5) W represents a 3-pyrrolidinyl or 4-piperidinyl group having a group $R^4$ at the 1-position, wherein $R^4$ represents:
a hydrogen atom;
a methyl, ethyl or isopropyl group;
a cyclopropyl or adamantyl group;
a phenyl group which is unsubstituted or is substituted by 1 or 2 substituents selected from substituents J:

substituents J are selected from methyl, fluorine, chlorine, acetyl, methoxy, ethoxy, nitro, cyano and dimethylamino groups at the 2-to 5-position thereof;

a benzyl, 2-phenylethyl, 3-propylphenyl or 4-butylphenyl group which is unsubstituted or is substituted by 1 or 2 substituents selected from substituents J;
a benzoyl group which is unsubstituted or is substituted by 1 or 2 substituents selected from substituents J;

a thienyl group which is unsubstituted or is substituted by 1 or 2 substituents selected from methyl, fluorine, chlorine and methoxy groups;

and a 2-indolinyl group which is unsubstituted or is substituted by 1 or 2 substituents selected from methyl, fluorine, chlorine and methoxy groups;

(6) W represents:

a methylamino, ethylamino or propylamino group;

a dimethylamino or diisopropylamino group;

an anilino group which is unsubstituted or is substituted by 1 or 2 substituents selected from substituents K:

substituents K are selected from methyl, fluorine, chlorine, acetyl, methoxy, ethoxy, nitro, cyano and dimethylamino groups at the 2-to 5-positions thereof;

a phenylmethylamino group which is unsubstituted or is substituted by 1 or 2 substituents selected from substituents K;

a benzylamino group which is unsubstituted or is substituted by 1 or 2 substituents selected from substituents K;

a benzylmethylamino group which is unsubstituted or is substituted by 1 or 2 substituents selected from substituents K;

a benzylethylamino group which is unsubstituted or is substituted by 1 or 2 substituents selected from substituents K;

a benzylisopropylamino group which is unsubstituted or is substituted by 1 or 2 substituents selected from substituents K;

or a 2-thenyl group which is unsubstituted or is substituted by 1 or 2 substituents selected from methyl, fluorine, chlorine and methoxy groups.

Most preferred compounds of the present invention are those compounds of formula (I) and salts thereof, in which:

(1) $R^1$ represents a hydrogen atom, a methyl, ethyl, isopropyl or isobutyl group, or a trifluoromethyl group;

(2) $R^2$ represents a hydrogen atom, a methyl or ethyl group, a fluorine or chlorine atom, a methoxy or ethoxyamino group, a dimethylamino group, or a trifluoromethyl or 2,2,2-trifluoroethyl group;

(3) Compounds in which U represents a -CO- group;

(4) Compounds in which V represents a vinyl group or an ethylene group;

(5) Compounds in which W represents a 1-benzyl-3-pyrrolidinyl, 1-phenylmethyl-3-pyrrolidinyl, 1-benzyl-4-piperidinyl or 1-phenylmethyl-4-piperidinyl group, and in which said benzyl or 1-phenylmethyl groups are unsubstituted or are substituted by 1 or 2 substituents selected from methyl, fluorine, chlorine, methoxy, dimethylamino and trifluoromethyl groups at the 2- to 5-positions thereof.

Examples of specific preferred compounds of the present invention are shown in the following formulae (I-1) and (I-2), in which the substituents are as shown in the corresponding ones of Tables 1 and 2, that is Tables 1, 1A and 1B relate to formula (I-1), whilst Tables 2, 2A and 2B relate to formula (I-2).

In the Tables, the following abbreviations are used:

| | |
|---|---|
| Ac | acetyl |
| Adam | adamantyl |
| Boz | benzoyl |
| Bu | butyl |
| Bz | benzyl |
| Et | ethyl |
| .HCl | hydrochloride |
| Ind | indolinyl |
| Me | methyl |
| .Ox | oxalate |
| Ph | phenyl |
| Pip | piperidinyl |
| Pr | propyl |
| Prg | propargyl |
| Pyrd | pyrrolidinyl |
| Quin | quinolyl |
| Then | thenyl |
| Thi | thienyl |
| <u>i</u> | iso |
| tet.hydro | tetrahydro |

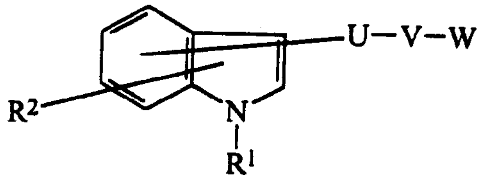

(I-1)

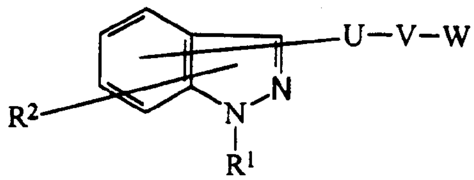

(I-2)

TABLE 1

In the following compounds of formula (I-1), the group -U-V-W is attached to the 3-position of the indole ring.

| Cmpd | $R^1$ | $R^2$ | U | V | W |
|---|---|---|---|---|---|
| 1-1 | Me | H | -CO- | -CH=CH- | 1-Bz-4-Pip- |
| 1-2 | Me | H | -CO- | $-(CH_2)_2-$ | 1-Bz-4-Pip- |
| 1-3 | Me | H | -CO- | $-(CH_2)_2-$ | 1-Bz-4-Pip- .HCl |
| 1-4 | Me | H | -CO- | $-(CH_2)_2-$ | 1-Bz-4-Pip- .Ox |
| 1-5 | Me | 2-Me | -CO- | -CH=CH- | 1-Bz-4-Pip- |
| 1-6 | Me | 2-Me | -CO- | $-(CH_2)_2-$ | 1-Bz-4-Pip- |
| 1-7 | Me | 4-Me | -CO- | -CH=CH- | 1-Bz-4-Pip- |
| 1-8 | Me | 4-Me | -CO- | $-(CH_2)_2-$ | 1-Bz-4-Pip- |
| 1-9 | Me | 5-Me | -CO- | -CH=CH- | 1-Bz-4-Pip- |
| 1-10 | Me | 5-Me | -CO- | $-(CH_2)_2-$ | 1-Bz-4-Pip- |
| 1-11 | Me | 6-Me | -CO- | -CH=CH- | 1-Bz-4-Pip- |
| 1-12 | Me | 6-Me | -CO- | $-(CH_2)_2-$ | 1-Bz-4-Pip- |
| 1-13 | Me | 5-Et | -CO- | -CH=CH- | 1-Bz-4-Pip- |
| 1-14 | Me | 5-Et | -CO- | $-(CH_2)_2-$ | 1-Bz-4-Pip- |
| 1-15 | Me | 6-Et | -CO- | -CH=CH- | 1-Bz-4-Pip- |
| 1-16 | Me | 6-Et | -CO- | $-(CH_2)_2-$ | 1-Bz-4-Pip- |
| 1-17 | Me | 5-iPr | -CO- | -CH=CH- | 1-Bz-4-Pip- |
| 1-18 | Me | 5-iPr | -CO- | $-(CH_2)_2-$ | 1-Bz-4-Pip- |
| 1-19 | Me | 6-iPr | -CO- | -CH=CH- | 1-Bz-4-Pip- |
| 1-20 | Me | 6-iPr | -CO- | $-(CH_2)_2-$ | 1-Bz-4-Pip- |
| 1-21 | Me | 5-OMe | -CO- | -CH=CH- | 1-Bz-4-Pip- |
| 1-22 | Me | 5-OMe | -CO- | $-(CH_2)_2-$ | 1-Bz-4-Pip- |
| 1-23 | Me | 6-OMe | -CO- | -CH=CH- | 1-Bz-4-Pip- |

TABLE 1 CONT'D

| Cmpd | $R^1$ | $R^2$ | U | V | W |
|---|---|---|---|---|---|
| 1-24 | Me | 6-OMe | -CO- | $-(CH_2)_2-$ | 1-Bz-4-Pip- |
| 1-25 | Me | 4-Cl | -CO- | -CH=CH- | 1-Bz-4-Pip- |
| 1-26 | Me | 4-Cl | -CO- | $-(CH_2)_2-$ | 1-Bz-4-Pip- |
| 1-27 | Me | 5-Cl | -CO- | -CH=CH- | 1-Bz-4-Pip- |
| 1-28 | Me | 5-Cl | -CO- | $-(CH_2)_2-$ | 1-Bz-4-Pip- |
| 1-29 | Me | 6-Cl | -CO- | -CH=CH- | 1-Bz-4-Pip- |
| 1-30 | Me | 6-Cl | -CO- | $-(CH_2)_2-$ | 1-Bz-4-Pip- |
| 1-31 | Me | 7-Cl | -CO- | -CH=CH- | 1-Bz-4-Pip- |
| 1-32 | Me | 7-Cl | -CO- | $-(CH_2)_2-$ | 1-Bz-4-Pip- |
| 1-33 | Me | $4-NO_2$ | -CO- | -CH=CH- | 1-Bz-4-Pip- |
| 1-34 | Me | $4-NO_2$ | -CO- | $-(CH_2)_2-$ | 1-Bz-4-Pip- |
| 1-35 | Me | $5-NO_2$ | -CO- | -CH=CH- | 1-Bz-4-Pip- |
| 1-36 | Me | $5-NO_2$ | -CO- | $-(CH_2)_2-$ | 1-Bz-4-Pip- |
| 1-37 | Me | $6-NO_2$ | -CO- | -CH=CH- | 1-Bz-4-Pip- |
| 1-38 | Me | $6-NO_2$ | -CO- | $-(CH_2)_2-$ | 1-Bz-4-Pip- |
| 1-39 | Me | 5-F | -CO- | -CH=CH- | 1-Bz-4-Pip- |
| 1-40 | Me | 5-F | -CO- | $-(CH_2)_2-$ | 1-Bz-4-Pip- |
| 1-41 | Me | 4-F | -CO- | -CH=CH- | 1-Bz-4-Pip- |
| 1-42 | Me | 4-F | -CO- | $-(CH_2)_2-$ | 1-Bz-4-Pip- |
| 1-43 | Me | 6-F | -CO- | -CH=CH- | 1-Bz-4-Pip- |
| 1-44 | Me | 6-F | -CO- | $-(CH_2)_2-$ | 1-Bz-4-Pip- |
| 1-45 | Me | 7-F | -CO- | -CH=CH- | 1-Bz-4-Pip- |
| 1-46 | Me | 7-F | -CO- | $-(CH_2)_2-$ | 1-Bz-4-Pip- |
| 1-47 | Me | 4-CN | -CO- | -CH=CH- | 1-Bz-4-Pip- |
| 1-48 | Me | 4-CN | -CO- | $-(CH_2)_2-$ | 1-Bz-4-Pip- |
| 1-49 | Me | 5-CN | -CO- | -CH=CH- | 1-Bz-4-Pip- |
| 1-50 | Me | 5-CN | -CO- | $-(CH_2)_2-$ | 1-Bz-4-Pip- |

TABLE 1 CONT'D

| Cmpd | $R^1$ | $R^2$ | U | V | W |
|---|---|---|---|---|---|
| 1-51 | Me | 6-CN | -CO- | -CH=CH- | 1-Bz-4-Pip- |
| 1-52 | Me | 6-CN | -CO- | $-(CH_2)_2-$ | 1-Bz-4-Pip- |
| 1-53 | Me | 7-CN | -CO- | -CH=CH- | 1-Bz-4-Pip- |
| 1-54 | Me | 7-CN | -CO- | $-(CH_2)_2-$ | 1-Bz-4-Pip- |
| 1-55 | Me | $2-CF_3$ | -CO- | -CH=CH- | 1-Bz-4-Pip- |
| 1-56 | Me | $2-CF_3$ | -CO- | $-(CH_2)_2-$ | 1-Bz-4-Pip- |
| 1-57 | Me | $4-CF_3$ | -CO- | -CH=CH- | 1-Bz-4-Pip- |
| 1-58 | Me | $4-CF_3$ | -CO- | $-(CH_2)_2-$ | 1-Bz-4-Pip- |
| 1-59 | Me | $5-CF_3$ | -CO- | -CH=CH- | 1-Bz-4-Pip- |
| 1-60 | Me | $5-CF_3$ | -CO- | $-(CH_2)_2-$ | 1-Bz-4-Pip- |
| 1-61 | Me | $6-CF_3$ | -CO- | -CH=CH- | 1-Bz-4-Pip- |
| 1-62 | Me | $6-CF_3$ | -CO- | $-(CH_2)_2-$ | 1-Bz-4-Pip- |
| 1-63 | Me | $7-CF_3$ | -CO- | -CH=CH- | 1-Bz-4-Pip- |
| 1-64 | Me | $7-CF_3$ | -CO- | $-(CH_2)_2-$ | 1-Bz-4-Pip- |
| 1-65 | Me | $2-CH_2CF_3$ | -CO- | -CH=CH- | 1-Bz-4-Pip- |
| 1-66 | Me | $2-CH_2CF_3$ | -CO- | $-(CH_2)_2-$ | 1-Bz-4-Pip- |
| 1-67 | Me | $4-CH_2CF_3$ | -CO- | -CH=CH- | 1-Bz-4-Pip- |
| 1-68 | Me | $4-CH_2CF_3$ | -CO- | $-(CH_2)_2-$ | 1-Bz-4-Pip- |
| 1-69 | Me | $5-CH_2CF_3$ | -CO- | -CH=CH- | 1-Bz-4-Pip- |
| 1-70 | Me | $5-CH_2CF_3$ | -CO- | $-(CH_2)_2-$ | 1-Bz-4-Pip- |
| 1-71 | Me | $6-CH_2CF_3$ | -CO- | -CH=CH- | 1-Bz-4-Pip- |
| 1-72 | Me | $6-CH_2CF_3$ | -CO- | $-(CH_2)_2-$ | 1-Bz-4-Pip- |
| 1-73 | Me | $7-CH_2CF_3$ | -CO- | -CH=CH- | 1-Bz-4-Pip- |
| 1-74 | Me | $7-CH_2CF_3$ | -CO- | $-(CH_2)_2-$ | 1-Bz-4-Pip- |
| 1-75 | Me | H | -CO- | -CH=CH- | 1-(2-F-Bz)-4-Pip- |
| 1-76 | Me | H | -CO- | $-(CH_2)_2-$ | 1-(2-F-Bz)-4-Pip- |
| 1-77 | Me | H | -CO- | -CH=CH- | 1-(3-F-Bz)-4-Pip- |
| 1-78 | Me | H | -CO- | $-(CH_2)_2-$ | 1-(3-F-Bz)-4-Pip- |

TABLE 1 CONT'D

| Cmpd | $R^1$ | $R^2$ | U | V | W |
|---|---|---|---|---|---|
| 1-79 | Me | H | -CO- | -CH=CH- | 1-(4-F-Bz)-4-Pip- |
| 1-80 | Me | H | -CO- | $-(CH_2)_2-$ | 1-(4-F-Bz)-4-Pip- |
| 1-81 | Me | H | -CO- | -CH=CH- | 1-(2-Cl-Bz)-4-Pip- |
| 1-82 | Me | H | -CO- | $-(CH_2)_2-$ | 1-(2-Cl-Bz)-4-Pip- |
| 1-83 | Me | H | -CO- | -CH=CH- | 1-(3-Cl-Bz)-4-Pip- |
| 1-84 | Me | H | -CO- | $-(CH_2)_2-$ | 1-(3-Cl-Bz)-4-Pip- |
| 1-85 | Me | H | -CO- | $-(CH_2)_2-$ | 1-(3-Cl-Bz)-4-Pip- .HCl |
| 1-86 | Me | H | -CO- | -CH=CH- | 1-(4-Cl-Bz)-4-Pip- |
| 1-87 | Me | H | -CO- | $-(CH_2)_2-$ | 1-(4-Cl-Bz)-4-Pip- |
| 1-88 | Me | H | -CO- | -CH=CH- | 1-(3-Br-Bz)-4-Pip- |
| 1-89 | Me | H | -CO- | $-(CH_2)_2-$ | 1-(3-Br-Bz)-4-Pip- |
| 1-90 | Me | H | -CO- | -CH=CH- | 1-(4-Br-Bz)-4-Pip- |
| 1-91 | Me | H | -CO- | $-(CH_2)_2-$ | 1-(4-Br-Bz)-4-Pip- |
| 1-92 | Me | H | -CO- | -CH=CH- | 1-(3-I-Bz)-4-Pip- |
| 1-93 | Me | H | -CO- | $-(CH_2)_2-$ | 1-(3-I-Bz)-4-Pip- |
| 1-94 | Me | H | -CO- | -CH=CH- | 1-(2-Me-Bz)-4-Pip- |
| 1-95 | Me | H | -CO- | $-(CH_2)_2-$ | 1-(2-Me-Bz)-4-Pip- |
| 1-96 | Me | H | -CO- | -CH=CH- | 1-(3-Me-Bz)-4-Pip- |
| 1-97 | Me | H | -CO- | $-(CH_2)_2-$ | 1-(3-Me-Bz)-4-Pip- |
| 1-98 | Me | H | -CO- | -CH=CH- | 1-(4-Me-Bz)-4-Pip- |
| 1-99 | Me | H | -CO- | $-(CH_2)_2-$ | 1-(4-Me-Bz)-4-Pip- |
| 1-100 | Me | H | -CO- | -CH=CH- | 1-(3-Et-Bz)-4-Pip- |
| 1-101 | Me | H | -CO- | $-(CH_2)_2-$ | 1-(3-Et-Bz)-4-Pip- |
| 1-102 | Me | H | -CO- | -CH=CH- | 1-(3-MeO-Bz)-4-Pip- |
| 1-103 | Me | H | -CO- | $-(CH_2)_2-$ | 1-(3-MeO-Bz)-4-Pip- |
| 1-104 | Me | H | -CO- | -CH=CH- | 1-(4-MeO-Bz)-4-Pip- |
| 1-105 | Me | H | -CO- | $-(CH_2)_2-$ | 1-(4-MeO-Bz)-4-Pip- |

TABLE 1 CONT'D

| Cmpd | $R^1$ | $R^2$ | U | V | W |
|---|---|---|---|---|---|
| 1-106 | Me | H | -CO- | -CH=CH- | 1-(2-$CF_3$-Bz)-4-Pip- |
| 1-107 | Me | H | -CO- | -$(CH_2)_2$- | 1-(2-$CF_3$-Bz)-4-Pip- |
| 1-108 | Me | H | -CO- | -CH=CH- | 1-(3-$CF_3$-Bz)-4-Pip- |
| 1-109 | Me | H | -CO- | -$(CH_2)_2$- | 1-(3-$CF_3$-Bz)-4-Pip- |
| 1-110 | Me | H | -CO- | -$(CH_2)_2$- | 1-(3-$CF_3$-Bz)-4-Pip-.HCl |
| 1-111 | Me | H | -CO- | -CH=CH- | 1-(4-$CF_3$-Bz)-4-Pip- |
| 1-112 | Me | H | -CO- | -$(CH_2)_2$- | 1-(4-$CF_3$-Bz)-4-Pip- |
| 1-113 | Me | H | -CO- | -CH=CH- | 1-(3-CN-Bz)-4-Pip- |
| 1-114 | Me | H | -CO- | -$(CH_2)_2$- | 1-(3-CN-Bz)-4-Pip- |
| 1-115 | Me | H | -CO- | -CH=CH- | 1-(2-ThiMe)-4-Pip- |
| 1-116 | Me | H | -CO- | -$(CH_2)_2$- | 1-(2-ThiMe)-4-Pip- |
| 1-117 | Me | 5-F | -CO- | -CH=CH- | 1-(2-ThiMe)-4-Pip- |
| 1-118 | Me | 5-F | -CO- | -$(CH_2)_2$- | 1-(2-ThiMe)-4-Pip- |
| 1-119 | Me | 5-Cl | -CO- | -CH=CH- | 1-(2-ThiMe)-4-Pip- |
| 1-120 | Me | 5-Cl | -CO- | -$(CH_2)_2$- | 1-(2-ThiMe)-4-Pip- |
| 1-121 | Me | 5-Me | -CO- | -CH=CH- | 1-(2-ThiMe)-4-Pip- |
| 1-122 | Me | 5-Me | -CO- | -$(CH_2)_2$- | 1-(2-ThiMe)-4-Pip- |
| 1-123 | Me | 5-CN | -CO- | -CH=CH- | 1-(2-ThiMe)-4-Pip- |
| 1-124 | Me | 5-CN | -CO- | -$(CH_2)_2$- | 1-(2-ThiMe)-4-Pip- |
| 1-125 | Et | H | -CO- | -CH=CH- | 1-(2-ThiMe)-4-Pip- |
| 1-126 | Et | H | -CO- | -$(CH_2)_2$- | 1-(2-ThiMe)-4-Pip- |
| 1-127 | Et | 5-F | -CO- | -CH=CH- | 1-(2-ThiMe)-4-Pip- |
| 1-128 | Et | 5-F | -CO- | -$(CH_2)_2$- | 1-(2-ThiMe)-4-Pip- |
| 1-129 | Et | 5-Cl | -CO- | -CH=CH- | 1-(2-ThiMe)-4-Pip- |
| 1-130 | Et | 5-Cl | -CO- | -$(CH_2)_2$- | 1-(2-ThiMe)-4-Pip- |
| 1-131 | Et | 5-Me | -CO- | -CH=CH- | 1-(2-ThiMe)-4-Pip- |
| 1-132 | Et | 5-Me | -CO- | -$(CH_2)_2$- | 1-(2-ThiMe)-4-Pip- |

TABLE 1 CONT'D

| Cmpd | $R^1$ | $R^2$ | U | V | W |
|---|---|---|---|---|---|
| 1-133 | Et | 5-CN | -CO- | -CH=CH- | 1-(2-ThiMe)-4-Pip- |
| 1-134 | Et | 5-CN | -CO- | $-(CH_2)_2-$ | 1-(2-ThiMe)-4-Pip- |
| 1-135 | iPr | H | -CO- | -CH=CH- | 1-(2-ThiMe)-4-Pip- |
| 1-136 | iPr | H | -CO- | $-(CH_2)_2-$ | 1-(2-ThiMe)-4-Pip- |
| 1-137 | iPr | 5-F | -CO- | -CH=CH- | 1-(2-ThiMe)-4-Pip- |
| 1-138 | iPr | 5-F | -CO- | $-(CH_2)_2-$ | 1-(2-ThiMe)-4-Pip- |
| 1-139 | iPr | 5-Cl | -CO- | -CH=CH- | 1-(2-ThiMe)-4-Pip- |
| 1-140 | iPr | 5-Cl | -CO- | $-(CH_2)_2-$ | 1-(2-ThiMe)-4-Pip- |
| 1-141 | iPr | 5-Me | -CO- | -CH=CH- | 1-(2-ThiMe)-4-Pip- |
| 1-142 | iPr | 5-Me | -CO- | $-(CH_2)_2-$ | 1-(2-ThiMe)-4-Pip- |
| 1-143 | iPr | 5-CN | -CO- | -CH=CH- | 1-(2-ThiMe)-4-Pip- |
| 1-144 | iPr | 5-CN | -CO- | $-(CH_2)_2-$ | 1-(2-ThiMe)-4-Pip- |
| 1-145 | Me | H | -CO- | -CH=CH- | 1-Me-4-Pip- |
| 1-146 | Me | H | -CO- | $-(CH_2)_2-$ | 1-Me-4-Pip- |
| 1-147 | Me | H | -CO- | $-(CH_2)_2-$ | 1-Me-4-Pip- .HCl |
| 1-148 | Me | H | -CO- | -CH=CH- | 1-Et-4-Pip- |
| 1-149 | Me | H | -CO- | $-(CH_2)_2-$ | 1-Et-4-Pip- |
| 1-150 | Me | H | -CO- | -CH=CH- | 1-iPr-4-Pip- |
| 1-151 | Me | H | -CO- | $-(CH_2)_2-$ | 1-iPr-4-Pip- |
| 1-152 | Me | H | -CO- | -CH=CH- | 1-iBu-4-Pip- |
| 1-153 | Me | H | -CO- | $-(CH_2)_2-$ | 1-iBu-4-Pip- |
| 1-154 | Me | H | -CO- | -CH=CH- | $1-CF_3-4-Pip-$ |
| 1-155 | Me | H | -CO- | $-(CH_2)_2-$ | $1-CF_3-4-Pip-$ |
| 1-156 | Me | H | -CO- | -CH=CH- | $1-CH_2CF_3-4-Pip-$ |
| 1-157 | Me | H | -CO- | $-(CH_2)_2-$ | $1-CH_2CF_3-4-Pip-$ |
| 1-158 | Et | H | -CO- | -CH=CH- | 1-Bz-4-Pip- |
| 1-159 | Et | H | -CO- | $-(CH_2)_2-$ | 1-Bz-4-Pip- |
| 1-160 | Et | 2-Me | -CO- | -CH=CH- | 1-Bz-4-Pip- |

TABLE 1 CONT'D

| Cmpd | $R^1$ | $R^2$ | U | V | W |
|---|---|---|---|---|---|
| 1-161 | Et | 2-Me | -CO- | $-(CH_2)_2-$ | 1-Bz-4-Pip- |
| 1-162 | Et | 5-Me | -CO- | -CH=CH- | 1-Bz-4-Pip- |
| 1-163 | Et | 5-Me | -CO- | $-(CH_2)_2-$ | 1-Bz-4-Pip- |
| 1-164 | Et | 6-Me | -CO- | -CH=CH- | 1-Bz-4-Pip- |
| 1-165 | Et | 6-Me | -CO- | $-(CH_2)_2-$ | 1-Bz-4-Pip- |
| 1-166 | Et | 2-F | -CO- | -CH=CH- | 1-Bz-4-Pip- |
| 1-167 | Et | 2-F | -CO- | $-(CH_2)_2-$ | 1-Bz-4-Pip- |
| 1-168 | Et | 4-F | -CO- | -CH=CH- | 1-Bz-4-Pip- |
| 1-169 | Et | 4-F | -CO- | $-(CH_2)_2-$ | 1-Bz-4-Pip- |
| 1-170 | Et | 5-F | -CO- | -CH=CH- | 1-Bz-4-Pip- |
| 1-171 | Et | 5-F | -CO- | $-(CH_2)_2-$ | 1-Bz-4-Pip- |
| 1-172 | Et | 6-F | -CO- | -CH=CH- | 1-Bz-4-Pip- |
| 1-173 | Et | 6-F | -CO- | $-(CH_2)_2-$ | 1-Bz-4-Pip- |
| 1-174 | Et | 2-Cl | -CO- | -CH=CH- | 1-Bz-4-Pip- |
| 1-175 | Et | 2-Cl | -CO- | $-(CH_2)_2-$ | 1-Bz-4-Pip- |
| 1-176 | Et | 4-Cl | -CO- | -CH=CH- | 1-Bz-4-Pip- |
| 1-177 | Et | 4-Cl | -CO- | $-(CH_2)_2-$ | 1-Bz-4-Pip- |
| 1-178 | Et | 5-Cl | -CO- | -CH=CH- | 1-Bz-4-Pip- |
| 1-179 | Et | 5-Cl | -CO- | $-(CH_2)_2-$ | 1-Bz-4-Pip- |
| 1-180 | Et | 6-Cl | -CO- | -CH=CH- | 1-Bz-4-Pip- |
| 1-181 | Et | 6-Cl | -CO- | $-(CH_2)_2-$ | 1-Bz-4-Pip- |
| 1-182 | Et | 5-Br | -CO- | -CH=CH- | 1-Bz-4-Pip- |
| 1-183 | Et | 5-Br | -CO- | $-(CH_2)_2-$ | 1-Bz-4-Pip- |
| 1-184 | Et | 6-Br | -CO- | -CH=CH- | 1-Bz-4-Pip- |
| 1-185 | Et | 6-Br | -CO- | $-(CH_2)_2-$ | 1-Bz-4-Pip- |
| 1-186 | Et | 5-I | -CO- | -CH=CH- | 1-Bz-4-Pip- |
| 1-187 | Et | 5-I | -CO- | $-(CH_2)_2-$ | 1-Bz-4-Pip- |
| 1-188 | Et | 6-I | -CO- | -CH=CH- | 1-Bz-4-Pip- |

TABLE 1 CONT'D

| Cmpd | $R^1$ | $R^2$ | U | V | W |
|---|---|---|---|---|---|
| 1-189 | Et | 6-I | -CO- | $-(CH_2)_2-$ | 1-Bz-4-Pip- |
| 1-190 | Et | 5-CN | -CO- | -CH=CH- | 1-Bz-4-Pip- |
| 1-191 | Et | 5-CN | -CO- | $-(CH_2)_2-$ | 1-Bz-4-Pip- |
| 1-192 | Et | 6-CN | -CO- | -CH=CH- | 1-Bz-4-Pip- |
| 1-193 | Et | 6-CN | -CO- | $-(CH_2)_2-$ | 1-Bz-4-Pip- |
| 1-194 | Et | $5-NO_2$ | -CO- | -CH=CH- | 1-Bz-4-Pip- |
| 1-195 | Et | $5-NO_2$ | -CO- | $-(CH_2)_2-$ | 1-Bz-4-Pip- |
| 1-196 | iPr | H | -CO- | -CH=CH- | 1-Bz-4-Pip- |
| 1-197 | iPr | H | -CO- | $-(CH_2)_2-$ | 1-Bz-4-Pip- |
| 1-198 | iPr | 5-F | -CO- | -CH=CH- | 1-Bz-4-Pip- |
| 1-199 | iPr | 5-F | -CO- | $-(CH_2)_2-$ | 1-Bz-4-Pip- |
| 1-200 | iPr | 5-Cl | -CO- | -CH=CH- | 1-Bz-4-Pip- |
| 1-201 | iPr | 5-Cl | -CO- | $-(CH_2)_2-$ | 1-Bz-4-Pip- |
| 1-202 | iPr | 5-Br | -CO- | -CH=CH- | 1-Bz-4-Pip- |
| 1-203 | iPr | 5-Br | -CO- | $-(CH_2)_2-$ | 1-Bz-4-Pip- |
| 1-204 | iPr | 5-CN | -CO- | -CH=CH- | 1-Bz-4-Pip- |
| 1-205 | iPr | 5-CN | -CO- | $-(CH_2)_2-$ | 1-Bz-4-Pip- |
| 1-206 | iPr | $5-NO_2$ | -CO- | -CH=CH- | 1-Bz-4-Pip- |
| 1-207 | iPr | $5-NO_2$ | -CO- | $-(CH_2)_2-$ | 1-Bz-4-Pip- |
| 1-208 | iBu | H | -CO- | -CH=CH- | 1-Bz-4-Pip- |
| 1-209 | iBu | H | -CO- | $-(CH_2)_2-$ | 1-Bz-4-Pip- |
| 1-210 | iBu | 5-Cl | -CO- | -CH=CH- | 1-Bz-4-Pip- |
| 1-211 | iBu | 5-Cl | -CO- | $-(CH_2)_2-$ | 1-Bz-4-Pip- |
| 1-212 | iBu | 5-CN | -CO- | -CH=CH- | 1-Bz-4-Pip- |
| 1-213 | iBu | 5-CN | -CO- | $-(CH_2)_2-$ | 1-Bz-4-Pip- |
| 1-214 | iBu | $5-NO_2$ | -CO- | -CH=CH- | 1-Bz-4-Pip- |
| 1-215 | iBu | $5-NO_2$ | -CO- | $-(CH_2)_2-$ | 1-Bz-4-Pip- |
| 1-216 | COOBz | H | -CO- | -CH=CH- | 1-Bz-4-Pip- |

TABLE 1 CONT'D

| Cmpd | $R^1$ | $R^2$ | U | V | W |
|---|---|---|---|---|---|
| 1-217 | COOBz | H | -CO- | $-(CH_2)_2-$ | 1-Bz-4-Pip- |
| 1-218 | H | H | -CO- | $-(CH_2)_2-$ | 1-Bz-4-Pip- |
| 1-219 | Me | H | -CO- | -CH=CH- | 1-Bz-3-Pyrd- |
| 1-220 | Me | H | -CO- | $-(CH_2)_2-$ | 1-Bz-3-Pyrd- |
| 1-221 | Me | H | -CO- | -CH=CH- | 1-PhEt-4-Pip- |
| 1-222 | Me | H | -CO- | $-(CH_2)_2-$ | 1-PhEt-4-Pip- |
| 1-223 | Me | 5-Cl | -CO- | -CH=CH- | 1-PhEt-4-Pip- |
| 1-224 | Me | 5-Cl | -CO- | $-(CH_2)_2-$ | 1-PhEt-4-Pip- |
| 1-225 | Me | 6-Cl | -CO- | -CH=CH- | 1-PhEt-4-Pip- |
| 1-226 | Me | 6-Cl | -CO- | $-(CH_2)_2-$ | 1-PhEt-4-Pip- |
| 1-227 | Me | 5-OMe | -CO- | -CH=CH- | 1-PhEt-4-Pip- |
| 1-228 | Me | 5-OMe | -CO- | $-(CH_2)_2-$ | 1-PhEt-4-Pip- |
| 1-229 | Me | 5-OMe | -CO- | $-(CH_2)_2-$ | 1-PhEt-4-Pip- .HCl |
| 1-230 | Me | H | -CO- | -CH=CH- | 1-(2-F-Ph)Et-4-Pip- |
| 1-231 | Me | H | -CO- | $-(CH_2)_2-$ | 1-(2-F-Ph)Et-4-Pip- |
| 1-232 | Me | H | -CO- | -CH=CH- | 1-(3-F-Ph)Et-4-Pip- |
| 1-233 | Me | H | -CO- | $-(CH_2)_2-$ | 1-(3-F-Ph)Et-4-Pip- |
| 1-234 | Me | H | -CO- | -CH=CH- | 1-(4-F-Ph)Et-4-Pip- |
| 1-235 | Me | H | -CO- | $-(CH_2)_2-$ | 1-(4-F-Ph)Et-4-Pip- |
| 1-236 | Me | H | -CO- | -CH=CH- | 1-(2-Cl-Ph)Et-4-Pip- |
| 1-237 | Me | H | -CO- | $-(CH_2)_2-$ | 1-(2-Cl-Ph)Et-4-Pip- |
| 1-238 | Me | H | -CO- | -CH=CH- | 1-(3-Cl-Ph)Et-4-Pip- |
| 1-239 | Me | H | -CO- | $-(CH_2)_2-$ | 1-(3-Cl-Ph)Et-4-Pip- |
| 1-240 | Me | H | -CO- | $-(CH_2)_2-$ | 1-(3-Cl-Ph)Et-4-Pip- .HCl |
| 1-241 | Me | H | -CO- | -CH=CH- | 1-(4-Cl-Ph)Et-4-Pip- |
| 1-242 | Me | H | -CO- | $-(CH_2)_2-$ | 1-(4-Cl-Ph)Et-4-Pip- |

TABLE 1 CONT'D

| Cmpd | $R^1$ | $R^2$ | U | V | W |
|---|---|---|---|---|---|
| 1-243 | Me | H | -CO- | $-(CH_2)_2-$ | 1-(4-Cl-Ph)Et-4-Pip- .HCl |
| 1-244 | Me | H | -CO- | -CH=CH- | 1-(3-$CF_3$-Ph)Et-4-Pip- |
| 1-245 | Me | H | -CO- | $-(CH_2)_2-$ | 1-(3-$CF_3$-Ph)Et-4-Pip- |
| 1-246 | Me | H | -CO- | $-(CH_2)_2-$ | 1-(3-$CF_3$-Ph)Et-4-Pip- .HCl |
| 1-247 | Et | H | -CO- | -CH=CH- | 1-PhEt-4-Pip- |
| 1-248 | Et | H | -CO- | $-(CH_2)_2-$ | 1-PhEt-4-Pip- |
| 1-249 | Et | H | -CO- | $-(CH_2)_2-$ | 1-PhEt-4-Pip- .HCl |
| 1-250 | Me | H | -CO- | $-(CH_2)_5-$ | Bz(Me)N- |
| 1-251 | Me | H | -CO- | $-(CH_2)_5-$ | Bz(Et)N- |
| 1-252 | Me | H | -CO- | $-(CH_2)_5-$ | Bz(iPr)N- |
| 1-253 | Et | H | -CO- | $-(CH_2)_5-$ | Bz(Et)N- |
| 1-254 | iPr | H | -CO- | $-(CH_2)_5-$ | Bz(Et)N- |
| 1-255 | H | H | -CO- | -CH=CH- | 2-tet.hydro-iQuin- |
| 1-256 | H | H | -CO- | $-(CH_2)_2-$ | 2-tet.hydro-iQuin- |
| 1-257 | Me | H | -CO- | -CH=CH- | 2-tet.hydro-iQuin- |
| 1-258 | Me | H | -CO- | $-(CH_2)_2-$ | 2-tet.hydro-iQuin- |
| 1-259 | Et | H | -CO- | -CH=CH- | 2-tet.hydro-iQuin- |
| 1-260 | Et | H | -CO- | $-(CH_2)_2-$ | 2-tet.hydro-iQuin- |
| 1-261 | Me | H | -CO- | $-(CH_2)_5-$ | 2-tet.hydro-iQuin- |
| 1-262 | Me | H | -CO- | $-(CH_2)_5-$ | 2-tet.hydro-iQuin- .HCl |
| 1-263 | Me | H | -CO- | -CH=CH- | 2-Ind- |
| 1-264 | Me | H | -CO- | $-(CH_2)_2-$ | 2-Ind- |
| 1-265 | Me | H | -CO- | $-(CH_2)_5-$ | 2-Ind- |
| 1-266 | Me | H | -CO- | $-(CH_2)_5-$ | 2-Ind- .HCl |
| 1-267 | Me | H | -CO- | $-(CH_2)_5-$ | PhEtNH- |

TABLE 1 CONT'D

| Cmpd | $R^1$ | $R^2$ | U | V | W |
|---|---|---|---|---|---|
| 1-268 | Me | H | -CO- | $-(CH_2)_5-$ | PhEtNH- .HCl |
| 1-269 | Me | H | -CO- | $-(CH_2)_5-$ | 2-(3,4-diMeOBz- |
| 1-270 | Me | H | -CO- | $-(CH_2)_5-$ | 2-(3,4-diMeOBz .HCl- |
| 1-271 | Me | H | -CO- | $-CH_2-$ | 4-[Bz(Me)N]-Ph- |
| 1-272 | Me | H | -CO- | $-CH_2-$ | 4-[Bz(Me)N]-Ph- .HCl |
| 1-273 | Me | H | -CO- | $-(CH_2)_2-$ | 4-Pip- |
| 1-274 | Me | H | -CO- | $-(CH_2)_2-$ | 4-Pip- .HBr |
| 1-275 | Me | H | -CHOH- | $-(CH_2)_2-$ | 1-Bz-4-Pip- |
| 1-276 | Et | H | -CHOH- | $-(CH_2)_2-$ | 1-Bz-4-Pip- |
| 1-277 | iPr | H | -CHOH- | $-(CH_2)_2-$ | 1-Bz-4-Pip- |
| 1-278 | iBu | H | -CHOH- | $-(CH_2)_2-$ | 1-Bz-4-Pip- |
| 1-279 | Me | H | -CHOAc- | $-(CH_2)_2-$ | 1-Bz-4-Pip- |
| 1-280 | Et | H | -CHOAc- | $-(CH_2)_2-$ | 1-Bz-4-Pip- |
| 1-281 | iPr | H | -CHOAc- | $-(CH_2)_2-$ | 1-Bz-4-Pip- |
| 1-282 | iBu | H | -CHOAc- | $-(CH_2)_2-$ | 1-Bz-4-Pip- |
| 1-283 | Me | H | -CHOBoz- | $-(CH_2)_2-$ | 1-Bz-4-Pip- |
| 1-284 | Et | H | -CHOBoz- | $-(CH_2)_2-$ | 1-Bz-4-Pip- |
| 1-285 | Me | H | -CO- | $-(CH_2)_5-$ | 4-MeBzNH- .HCl |
| 1-286 | Me | H | -CO- | $-(CH_2)_5-$ | Bz(Et)N- .HCl |
| 1-287 | Me | H | -CO- | $-(CH_2)_5-$ | PhEt(Me)N- |
| 1-288 | Me | H | -CO- | $-(CH_2)_5-$ | PhEt(Me)N- .HCl |
| 1-289 | Me | H | -CO- | $-(CH_2)_5-$ | $AdamCH_2NH-$ |
| 1-290 | Me | H | -CO- | $-(CH_2)_5-$ | $AdamCH_2NH-$ .HCl |
| 1-291 | Me | H | -CO- | $-(CH_2)_5-$ | 3,4-diMeOBzNH- |
| 1-292 | Me | H | -CO- | $-(CH_2)_5-$ | 3,4-diMeOBzNH- .HCl |
| 1-293 | Me | H | -CO- | $-(CH_2)_5-$ | 3,4-diMeOBz(Me)N- |
| 1-294 | Me | H | -CO- | $-(CH_2)_5-$ | 3,4-diMeOBz(Me)N- .HCl |
| 1-295 | Me | H | -CO- | $-(CH_2)_5-$ | 2,4-diClBzNH- |

TABLE 1 CONT'D

| Cmpd | $R^1$ | $R^2$ | U | V | W |
|---|---|---|---|---|---|
| 1-296 | Me | H | -CO- | $-(CH_2)_5-$ | 2,4-diClBzNH- .HCl |
| 1-297 | Me | H | -CO- | $-(CH_2)_5-$ | 2,4-diClBz(Me)N- |
| 1-298 | Me | H | -CO- | $-(CH_2)_5-$ | 2,4-diClBz(Me)N- .HCl |
| 1-299 | Me | 5-Cl | -CO- | $-(CH_2)_2-$ | 1-(2-Cl-Bz)-4-Pip- |
| 1-300 | Me | 5-Cl | -CO- | -CH=CH- | 1-(2-Cl-Bz)-4-Pip- |
| 1-301 | Me | 5-Cl | -CO- | $-(CH_2)_2-$ | 1-(2-Cl-Bz)-4-Pip- .HCl |
| 1-302 | Me | 5-Cl | -CO- | $-(CH_2)_2-$ | 1-(3-Cl-Bz)-4-Pip- |
| 1-303 | Me | 5-Cl | -CO- | -CH=CH- | 1-(3-Cl-Bz)-4-Pip- |
| 1-304 | Me | 5-Cl | -CO- | $-(CH_2)_2-$ | 1-(3-Cl-Bz)-4-Pip- .HCl |
| 1-305 | Me | 5-Cl | -CO- | $-(CH_2)_2-$ | 1-(4-Cl-Bz)-4-Pip- |
| 1-306 | Me | 5-Cl | -CO- | -CH=CH- | 1-(4-Cl-Bz)-4-Pip- |
| 1-307 | Me | 5-Cl | -CO- | $-(CH_2)_2-$ | 1-(4-Cl-Bz)-4-Pip- .HCl |
| 1-308 | Me | 5-Cl | -CO- | -CH=CH- | 1-(3-F-Bz)-4-Pip- |
| 1-309 | Me | 5-Cl | -CO- | $-(CH_2)_2-$ | 1-(3-F-Bz)-4-Pip- |
| 1-310 | Me | 5-F | -CO- | -CH=CH- | 1-(2-F-Bz)-4-Pip- |
| 1-311 | Me | 5-F | -CO- | $-(CH_2)_2-$ | 1-(2-F-Bz)-4-Pip- |
| 1-312 | Me | 5-F | -CO- | -CH=CH- | 1-(3-F-Bz)-4-Pip- |
| 1-313 | Me | 5-F | -CO- | $-(CH_2)_2-$ | 1-(3-F-Bz)-4-Pip- |
| 1-314 | Me | 5-F | -CO- | $-(CH_2)_2-$ | 1-(3-F-Bz)-4-Pip- .HCl |
| 1-315 | Me | 5-F | -CO- | $-(CH_2)_2-$ | 1-(3-F-Bz)-4-Pip- .Ox |
| 1-316 | Me | 5-F | -CO- | $-(CH_2)_2-$ | 1-(3-F-Bz)-4-Pip- $.MeSO_3H$ |
| 1-317 | Me | 5-F | -CO- | -CH=CH- | 1-(4-F-Bz)-4-Pip- |
| 1-318 | Me | 5-F | -CO- | $-(CH_2)_2-$ | 1-(4-F-Bz)-4-Pip- |
| 1-319 | Me | 5-F | -CO- | -CH=CH- | (3,4-diMeOBz)-4-Pip- |
| 1-320 | Me | 5-F | -CO- | $-(CH_2)_2-$ | (3,4-diMeOBz)-4-Pip- |
| 1-321 | Me | 5-F | -CO- | $-(CH_2)_2-$ | 1-PhEt-4-Pip- .HCl |
| 1-322 | Me | 5-F | -CO- | -CH=CH- | 1-[(3-F-Ph)Et]-4-Pip- |

TABLE 1 CONT'D

| Cmpd | $R^1$ | $R^2$ | U | V | W |
|---|---|---|---|---|---|
| 1-323 | Me | 5-F | -CO- | $-(CH_2)_2-$ | 1-[(3-F-Ph)Et]-4-Pip |
| 1-324 | Me | 5-F | -CO- | $-(CH_2)_2-$ | 1-[(3-F-Ph)Et]-4-Pip |
| 1-325 | Me | H | -CO- | $-(CH_2)_5-$ | 2-(2,4-diMeOBz)- |
| 1-326 | Me | H | -CO- | $-(CH_2)_5-$ | 2-(2,4-diMeOBz)- |
| 1-327 | Me | H | -CO- | $-C(CH_3)=CH-$ | 1-Bz-4-Pip- |
| 1-328 | Me | H | -CO- | $-CH(CH_3)CH_2-$ | 1-Bz-4-Pip- |
| 1-329 | Me | 5-Cl | -CO- | $-(CH_2)_2-$ | 1-Bz-4-Pip . HCl |
| 1-330 | Me | H | -CO- | -CH=CH- | 1-(3,4-diMeOBz)-4-Pi |
| 1-331 | Me | H | -CO- | $-(CH_2)_2-$ | 1-(3,4-diMeOBz)-4-Pi |
| 1-332 | Me | H | -CO- | -CH=CH- | 1-(2-Thi)-4-Pip- |
| 1-333 | Me | H | -CO- | $-(CH_2)_2-$ | 1-(2-Thi)-4-Pip- |
| 1-334 | Me | 5-F | -CO- | -CH=CH- | 1-(2-Thi)-4-Pip- |
| 1-335 | Me | 5-F | -CO- | $-(CH_2)_2-$ | 1-(2-Thi)-4-Pip- |
| 1-336 | Me | 5-Cl | -CO- | -CH=CH- | 1-(2-Thi)-4-Pip- |
| 1-337 | Me | 5-Cl | -CO- | $-(CH_2)_2-$ | 1-(2-Thi)-4-Pip- |
| 1-338 | Me | 5-Me | -CO- | -CH=CH- | 1-(2-Thi)-4-Pip- |
| 1-339 | Me | 5-Me | -CO- | $-(CH_2)_2-$ | 1-(2-Thi)-4-Pip- |
| 1-340 | Me | 5-CN | -CO- | -CH=CH- | 1-(2-Thi)-4-Pip- |
| 1-341 | Me | 5-CN | -CO- | $-(CH_2)_2-$ | 1-(2-Thi)-4-Pip- |
| 1-342 | Et | H | -CO- | -CH=CH- | 1-(2-Thi)-4-Pip- |
| 1-343 | Et | H | -CO- | $-(CH_2)_2-$ | 1-(2-Thi)-4-Pip- |
| 1-344 | Et | 5-F | -CO- | -CH=CH- | 1-(2-Thi)-4-Pip- |
| 1-345 | Et | 5-F | -CO- | $-(CH_2)_2-$ | 1-(2-Thi)-4-Pip- |
| 1-346 | Et | 5-Cl | -CO- | -CH=CH- | 1-(2-Thi)-4-Pip- |
| 1-347 | Et | 5-Cl | -CO- | $-(CH_2)_2-$ | 1-(2-Thi)-4-Pip- |
| 1-348 | Et | 5-Me | -CO- | -CH=CH- | 1-(2-Thi)-4-Pip- |
| 1-349 | Et | 5-Me | -CO- | $-(CH_2)_2-$ | 1-(2-Thi)-4-Pip- |

TABLE 1 CONT'D

| Cmpd | $R^1$ | $R^2$ | U | V | W |
|---|---|---|---|---|---|
| 1-350 | Et | 5-CN | -CO- | -CH=CH- | 1-(2-Thi)-4-Pip- |
| 1-351 | Et | 5-CN | -CO- | $-(CH_2)_2-$ | 1-(2-Thi)-4-Pip- |
| 1-352 | iPr | H | -CO- | -CH=CH- | 1-(2-Thi)-4-Pip- |
| 1-353 | iPr | H | -CO- | $-(CH_2)_2-$ | 1-(2-Thi)-4-Pip- |
| 1-354 | iPr | 5-F | -CO- | -CH=CH- | 1-(2-Thi)-4-Pip- |
| 1-355 | iPr | 5-F | -CO- | $-(CH_2)_2-$ | 1-(2-Thi)-4-Pip- |
| 1-356 | iPr | 5-Cl | -CO- | -CH=CH- | 1-(2-Thi)-4-Pip- |
| 1-357 | iPr | 5-Cl | -CO- | $-(CH_2)_2-$ | 1-(2-Thi)-4-Pip- |
| 1-358 | iPr | 5-Me | -CO- | -CH=CH- | 1-(2-Thi)-4-Pip- |
| 1-359 | iPr | 5-Me | -CO- | $-(CH_2)_2-$ | 1-(2-Thi)-4-Pip- |
| 1-360 | iPr | 5-CN | -CO- | -CH=CH- | 1-(2-Thi)-4-Pip- |
| 1-361 | iPr | 5-CN | -CO- | $-(CH_2)_2-$ | 1-(2-Thi)-4-Pip- |

TABLE 1A

In the following compounds of formula (I-1), the group -U-V-W is attached to the 5-position of the indole ring.

| Cmpd | $R^1$ | $R^2$ | U | V | W |
|---|---|---|---|---|---|
| 1-362 | Me | H | -CO- | -CH=CH- | 1-Bz-4-Pip- |
| 1-363 | Me | H | -CO- | $(CH_2)_2-$ | 1-Bz-4-Pip- |
| 1-364 | Me | BzOCO(Me)NMe | -CO- | -CH=CH- | 1-Bz-4-Pip- |
| 1-365 | Me | MeNHMe | -CO- | $-(CH_2)_2-$ | 1-Bz-4-Pip- |
| 1-366 | Me | Prg(Me)NMe | -CO- | $-(CH_2)_2-$ | 1-Bz-4-Pip- |

TABLE 1B

In the following compounds of formula (I-1), the group -U-V-W is attached to the 4-position of the indole ring.

| Cmpd | $R^1$ | $R^2$ | U | V | W |
|---|---|---|---|---|---|
| 1-367 | Me | H | -CO- | -CH=CH- | 1-Bz-4-Pip- |
| 1-368 | Me | H | -CO- | $(CH_2)_2$- | 1-Bz-4-Pip- |
| 1-369 | Me | BzOCO(Me)NMe | -CO- | -CH=CH- | 1-Bz-4-Pip- |
| 1-370 | Me | MeNHMe | -CO- | $-(CH_2)_2-$ | 1-Bz-4-Pip- |
| 1-371 | Me | Prg(Me)NMe | -CO- | $-(CH_2)_2-$ | 1-Bz-4-Pip- |

TABLE 2

In the following compounds of formula (I-2), the group -U-V-W is attached at the 3-position of the indazole ring.

| Cmpd | $R^1$ | $R^2$ | U | V | W | |
|---|---|---|---|---|---|---|
| 2-1 | Me | H | -CO- | -CH=CH- | 1-Bz-4-Pip- | |
| 2-2 | Me | H | -CO- | $-(CH_2)_2-$ | 1-Bz-4-Pip- | |
| 2-3 | Me | H | -CO- | $-(CH_2)_2-$ | 1-Bz-4-Pip- | .HCl |
| 2-4 | Me | H | -CO- | $-(CH_2)_2-$ | 1-Bz-4-Pip- | .Ox |
| 2-5 | Me | 4-Me | -CO- | -CH=CH- | 1-Bz-4-Pip- | |
| 2-6 | Me | 4-Me | -CO- | $-(CH_2)_2-$ | 1-Bz-4-Pip- | |
| 2-7 | Me | 5-Me | -CO- | -CH=CH- | 1-Bz-4-Pip- | |
| 2-8 | Me | 5-Me | -CO- | $-(CH_2)_2-$ | 1-Bz-4-Pip- | |
| 2-9 | Me | 6-Me | -CO- | -CH=CH- | 1-Bz-4-Pip- | |
| 2-10 | Me | 6-Me | -CO- | $-(CH_2)_2-$ | 1-Bz-4-Pip- | |
| 2-11 | Me | 5-Et | -CO- | -CH=CH- | 1-Bz-4-Pip- | |
| 2-12 | Me | 5-Et | -CO- | $-(CH_2)_2-$ | 1-Bz-4-Pip- | |
| 2-13 | Me | 6-Et | -CO- | -CH=CH- | 1-Bz-4-Pip- | |
| 2-14 | Me | 6-Et | -CO- | $-(CH_2)_2-$ | 1-Bz-4-Pip- | |
| 2-15 | Me | 5-iPr | -CO- | -CH=CH- | 1-Bz-4-Pip- | |
| 2-16 | Me | 5-iPr | -CO- | $-(CH_2)_2-$ | 1-Bz-4-Pip- | |
| 2-17 | Me | 6-iPr | -CO- | -CH=CH- | 1-Bz-4-Pip- | |
| 2-18 | Me | 6-iPr | -CO- | $-(CH_2)_2-$ | 1-Bz-4-Pip- | |
| 2-19 | Me | 5-OMe | -CO- | -CH=CH- | 1-Bz-4-Pip- | |
| 2-20 | Me | 5-OMe | -CO- | $-(CH_2)_2-$ | 1-Bz-4-Pip- | |
| 2-21 | Me | 6-OMe | -CO- | -CH=CH- | 1-Bz-4-Pip- | |
| 2-22 | Me | 6-OMe | -CO- | $-(CH_2)_2-$ | 1-Bz-4-Pip- | |
| 2-23 | Me | 4-Cl | -CO- | -CH=CH- | 1-Bz-4-Pip- | |
| 2-24 | Me | 4-Cl | -CO- | $-(CH_2)_2-$ | 1-Bz-4-Pip- | |

TABLE 2 CONT'D

| Cmpd | $R^1$ | $R^2$ | U | V | W |
|---|---|---|---|---|---|
| 2-25 | Me | 5-Cl | -CO- | -CH=CH- | 1-Bz-4-Pip- |
| 2-26 | Me | 5-Cl | -CO- | $-(CH_2)_2-$ | 1-Bz-4-Pip- |
| 2-27 | Me | 6-Cl | -CO- | -CH=CH- | 1-Bz-4-Pip- |
| 2-28 | Me | 6-Cl | -CO- | $-(CH_2)_2-$ | 1-Bz-4-Pip- |
| 2-29 | Me | 7-Cl | -CO- | -CH=CH- | 1-Bz-4-Pip- |
| 2-30 | Me | 7-Cl | -CO- | $-(CH_2)_2-$ | 1-Bz-4-Pip- |
| 2-31 | Me | $4-NO_2$ | -CO- | -CH=CH- | 1-Bz-4-Pip- |
| 2-32 | Me | $4-NO_2$ | -CO- | $-(CH_2)_2-$ | 1-Bz-4-Pip- |
| 2-33 | Me | $5-NO_2$ | -CO- | -CH=CH- | 1-Bz-4-Pip- |
| 2-34 | Me | $5-NO_2$ | -CO- | $-(CH_2)_2-$ | 1-Bz-4-Pip- |
| 2-35 | Me | $6-NO_2$ | -CO- | -CH=CH- | 1-Bz-4-Pip- |
| 2-36 | Me | $6-NO_2$ | -CO- | $-(CH_2)_2-$ | 1-Bz-4-Pip- |
| 2-37 | Me | 5-F | -CO- | -CH=CH- | 1-Bz-4-Pip- |
| 2-38 | Me | 5-F | -CO- | $-(CH_2)_2-$ | 1-Bz-4-Pip- |
| 2-39 | Me | 4-F | -CO- | -CH=CH- | 1-Bz-4-Pip- |
| 2-40 | Me | 4-F | -CO- | $-(CH_2)_2-$ | 1-Bz-4-Pip- |
| 2-41 | Me | 6-F | -CO- | -CH=CH- | 1-Bz-4-Pip- |
| 2-42 | Me | 6-F | -CO- | $-(CH_2)_2-$ | 1-Bz-4-Pip- |
| 2-43 | Me | 7-F | -CO- | -CH=CH- | 1-Bz-4-Pip- |
| 2-44 | Me | 7-F | -CO- | $-(CH_2)_2-$ | 1-Bz-4-Pip- |
| 2-45 | Me | 4-CN | -CO- | -CH=CH- | 1-Bz-4-Pip- |
| 2-46 | Me | 4-CN | -CO- | $-(CH_2)_2-$ | 1-Bz-4-Pip- |
| 2-47 | Me | 5-CN | -CO- | -CH=CH- | 1-Bz-4-Pip- |
| 2-48 | Me | 5-CN | -CO- | $-(CH_2)_2-$ | 1-Bz-4-Pip- |
| 2-49 | Me | 6-CN | -CO- | -CH=CH- | 1-Bz-4-Pip- |
| 2-50 | Me | 6-CN | -CO- | $-(CH_2)_2-$ | 1-Bz-4-Pip- |
| 2-51 | Me | 7-CN | -CO- | -CH=CH- | 1-Bz-4-Pip- |

TABLE 2 CONT'D

| Cmpd | $R^1$ | $R^2$ | U | V | W |
|---|---|---|---|---|---|
| 2-52 | Me | 7-CN | -CO- | $-(CH_2)_2-$ | 1-Bz-4-Pip- |
| 2-53 | Me | $4-CF_3$ | -CO- | -CH=CH- | 1-Bz-4-Pip- |
| 2-54 | Me | $4-CF_3$ | -CO- | $-(CH_2)_2-$ | 1-Bz-4-Pip- |
| 2-55 | Me | $5-CF_3$ | -CO- | -CH=CH- | 1-Bz-4-Pip- |
| 2-56 | Me | $5-CF_3$ | -CO- | $-(CH_2)_2-$ | 1-Bz-4-Pip- |
| 2-57 | Me | $6-CF_3$ | -CO- | -CH=CH- | 1-Bz-4-Pip- |
| 2-58 | Me | $6-CF_3$ | -CO- | $-(CH_2)_2-$ | 1-Bz-4-Pip- |
| 2-59 | Me | $7-CF_3$ | -CO- | -CH=CH- | 1-Bz-4-Pip- |
| 2-60 | Me | $7-CF_3$ | -CO- | $-(CH_2)_2-$ | 1-Bz-4-Pip- |
| 2-61 | Me | $4-CH_2CF_3$ | -CO- | -CH=CH- | 1-Bz-4-Pip- |
| 2-62 | Me | $4-CH_2CF_3$ | -CO- | $-(CH_2)_2-$ | 1-Bz-4-Pip- |
| 2-63 | Me | $5-CH_2CF_3$ | -CO- | -CH=CH- | 1-Bz-4-Pip- |
| 2-64 | Me | $5-CH_2CF_3$ | -CO- | $-(CH_2)_2-$ | 1-Bz-4-Pip- |
| 2-65 | Me | $6-CH_2CF_3$ | -CO- | -CH=CH- | 1-Bz-4-Pip- |
| 2-66 | Me | $6-CH_2CF_3$ | -CO- | $-(CH_2)_2-$ | 1-Bz-4-Pip- |
| 2-67 | Me | $7-CH_2CF_3$ | -CO- | -CH=CH- | 1-Bz-4-Pip- |
| 2-68 | Me | $7-CH_2CF_3$ | -CO- | $-(CH_2)_2-$ | 1-Bz-4-Pip- |
| 2-69 | Me | H | -CO- | -CH=CH- | 1-(2-F-Bz)-4-Pip- |
| 2-70 | Me | H | -CO- | $-(CH_2)_2-$ | 1-(2-F-Bz)-4-Pip- |
| 2-71 | Me | H | -CO- | -CH=CH- | 1-(3-F-Bz)-4-Pip- |
| 2-72 | Me | H | -CO- | $-(CH_2)_2-$ | 1-(3-F-Bz)-4-Pip- |
| 2-73 | Me | H | -CO- | -CH=CH- | 1-(4-F-Bz)-4-Pip- |
| 2-74 | Me | H | -CO- | $-(CH_2)_2-$ | 1-(4-F-Bz)-4-Pip- |
| 2-75 | Me | 4-F | -CO- | -CH=CH- | 1-(2-F-Bz)-4-Pip- |
| 2-76 | Me | 4-F | -CO- | $-(CH_2)_2-$ | 1-(2-F-Bz)-4-Pip- |
| 2-77 | Me | 4-F | -CO- | -CH=CH- | 1-(3-F-Bz)-4-Pip- |
| 2-78 | Me | 4-F | -CO- | $-(CH_2)_2-$ | 1-(3-F-Bz)-4-Pip- |
| 2-79 | Me | 4-F | -CO- | -CH=CH- | 1-(4-F-Bz)-4-Pip- |

TABLE 2 CONT'D

| Cmpd | $R^1$ | $R^2$ | U | V | W | |
|---|---|---|---|---|---|---|
| 2-80 | Me | 4-F | -CO- | $-(CH_2)_2-$ | 1-(4-F-Bz)-4-Pip- | |
| 2-81 | Me | 5-F | -CO- | -CH=CH- | 1-(2-F-Bz)-4-Pip- | |
| 2-82 | Me | 5-F | -CO- | $-(CH_2)_2-$ | 1-(2-F-Bz)-4-Pip- | |
| 2-83 | Me | 5-F | -CO- | -CH=CH- | 1-(3-F-Bz)-4-Pip- | |
| 2-84 | Me | 5-F | -CO- | $-(CH_2)_2-$ | 1-(3-F-Bz)-4-Pip- | |
| 2-85 | Me | 5-F | -CO- | $-(CH_2)_2-$ | 1-(3-F-Bz)-4-Pip- .HCl | |
| 2-86 | Me | 5-F | -CO- | -CH=CH- | 1-(4-F-Bz)-4-Pip- | |
| 2-87 | Me | 5-F | -CO- | $-(CH_2)_2-$ | 1-(4-F-Bz)-4-Pip- | |
| 2-88 | Me | 6-F | -CO- | -CH=CH- | 1-(2-F-Bz)-4-Pip- | |
| 2-89 | Me | 6-F | -CO- | $-(CH_2)_2-$ | 1-(2-F-Bz)-4-Pip- | |
| 2-90 | Me | 6-F | -CO- | -CH=CH- | 1-(3-F-Bz)-4-Pip- | |
| 2-91 | Me | 6-F | -CO- | $-(CH_2)_2-$ | 1-(3-F-Bz)-4-Pip- | |
| 2-92 | Me | 6-F | -CO- | -CH=CH- | 1-(4-F-Bz)-4-Pip- | |
| 2-93 | Me | 6-F | -CO- | $-(CH_2)_2-$ | 1-(4-F-Bz)-4-Pip- | |
| 2-94 | Me | H | -CO- | -CH=CH- | 1-(2-Cl-Bz)-4-Pip- | |
| 2-95 | Me | H | -CO- | $-(CH_2)_2-$ | 1-(2-Cl-Bz)-4-Pip- | |
| 2-96 | Me | H | -CO- | $-(CH_2)_2-$ | 1-(2-Cl-Bz)-4-Pip- | .HCl |
| 2-97 | Me | H | -CO- | -CH=CH- | 1-(3-Cl-Bz)-4-Pip- | |
| 2-98 | Me | H | -CO- | $-(CH_2)_2-$ | 1-(3-Cl-Bz)-4-Pip- | |
| 2-99 | Me | H | -CO- | $-(CH_2)_2-$ | 1-(3-Cl-Bz)-4-Pip- | .HCl |
| 2-100 | Me | H | -CO- | -CH=CH- | 1-(4-Cl-Bz)-4-Pip- | |
| 2-101 | Me | H | -CO- | $-(CH_2)_2-$ | 1-(4-Cl-Bz)-4-Pip- | |
| 2-102 | Me | H | -CO- | $-(CH_2)_2-$ | 1-(4-Cl-Bz)-4-Pip- | .HCl |
| 2-103 | Me | H | -CO- | -CH=CH- | 1-(3-Br-Bz)-4-Pip- | |
| 2-104 | Me | H | -CO- | $-(CH_2)_2-$ | 1-(3-Br-Bz)-4-Pip- | |
| 2-105 | Me | H | -CO- | -CH=CH- | 1-(4-Br-Bz)-4-Pip- | |
| 2-106 | Me | H | -CO- | $-(CH_2)_2-$ | 1-(4-Br-Bz)-4-Pip- | |

TABLE 2 CONT'D

| Cmpd | $R^1$ | $R^2$ | U | V | W |
|---|---|---|---|---|---|
| 2-107 | Me | H | -CO- | -CH=CH- | 1-(3-I-Bz)-4-Pip- |
| 2-108 | Me | H | -CO- | $-(CH_2)_2-$ | 1-(3-I-Bz)-4-Pip- |
| 2-109 | Me | 4-Cl | -CO- | -CH=CH- | 1-(2-Cl-Bz)-4-Pip- |
| 2-110 | Me | 4-Cl | -CO- | $-(CH_2)_2-$ | 1-(2-Cl-Bz)-4-Pip- |
| 2-111 | Me | 5-Cl | -CO- | -CH=CH- | 1-(2-Cl-Bz)-4-Pip- |
| 2-112 | Me | 5-Cl | -CO- | $-(CH_2)_2-$ | 1-(2-Cl-Bz)-4-Pip- |
| 2-113 | Me | 6-Cl | -CO- | -CH=CH- | 1-(2-Cl-Bz)-4-Pip- |
| 2-114 | Me | 6-Cl | -CO- | $-(CH_2)_2-$ | 1-(2-Cl-Bz)-4-Pip- |
| 2-115 | Me | 4-Cl | -CO- | -CH=CH- | 1-(3-Cl-Bz)-4-Pip- |
| 2-116 | Me | 4-Cl | -CO- | $-(CH_2)_2-$ | 1-(3-Cl-Bz)-4-Pip- |
| 2-117 | Me | 5-Cl | -CO- | -CH=CH- | 1-(3-Cl-Bz)-4-Pip- |
| 2-118 | Me | 5-Cl | -CO- | $-(CH_2)_2-$ | 1-(3-Cl-Bz)-4-Pip- |
| 2-119 | Me | 5-Cl | -CO- | $-(CH_2)_2-$ | 1-(3-Cl-Bz)-4-Pip- .HCl |
| 2-120 | Me | 6-Cl | -CO- | -CH=CH- | 1-(3-Cl-Bz)-4-Pip- |
| 2-121 | Me | 6-Cl | -CO- | $-(CH_2)_2-$ | 1-(3-Cl-Bz)-4-Pip- |
| 2-122 | Me | 4-Cl | -CO- | -CH=CH- | 1-(4-Cl-Bz)-4-Pip- |
| 2-123 | Me | 4-Cl | -CO- | $-(CH_2)_2-$ | 1-(4-Cl-Bz)-4-Pip- |
| 2-124 | Me | 5-Cl | -CO- | -CH=CH- | 1-(4-Cl-Bz)-4-Pip- |
| 2-125 | Me | 5-Cl | -CO- | $-(CH_2)_2-$ | 1-(4-Cl-Bz)-4-Pip- |
| 2-126 | Me | 6-Cl | -CO- | -CH=CH- | 1-(4-Cl-Bz)-4-Pip- |
| 2-127 | Me | 6-Cl | -CO- | $-(CH_2)_2-$ | 1-(4-Cl-Bz)-4-Pip- |
| 2-128 | Me | H | -CO- | -CH=CH- | 1-(2-Me-Bz)-4-Pip- |
| 2-129 | Me | H | -CO- | $-(CH_2)_2-$ | 1-(2-Me-Bz)-4-Pip- |
| 2-130 | Me | H | -CO- | -CH=CH- | 1-(3-Me-Bz)-4-Pip- |
| 2-131 | Me | H | -CO- | $-(CH_2)_2-$ | 1-(3-Me-Bz)-4-Pip- |
| 2-132 | Me | H | -CO- | -CH=CH- | 1-(4-Me-Bz)-4-Pip- |
| 2-133 | Me | H | -CO- | $-(CH_2)_2-$ | 1-(4-Me-Bz)-4-Pip- |
| 2-134 | Me | H | -CO- | -CH=CH- | 1-(3-Et-Bz)-4-Pip- |

TABLE 2 CONT'D

| Cmpd | $R^1$ | $R^2$ | U | V | W |
|---|---|---|---|---|---|
| 2-135 | Me | H | -CO- | $-(CH_2)_2-$ | $1-(3-Et-Bz)-4-Pip-$ |
| 2-136 | Me | H | -CO- | -CH=CH- | $1-(2-CF_3-Bz)-4-Pip-$ |
| 2-137 | Me | H | -CO- | $-(CH_2)_2-$ | $1-(2-CF_3-Bz)-4-Pip-$ |
| 2-138 | Me | H | -CO- | -CH=CH- | $1-(3-CF_3-Bz)-4-Pip-$ |
| 2-139 | Me | H | -CO- | $-(CH_2)_2-$ | $1-(3-CF_3-Bz)-4-Pip-$ |
| 2-140 | Me | H | -CO- | -CH=CH- | $1-(4-CF_3-Bz)-4-Pip-$ |
| 2-141 | Me | H | -CO- | $-(CH_2)_2-$ | $1-(4-CF_3-Bz)-4-Pip-$ |
| 2-142 | Me | H | -CO- | -CH=CH- | 1-(3-CN-Bz)-4-Pip- |
| 2-143 | Me | H | -CO- | $-(CH_2)_2-$ | 1-(3-CN-Bz)-4-Pip- |
| 2-144 | Me | H | -CO- | -CH=CH- | 1-(2-Thi)-4-Pip- |
| 2-145 | Me | H | -CO- | $-(CH_2)_2-$ | 1-(2-Thi)-4-Pip- |
| 2-146 | Me | 5-F | -CO- | -CH=CH- | 1-(2-Thi)-4-Pip- |
| 2-147 | Me | 5-F | -CO- | $-(CH_2)_2-$ | 1-(2-Thi)-4-Pip- |
| 2-148 | Me | 5-Cl | -CO- | -CH=CH- | 1-(2-Thi)-4-Pip- |
| 2-149 | Me | 5-Cl | -CO- | $-(CH_2)_2-$ | 1-(2-Thi)-4-Pip- |
| 2-150 | Me | 5-Me | -CO- | -CH=CH- | 1-(2-Thi)-4-Pip- |
| 2-151 | Me | 5-Me | -CO- | $-(CH_2)_2-$ | 1-(2-Thi)-4-Pip- |
| 2-152 | Me | 5-CN | -CO- | -CH=CH- | 1-(2-Thi)-4-Pip- |
| 2-153 | Me | 5-CN | -CO- | $-(CH_2)_2-$ | 1-(2-Thi)-4-Pip- |
| 2-154 | Et | H | -CO- | -CH=CH- | 1-(2-Thi)-4-Pip- |
| 2-155 | Et | H | -CO- | $-(CH_2)_2-$ | 1-(2-Thi)-4-Pip- |
| 2-156 | Et | 5-F | -CO- | -CH=CH- | 1-(2-Thi)-4-Pip- |
| 2-157 | Et | 5-F | -CO- | $-(CH_2)_2-$ | 1-(2-Thi)-4-Pip- |
| 2-158 | Et | 5-Cl | -CO- | -CH=CH- | 1-(2-Thi)-4-Pip- |
| 2-159 | Et | 5-Cl | -CO- | $-(CH_2)_2-$ | 1-(2-Thi)-4-Pip- |
| 2-160 | Et | 5-Me | -CO- | -CH=CH- | 1-(2-Thi)-4-Pip- |
| 2-161 | Et | 5-Me | -CO- | $-(CH_2)_2-$ | 1-(2-Thi)-4-Pip- |
| 2-162 | Et | 5-CN | -CO- | -CH=CH- | 1-(2-Thi)-4-Pip- |

TABLE 2 CONT'D

| Cmpd | $R^1$ | $R^2$ | U | V | W | |
|---|---|---|---|---|---|---|
| 2-163 | Et | 5-CN | -CO- | $-(CH_2)_2-$ | 1-(2-Thi)-4-Pip- | |
| 2-164 | Me | H | -CO- | -CH=CH- | 1-(2-MeO-Bz)-4-Pip- | |
| 2-165 | Me | H | -CO- | $-(CH_2)_2-$ | 1-(2-MeO-Bz)-4-Pip- | |
| 2-166 | Me | H | -CO- | $-(CH_2)_2-$ | 1-(2-MeO-Bz)-4-Pip- | .HCl |
| 2-167 | Me | H | -CO- | -CH=CH- | 1-(3-MeO-Bz)-4-Pip- | |
| 2-168 | Me | H | -CO- | $-(CH_2)_2-$ | 1-(3-MeO-Bz)-4-Pip- | |
| 2-169 | Me | H | -CO- | $-(CH_2)_2-$ | 1-(3-MeO-Bz)-4-Pip- | .HCl |
| 2-170 | Me | H | -CO- | -CH=CH- | 1-(4-MeO-Bz)-4-Pip- | |
| 2-171 | Me | H | -CO- | $-(CH_2)_2-$ | 1-(4-MeO-Bz)-4-Pip- | |
| 2-172 | Me | H | -CO- | $-(CH_2)_2-$ | 1-(4-MeO-Bz)-4-Pip- | .HCl |
| 2-173 | Me | H | -CO- | -CH=CH- | 1-Me-4-Pip- | |
| 2-174 | Me | H | -CO- | $-(CH_2)_2-$ | 1-Me-4-Pip- | |
| 2-175 | Me | H | -CO- | -CH=CH- | 1-Et-4-Pip- | |
| 2-176 | Me | H | -CO- | $-(CH_2)_2-$ | 1-Et-4-Pip- | |
| 2-177 | Me | H | -CO- | -CH=CH- | 1-iPr-4-Pip- | |
| 2-178 | Me | H | -CO- | $-(CH_2)_2-$ | 1-iPr-4-Pip- | |
| 2-179 | Me | H | -CO- | -CH=CH- | $1-CF_3-4-Pip-$ | |
| 2-180 | Me | H | -CO- | $-(CH_2)_2-$ | $1-CF_3-4-Pip-$ | |
| 2-181 | Me | H | -CO- | -CH=CH- | $1-CH_2CF_3-4-Pip-$ | |
| 2-182 | Me | H | -CO- | $-(CH_2)_2-$ | $1-CH_2CF_3-4-Pip-$ | |
| 2-183 | Et | H | -CO- | -CH=CH- | 1-Bz-4-Pip- | |
| 2-184 | Et | H | -CO- | $-(CH_2)_2-$ | 1-Bz-4-Pip- | |
| 2-185 | Et | 5-Me | -CO- | -CH=CH- | 1-Bz-4-Pip- | |
| 2-186 | Et | 5-Me | -CO- | $-(CH_2)_2-$ | 1-Bz-4-Pip- | |
| 2-187 | Et | 6-Me | -CO- | -CH=CH- | 1-Bz-4-Pip- | |
| 2-188 | Et | 6-Me | -CO- | $-(CH_2)_2-$ | 1-Bz-4-Pip- | |
| 2-189 | Et | 4-F | -CO- | -CH=CH- | 1-Bz-4-Pip- | |
| 2-190 | Et | 4-F | -CO- | $-(CH_2)_2-$ | 1-Bz-4-Pip- | |

TABLE 2 CONT'D

| Cmpd | $R^1$ | $R^2$ | U | V | W |
|---|---|---|---|---|---|
| 2-191 | Et | 5-F | -CO- | -CH=CH- | 1-Bz-4-Pip- |
| 2-192 | Et | 5-F | -CO- | $-(CH_2)_2-$ | 1-Bz-4-Pip- |
| 2-193 | Et | 6-F | -CO- | -CH=CH- | 1-Bz-4-Pip- |
| 2-194 | Et | 6-F | -CO- | $-(CH_2)_2-$ | 1-Bz-4-Pip- |
| 2-195 | Et | 4-Cl | -CO- | -CH=CH- | 1-Bz-4-Pip- |
| 2-196 | Et | 4-Cl | -CO- | $-(CH_2)_2-$ | 1-Bz-4-Pip- |
| 2-197 | Et | 5-Cl | -CO- | -CH=CH- | 1-Bz-4-Pip- |
| 2-198 | Et | 5-Cl | -CO- | $-(CH_2)_2-$ | 1-Bz-4-Pip- |
| 2-199 | Et | 6-Cl | -CO- | -CH=CH- | 1-Bz-4-Pip- |
| 2-200 | Et | 6-Cl | -CO- | $-(CH_2)_2-$ | 1-Bz-4-Pip- |
| 2-201 | Et | 5-CN | -CO- | -CH=CH- | 1-Bz-4-Pip- |
| 2-202 | Et | 5-CN | -CO- | $-(CH_2)_2-$ | 1-Bz-4-Pip- |
| 2-203 | Et | 6-CN | -CO- | -CH=CH- | 1-Bz-4-Pip- |
| 2-204 | Et | 6-CN | -CO- | $-(CH_2)_2-$ | 1-Bz-4-Pip- |
| 2-205 | iPr | H | -CO- | -CH=CH- | 1-Bz-4-Pip- |
| 2-206 | iPr | H | -CO- | $-(CH_2)_2-$ | 1-Bz-4-Pip- |
| 2-207 | iPr | 5-Cl | -CO- | -CH=CH- | 1-Bz-4-Pip- |
| 2-208 | iPr | 5-Cl | -CO- | $-(CH_2)_2-$ | 1-Bz-4-Pip- |
| 2-209 | iPr | 5-CN | -CO- | -CH=CH- | 1-Bz-4-Pip- |
| 2-210 | iPr | 5-CN | -CO- | $-(CH_2)_2-$ | 1-Bz-4-Pip- |
| 2-211 | iPr | $5-NO_2$ | -CO- | -CH=CH- | 1-Bz-4-Pip- |
| 2-212 | iPr | $5-NO_2$ | -CO- | $-(CH_2)_2-$ | 1-Bz-4-Pip- |
| 2-213 | iBu | H | -CO- | -CH=CH- | 1-Bz-4-Pip- |
| 2-214 | iBu | H | -CO- | $-(CH_2)_2-$ | 1-Bz-4-Pip- |
| 2-215 | iBu | 5-Cl | -CO- | -CH=CH- | 1-Bz-4-Pip- |
| 2-216 | iBu | 5-Cl | -CO- | $-(CH_2)_2-$ | 1-Bz-4-Pip- |
| 2-217 | iBu | 5-CN | -CO- | -CH=CH- | 1-Bz-4-Pip- |
| 2-218 | iBu | 5-CN | -CO- | $-(CH_2)_2-$ | 1-Bz-4-Pip- |

TABLE 2 CONT'D

| Cmpd | $R^1$ | $R^2$ | U | V | W |
|---|---|---|---|---|---|
| 2-219 | iBu | 5-$NO_2$ | -CO- | -CH=CH- | 1-Bz-4-Pip- |
| 2-220 | iBu | 5-$NO_2$ | -CO- | -$(CH_2)_2$- | 1-Bz-4-Pip- |
| 2-221 | H | H | -CO- | -$(CH_2)_2$- | 1-Bz-4-Pip- |
| 2-222 | Me | H | -CO- | -CH=CH- | 1-Bz-3-Pyrd- |
| 2-223 | Me | H | -CO- | -$(CH_2)_2$- | 1-Bz-3-Pyrd- |
| 2-224 | Me | H | -CO- | -CH=CH- | 1-PhEt-4-Pip- |
| 2-225 | Me | H | -CO- | -$(CH_2)_2$- | 1-PhEt-4-Pip- |
| 2-226 | Me | H | -CO- | -$(CH_2)_2$- | 1-PhEt-4-Pip- .HCl |
| 2-227 | Me | 5-Cl | -CO- | -CH=CH- | 1-PhEt-4-Pip- |
| 2-228 | Me | 5-Cl | -CO- | -$(CH_2)_2$- | 1-PhEt-4-Pip- |
| 2-229 | Me | 6-Cl | -CO- | -CH=CH- | 1-PhEt-4-Pip- |
| 2-230 | Me | 6-Cl | -CO- | -$(CH_2)_2$- | 1-PhEt-4-Pip- |
| 2-231 | Me | 5-OMe | -CO- | -CH=CH- | 1-PhEt-4-Pip- |
| 2-232 | Me | 5-OMe | -CO- | -$(CH_2)_2$- | 1-PhEt-4-Pip- |
| 2-233 | Me | 5-OMe | -CO- | -$(CH_2)_2$- | 1-PhEt-4-Pip- .HCl |
| 2-234 | Me | H | -CO- | -CH=CH- | 1-(2-F-Ph)Et-4-Pip- |
| 2-235 | Me | H | -CO- | -$(CH_2)_2$- | 1-(2-F-Ph)Et-4-Pip- |
| 2-236 | Me | H | -CO- | -CH=CH- | 1-(3-F-Ph)Et-4-Pip- |
| 2-237 | Me | H | -CO- | -$(CH_2)_2$- | 1-(3-F-Ph)Et-4-Pip- |
| 2-238 | Me | H | -CO- | -CH=CH- | 1-(4-F-Ph)Et-4-Pip- |
| 2-239 | Me | H | -CO- | -$(CH_2)_2$- | 1-(4-F-Ph)Et-4-Pip- |
| 2-240 | Me | H | -CO- | -CH=CH- | 1-(2-Cl-Ph)Et-4-Pip- |
| 2-241 | Me | H | -CO- | -$(CH_2)_2$- | 1-(2-Cl-Ph)Et-4-Pip- |
| 2-242 | Me | H | -CO- | -CH=CH- | 1-(3-Cl-Ph)Et-4-Pip- |
| 2-243 | Me | H | -CO- | -$(CH_2)_2$- | 1-(3-Cl-Ph)Et-4-Pip- |
| 2-244 | Me | H | -CO- | -CH=CH- | 1-(4-Cl-Ph)Et-4-Pip- |
| 2-245 | Me | H | -CO- | -$(CH_2)_2$- | 1-(4-Cl-Ph)Et-4-Pip- .HCl |

TABLE 2 CONT'D

| Cmpd | $R^1$ | $R^2$ | U | V | W |
|---|---|---|---|---|---|
| 2-246 | Me | H | -CO- | -CH=CH- | 1-(3-$CF_3$-Ph)Et-4-Pip- |
| 2-247 | Me | H | -CO- | -$(CH_2)_2$- | 1-(3-$CF_3$-Ph)Et-4-Pip- |
| 2-248 | Me | H | -CO- | -$(CH_2)_2$- | 1-(3-$CF_3$-Ph)Et-4-Pip- .HCl |
| 2-249 | Et | H | -CO- | -CH=CH- | 1-PhEt-4-Pip- |
| 2-250 | Et | H | -CO- | -$(CH_2)_2$- | 1-PhEt-4-Pip- |
| 2-251 | Et | H | -CO- | -$(CH_2)_2$- | 1-PhEt-4-Pip- .HCl |
| 2-252 | Me | H | -CO- | -$(CH_2)_5$- | Bz(Me)N- |
| 2-253 | Me | H | -CO- | -$(CH_2)_5$- | Bz(Et)N- |
| 2-254 | Me | H | -CO- | -$(CH_2)_5$- | Bz(iPr)N- |
| 2-255 | Et | H | -CO- | -$(CH_2)_5$- | Bz(Et)N- |
| 2-256 | iPr | H | -CO- | -$(CH_2)_5$- | Bz(Et)N- |
| 2-257 | H | H | -CO- | -CH=CH- | 2-tet.hydro-iQuin- |
| 2-258 | H | H | -CO- | -$(CH_2)_2$- | 2-tet.hydro-iQuin- |
| 2-259 | Me | H | -CO- | -CH=CH- | 2-tet.hydro-iQuin- |
| 2-260 | Me | H | -CO- | -$(CH_2)_2$- | 2-tet.hydro-iQuin- |
| 2-260 | Et | H | -CO- | -CH=CH- | 2-tet.hydro-iQuin- |
| 2-262 | Et | H | -CO- | -$(CH_2)_2$- | 2-tet.hydro-iQuin- |
| 2-263 | Me | H | -CO- | -$(CH_2)_5$- | 2-tet.hydro-iQuin- |
| 2-264 | Me | H | -CO- | -$(CH_2)_5$- | 2-tet.hydro-iQuin- .HCl |
| 2-265 | Me | H | -CO- | -CH=CH- | 2-Ind- |
| 2-266 | Me | H | -CO- | -$(CH_2)_2$- | 2-Ind- |
| 2-267 | Me | H | -CO- | -$(CH_2)_5$- | 2-Ind- |
| 2-268 | Me | H | -CO- | -$(CH_2)_5$- | 2-Ind- .HCl |
| 2-269 | Me | H | -CO- | -$(CH_2)_5$- | PhEtNH- |
| 2-270 | Me | H | -CO- | -$(CH_2)_5$- | PhEtNH- .HCl |
| 2-271 | Me | H | -CO- | -$(CH_2)_5$- | 2-(3,4-diMeOPh)Et- |

TABLE 2 CONT'D

| Cmpd | $R^1$ | $R^2$ | U | V | W |
|---|---|---|---|---|---|
| 2-272 | Me | H | -CO- | $-(CH_2)_5-$ | 2-(3,4-diMeOPh)Et-.HCl |
| 2-273 | Me | 5-F | -CHOH- | $-(CH_2)_2-$ | 1-Bz-4-Pip- |
| 2-274 | Et | 6-F | -CHOH- | $-(CH_2)_2-$ | 1-Bz-4-Pip- |
| 2-275 | iPr | 5-Cl | -CHOH- | $-(CH_2)_2-$ | 1-Bz-4-Pip- |
| 2-276 | iBu | 6-Cl | -CHOH- | $-(CH_2)_2-$ | 1-Bz-4-Pip- |
| 2-277 | Me | 5-F | -CHOAc- | $-(CH_2)_2-$ | 1-Bz-4-Pip- |
| 2-278 | Me | 6-F | -CHOAc- | $-(CH_2)_2-$ | 1-Bz-4-Pip- |
| 2-279 | iPr | 5-Me | -CHOAc- | $-(CH_2)_2-$ | 1-Bz-4-Pip- |
| 2-280 | iBu | 4-Me | -CHOAc- | $-(CH_2)_2-$ | 1-Bz-4-Pip- |
| 2-281 | Me | 5-F | -CHOBoz- | $-(CH_2)_2-$ | 1-Bz-4-Pip- |
| 2-282 | Et | 6-Me | -CHOBoz- | $-(CH_2)_2-$ | 1-Bz-4-Pip- |
| 2-283 | Me | H | -CO- | $-(CH_2)_2-$ | 1-(3-F-Bz)-4-Pip . HCl |

TABLE 2A

In the following compounds of formula (I-2), the group -U-V-W is attached to the 5-position of the indazole ring.

| Cmpd | $R^1$ | $R^2$ | U | V | W |
|---|---|---|---|---|---|
| 2-284 | Me | H | -CO- | -CH=CH- | 1-Bz-4-Pip- |
| 2-285 | Me | H | -CO- | $(CH_2)_2-$ | 1-Bz-4-Pip- |
| 2-286 | Me | BzOCO(Me)NMe | -CO- | -CH=CH- | 1-Bz-4-Pip- |
| 2-287 | Me | MeNHMe | -CO- | $-(CH_2)_2-$ | 1-Bz-4-Pip- |
| 2-288 | Me | Prg(Me)NMe | -CO- | $-(CH_2)_2-$ | 1-Bz-4-Pip- |

## TABLE 2B

In the following compounds of formula (I-2), the group -U-V-W is attached to the 4-position of the indazole ring.

| Cmpd | $R^1$ | $R^2$ | U | V | W |
|---|---|---|---|---|---|
| 2-289 | Me | H | -CO- | -CH=CH- | 1-Bz-4-Pip- |
| 2-290 | Me | H | -CO- | $(CH_2)_2$- | 1-Bz-4-Pip- |
| 2-291 | Me | BzOCO(Me)NMe | -CO- | -CH=CH- | 1-Bz-4-Pip- |
| 2-292 | Me | MeNHMe | -CO- | $-(CH_2)_2-$ | 1-Bz-4-Pip- |
| 2-293 | Me | Prg(Me)NMe | -CO- | $-(CH_2)_2-$ | 1-Bz-4-Pip- |

Of these compounds, we prefer Compounds No. 1-1, 1-2, 1-3, 1-4, 1-5, 1-6, 1-7, 1-8, 1-9, 1-10, 1-11, 1-12, 1-13, 1-14, 1-21, 1-22, 1-25, 1-26, 1-27, 1-28, 1-29, 1-30, 1-35, 1-36, 1-39, 1-40, 1-41, 1-42, 1-43, 1-44, 1-49, 1-50, 1-51, 1-52, 1-59, 1-60, 1-61, 1-62, 1-63, 1-69, 1-70, 1-71, 1-72, 1-77, 1-78, 1-79, 1-80, 1-81, 1-82, 1-83, 1-84, 1-85, 1-86, 1-87, 1-88, 1-89, 1-90, 1-102, 1-103, 1-108, 1-109, 1-110, 1-113, 1-114, 1-115, 1-118, 1-117, 1-118, 1-119, 1-120, 1-123, 1-124, 1-127, 1-128, 1-129, 1-130, 1-133, 1-134, 1-137, 1-139, 1-140, 1-143, 1-144, 1-145, 1-146, 1-158, 1-159, 1-170, 1-171, 1-172, 1-173, 1-178, 1-179, 1-180, 1-181, 1-182, 1-183, 1-190, 1-191, 1-192, 1-193, 1-194, 1-195, 1-198, 1-199, 1-200, 1-201, 1-204, 1-205, 1-208, 1-209, 1-215, 1-216, 1-217, 1-222, 1-223, 1-224, 1-225, 1-226, 1-233, 1-236, 1-241, 1-243, 1-244, 1-245, 1-246, 1-247, 1-248, 1-249, 1-250, 1-251, 1-261, 1-262, 1-265, 1-267, 1-268, 1-269, 1-270, 1-272, 1-273, 1-279, 1-301, 1-303, 1-304, 1-308, 1-309, 1-313, 1-314, 1-315, 1-316, 1-317, 1-321, 1-363, 1-366, 2-1, 2-2, 2-3, 2-4, 2-7, 2-8, 2-23, 2-24, 2-25, 2-26, 2-27, 2-28, 2-29, 2-30, 2-37, 2-38, 2-39, 2-40, 2-41, 2-42, 2-43, 2-44, 2-56, 2-57, 2-69, 2-70, 2-71, 2-72, 2-73, 2-74, 2-75, 2-76, 2-77, 2-78, 2-79, 2-80, 2-81, 2-82, 2-83, 2-84, 2-85, 2-86, 2-87, 2-88, 2-89, 2-90, 2-91, 2-92, 2-93, 2-94, 2-95, 2-96, 2-97, 2-98, 2-99, 2-100, 2-101, 2-102, 2-144, 2-145, 2-164, 2-165, 2-166, 2-167, 2-168, 2-169, 2-170, 2-171, 2-172, 2-183, 2-184, 2-205, 2-206, 2-213, 2-214, 2-224, 2-225 and 2-226. More preferred are Compounds no. 1-1, 1-2, 1-3, 1-4, 1-5, 1-10, 1-27, 1-28, 1-29, 1-30, 1-39, 1-40, 1-77, 1-78, 1-83, 1-84, 1-85, 1-116, 1-233, 1-279, 1-303, 1-304, 1-308, 1-309, 1-312, 1-313, 1-314, 1-315, 1-316, 1-321, 1-363, 1-366, 2-2, 2-3, 2-4, 2-23, 2-24, 2-25, 2-26, 2-27, 2-28, 2-37, 2-38, 2-39, 2-40, 2-41, 2-42, 2-69, 2-70, 2-71, 2-72, 2-73, 2-74, 2-75, 2-76, 2-77, 2-78, 2-79, 2-80, 2-81, 2-82, 2-83, 2-84, 2-85, 2-86, 2-87, 2-88, 2-89, 2-90, 2-91, 2-92, 2-93, 2-94, 2-95, 2-96, 2-97, 2-98, 2-99, 2-100 and 2-101.

The most preferred are Compounds No.:

1-1. 1-Methyl-3-[3-(1-benzyl-4-piperidyl)acryloyl]indole;
1-2. 1-Methyl-3-[3-(1-benzyl-4-piperidyl)propionyl]indole;
1-3. 1-Methyl-3-[3-(1-benzyl-4-piperidyl)propionyl]indole hydrochloride;
1-4. 1-Methyl-3-[3-(1-benzyl-4-piperidyl)propionyl]indole oxalate;
1-27. 1-Methyl-3-[3-(1-benzyl-4-piperidyl)acryloyl]-5-chloroindole;
1-28. 1-Methyl-3-[3-(1-benzyl-4-piperidyl)propionyl]-5-chloroindole;
1-30. 1-Methyl-3-[3-(1-benzyl-4-piperidyl)propionyl]-6-chloroindole;
1-39. 1-Methyl-3-[3-(1-benzyl-4-piperidyl)acryloyl]-5-fluoroindole;
1-40. 1-Methyl-3-[3-(1-benzyl-4-piperidyl)propionyl]-5-fluoroindole;
1-77. 1-Methyl-3-{3-[1-(3-fluorobenzyl)-4-piperidyl]-acryloyl}indole;
1-78. 1-Methyl-3-{3-[1-(3-fluorobenzyl)-4-piperidyl]-propionyl}indole;
1-84. 1-Methyl-3-{3-[1-(3-chlorobenzyl)-4-piperidyl]-propionyl}indole;
1-85. 1-Methyl-3-{3-[1-(3-chlorobenzyl)-4-piperidyl] propionyl}indole hydrochloride;
1-233. 1-Methyl-3-{3-[1-[2-(3-fluorophenyl)ethyl]-4-piperidyl]propionyl}indole;
1-279. 1-Methyl-3-[1-acetoxy-3-(1-benzyl-4-piperidyl)-propyl]indole;

1-303. 1-Methyl-3-{3-[1-(3-chlorobenzyl)-4-piperidyl]-acryloyl}-5-chloroindole;
1-304. 1-Methyl-3-{3-[1-(3-chlorobenzyl)-4-piperidyl]propionyl}-5-chloroindole hydrochloride;
1-308. 1-Methyl-3-{3-[1-(3-fluorobenzyl)-4-piperidyl]acryloyl}-5-chloroindole;
1-309. 1-Methyl-3-{3-[1-(3-fluorobenzyl)-4-piperidyl]propionyl}-5-chloroindole;
1-312. 1-Methyl-3-{3-[1-(3-fluorobenzyl)-4-piperidyl]acryloyl}-5-fluoroindole;
1-313. 1-Methyl-3-{3-[1-(3-fluorobenzyl)-4-piperidyl]propionyl}-5-fluoroindole;
1-314. 1-Methyl-3-{3-[1-(3-fluorobenzyl)-4-piperidyl]propionyl}-5-fluoroindole hydrochloride;
1-315. 1-Methyl-3-{3-[1-(3-fluorobenzyl)-4-piperidyl]propionyl}-5-fluoroindole oxalate;
1-316. 1-Methyl-3-{3-[1-(3-fluorobenzyl)-4-piperidyl]propionyl}-5-fluoroindole trifluoromethanesulphonate;
1-321. 1-Methyl-3-[3-(1-phenethyl-4-piperidyl]propionyl]-5-fluoroindole hydrochloride;
1-363. 1-Methyl-5-[3-(1-benzyl-4-piperidyl)propionyl]-indole;
1-366. 1-Methyl-5-[N-methyl-N-propargylaminomethyl]-3-[3-(1-benzyl-4-piperidyl)propionyl]indole;
2-2. 1-Methyl-3-[3-(1-benzyl-4-piperidyl)propionyl]indazole;
2-3. 1-Methyl-3-[3-(1-benzyl-4-piperidyl)propionyl]indazole hydrochloride;
2-4. 1-Methyl-3-[3-(1-benzyl-4-piperidyl)propionyl]indazole oxalate;
2-95. 1-Methyl-3-{3-[1-(2-chlorobenzyl)-4-piperidyl]-propionyl}indazole;
2-96. 1-Methyl-3-{3-[1-(2-chlorobenzyl)-4-piperidyl]-propionyl}indazole hydrochloride;
2-98. 1-Methyl-3-{3-[1-(3-chlorobenzyl)-4-piperidyl]-propionyl}indazole;
2-99. 1-Methyl-3-{3-[1-(3-chlorobenzyl)-4-piperidyl]-propionyl}indazole hydrochloride;

and pharmaceutically acceptable salts and esters thereof.

The compounds of the present invention may be prepared a variety of processes well known in the art for the preparation of compounds of this type. For example, in general terms, they may be prepared by reacting a compound of formula (A):

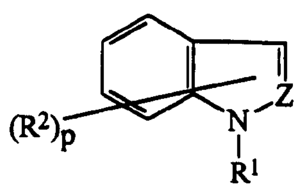

(A)

(in which $R^1$, $R^2$ and Z are as defined above) with a compound to introduce a group of formula -U-V-W (in which U, V and W are as defined above), and then, if desired, converting any group represented by U, V or W to any other group so represented. In more detail, the compounds may be prepared by the following reactions A to D.

*Reaction A:*

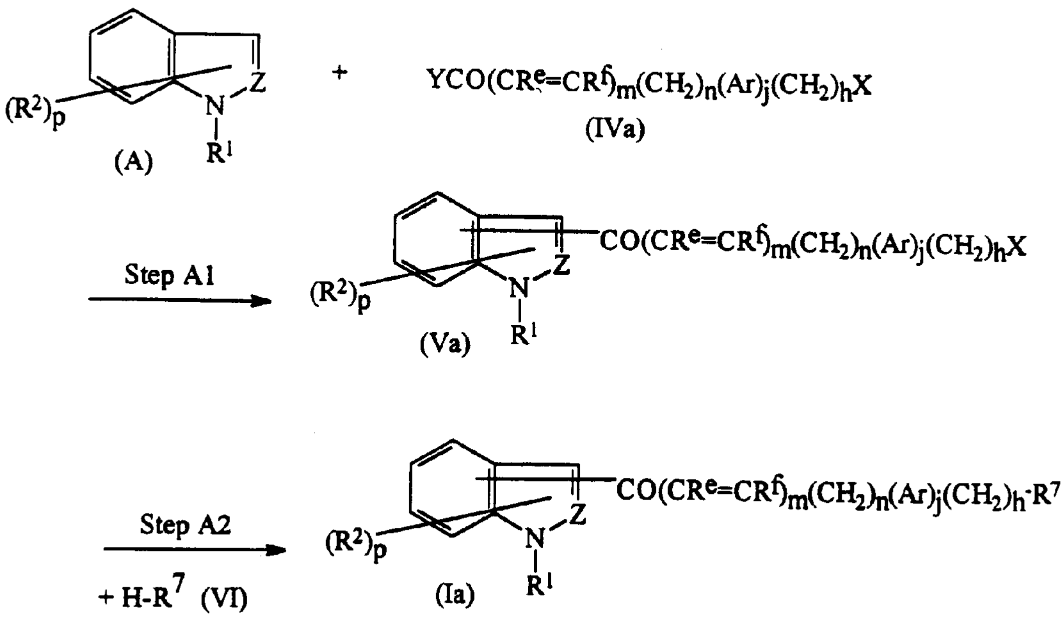


*Reaction B:*

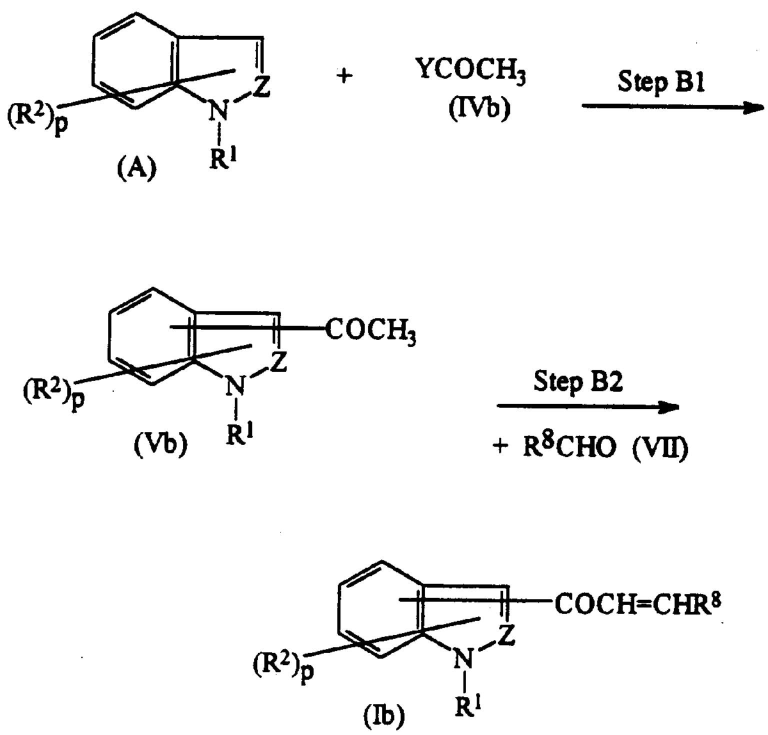

*Reaction C:*

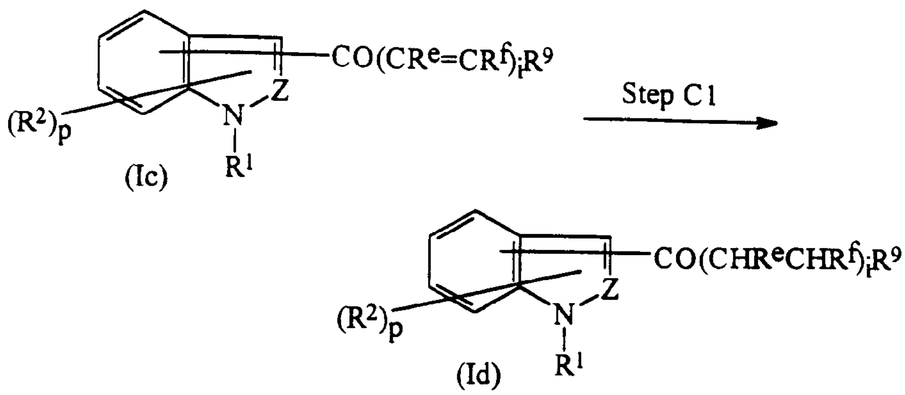


(Ic)

(Id)

*Reaction D:*

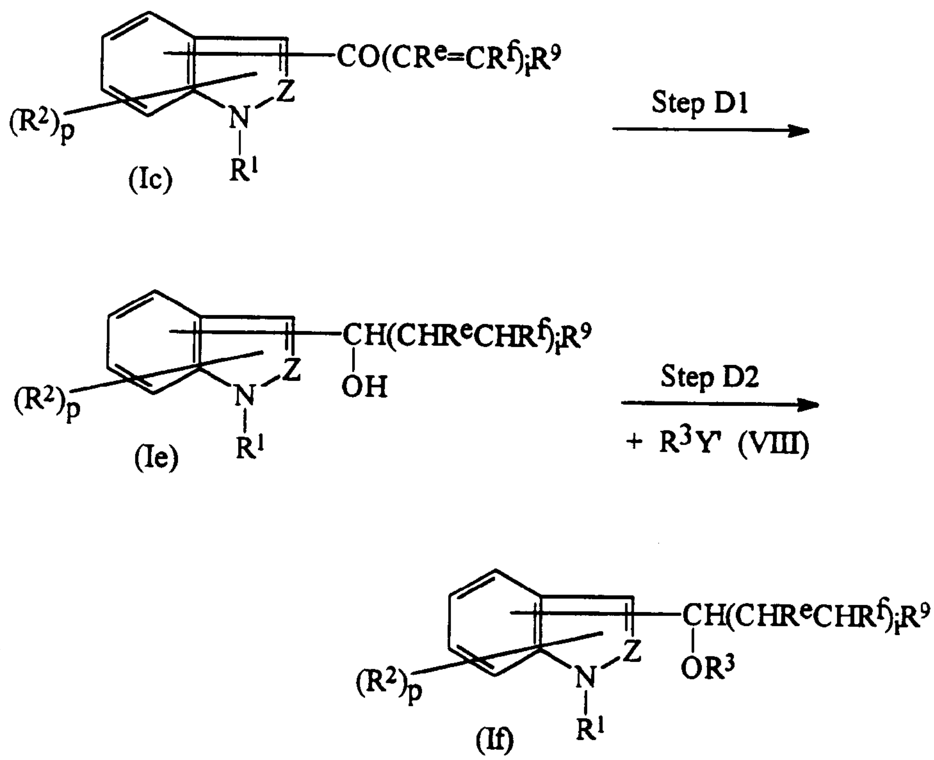


(Ic)

(Ie)

(If)

In the above formulae:

$R^1$, $R^2$, $R^3$, $R^e$, $R^f$, Z, p, m and n are as defined above;

$R^7$ represents a nitrogen-containing heterocyclic group attached to the group represented by V through its ring nitrogen atom or a group represented by the general formula $-NR^4R^5$, in which $R^4$ and $R^5$ are as defined above, among the groups defined for W in the formula (I);

$R^8$ has the same meaning as defined for W;

$R^9$ represents a group represented by $R^8$ or a group of formula $-(CH_2)_n-R^7$, where n and $R^7$ are as defined above;

i is 1 or 2; and

X, Y and Y' each represents a leaving group, that is a group capable of elimination as a nucleophilic residue.

For example, X may represent a halogen atom; a lower alkylsulphonyloxy group; a haloalkylsulphonyloxy

group or an arylsulphonyloxy group. Y may represent a halogen atom and Y' represents a lower alkanoyloxy group or a halogen atom.

Examples of halogen atoms in the definition for X, Y and Y' include the fluorine, chlorine, bromine or iodine atoms, preferably the chlorine atom.

Examples of lower alkylsulphonyloxy groups in the definition for X include alkylsulphonyloxy groups having from 1 to 3 carbon atoms, such as the methylsulphonyloxy, ethylsulphonyloxy and propylsulphonyloxy groups, preferably the methylsulphonyloxy group.

Examples of haloalkylsulphonyloxy groups in the definition for X include haloalkylsulphonyl groups having from 1 to 3 carbon atoms and at least one halogen atom, preferably from 1 to 5 halogen atoms, such as the trifluoromethylsulphonyloxy and pentafluoroethylsulphonyloxy groups, preferably the trifluoromethylsulphonyloxy group.

Examples of arylsulphonyloxy groups in the definition for X include arylsulphonyloxy groups having from 6 to 10 ring carbon atoms but otherwise as defined for $R^a$, such as the benzenesulphonyloxy, p-toluenesulphonyloxy and naphthalenesulphonyloxy groups, preferably the p-toluenesulphonyloxy group.

Examples of lower alkanoyloxy groups in the definition for Y' include alkanoyloxy groups having from 2 to 6 carbon atoms, such as the acetoxy, propionyloxy, butyryloxy, valeryloxy and hexanoyloxy groups, preferably the acetyl group.

Reaction A:

This process prepares a compound of formula (Ia), which is a compound of formula (I) in which $R^1$, $R^2$ and p are as defined above, U represents a group of formula -CO-, V represents a group $-(CR^e=CR^f)_m-$ (in which $R^e$, $R^f$ and m are as defined above) and W represents group of formula

$$-(Ar)_j-(CH_2)_i-R^7$$

where Ar, j, i and $R^7$ are as defined above,

In step A1, the desired compound of formula (Va) can be synthesised by reacting a benzene derivative of formula (A) with a compound of formula (IVa).

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride; ethers, such as tetrahydrofuran; and aromatic hydrocarbons, such as benzene and toluene.

The reaction is also normally carried out in the presence of a Lewis acid (for example, a metal chloride, such as ferric chloride, aluminium chloride, stannous chloride, stannic chloride, titanium chloride or boron trifluoride).

The reaction can take place over a wide range of temperatures, and the precise reaction temperature is not critical to the invention. In general, we find it convenient to carry out the reaction at a temperature of from -30°C to 50°C (more preferably from -10°C to 30°C). The time required for the reaction may also vary widely, depending on many factors, notably the reaction temperature and the nature of the reagents and solvent employed. However, provided that the reaction is effected under the preferred conditions outlined above, a period of from 1 to 3 hours (more preferably from 1 to 2 hours) will usually suffice.

After completion of the reaction, the desired compound can be recovered from the reaction mixture by conventional means. For example, one suitable recovery procedure comprises: mixing the reaction mixture with an organic water-immiscible solvent; washing the organic layer with water; and then removing the solvent by distillation, optionally under reduced pressure. If necessary, the resulting compound of formula (Va) can be further purified by conventional means, such as recrystallisation, reprecipitation or the various chromatography techniques, notably column chromatography.

In step A2, the desired compound of formula (Ia) can be synthesised by reacting the compound of formula (Va) prepared in step A1 with a nitrogen-containing derivative of formula (VI).

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride; ethers, such as tetrahydrofuran; and aromatic hydrocarbons, such as benzene and toluene.

The reaction is normally carried out in the presence of a base, for example: an organic base, such as pyridine, triethylamine or 4-dimethylaminopyridine; a metal alkoxide, such as sodium methoxide; or an alkali metal carbonate, such as sodium carbonate and potassium carbonate. Alternatively, it may be carried out in the presence of potassium iodide.

The reaction can take place over a wide range of temperatures, and the precise reaction temperature is not critical to the invention. In general, we find it convenient to carry out the reaction at a temperature of from -10°C to 150°C (more preferably from 0°C to 80°C). The time required for the reaction may also vary widely, depending on many factors, notably the reaction temperature and the nature of the reagents and solvent employed. However, provided that the reaction is effected under the preferred conditions outlined above, a period of from 1 to 30 hours (more preferably from 2 to 10 hours) will usually suffice.

After completion of the reaction, the compound of the invention can be recovered from the reaction mixture by conventional means. For example, the desired compound can be recovered by adding an organic water-immiscible solvent to the reaction mixture, washing the organic layer with water and distilling off the solvent.

If necessary, the resulting desired compound of formula (Ia) can be further purified by conventional means, such as recrystallisation, reprecipitation or the various chromatography techniques, notably column chromatography.

Reaction B:

This process prepares a compound of formula (Ib), which is a compound of formula (I) in which $R^1$, $R^2$ and p are as defined above; U represents a group of formula -CO-; V represents a group of formula

$$-(CR^e=CR^f)_m-$$

(in which m is 1 and $R^e$ and $R^f$ are both hydrogen atoms); and $R^7$ is as defined above.

In step B1, a compound of formula (A) is reacted with a compound of formula (IVb), to give a compound of formula (Vb). This reaction is essentially the same as that described in step A1 of Reaction A, and may be carried out under the same conditions and using the same solvents and other reagents.

In step B2, the resulting compound of formula (Vb) is reacted with an aldehyde compound of formula (VII). This reaction is an aldol condensation and provides a general method for preparing this type of compound. The reaction is preferably carried out by first reacting the compound of formula (Vb) with a base to produce an anion, and then condensing the anion with an aldehyde of formula (VII).

There is no particular restriction on the nature of the base employed, provided that it has no adverse effect on any other part of the molecule. Example of suitable bases include: organic bases, such as pyridine, triethylamine and 4-dimethylaminopyridine; alkyl metal compounds, such as methyllithium and butyllithium; or metal salts of an amine, such as lithium diisopropylamide, derived from the said alkyl metal compound and diisopropylamine.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride; ethers, such as diethyl ether and tetrahydrofuran; and aromatic hydrocarbons, such as toluene.

The reaction can take place over a wide range of temperatures, and the precise reaction temperature is not critical to the invention. In general, we find it convenient to carry out the reaction at a temperature of from -78°C to 150°C (more preferably from -78°C to 30°C). The time required for the reaction may also vary widely, depending on many factors, notably the reaction temperature and the nature of the reagents and solvent employed. However, provided that the reaction is effected under the preferred conditions outlined above, a period of from 30 minutes to 30 hours (more preferably from 1 to 10 hours) will usually suffice.

After completion of the reaction, the desired compound of the invention can be recovered from the reaction mixture by conventional means. An example of one such technique comprises: adding an organic water-immiscible solvent to the reaction mixture; washing the organic layer with water; and finally distilling off the organic solvent.

If necessary, the compounds obtained can be further purified by conventional means, such as recrystallisation, reprecipitation or the various chromatography techniques, notably column chromatography.

Reaction C:

This process prepares a compound of formula (Id) from a compound of formula (Ic), which is a compound of formula (Ia) or (Ib) in which $R^1$ is as defined above; i is 1 or 2; $R^9$ represents $R^8$ or a group represented by $-(CH_2)_n-R^7$. $R^1$, i and $R^9$ in the compound of formula (Id) are as defined for the compound of formula (Ic).

In step C1, a compound of formula (Ic), which may have been prepared as described in relation to Reaction A or B is reduced, to give a compound of formula (Id). Reduction may be carried out by using a catalyst, such as a catalytic metal (typically palladium, rhodium or platinum), or a chloride or oxide of one of these metals in the absence or presence of a carrier, such as charcoal, in the presence of hydrogen gas, normally in an atmosphere of hydrogen.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; ethers, such as tetrahydrofuran and dioxane; or a mixture of water and any one or more of the above organic solvents.

The pressure of hydrogen used for the catalytic reduction is preferably in the range of from atmospheric pressure to 150 kg/cm.

The reaction can take place over a wide range of temperatures, and the precise reaction temperature is not critical to the invention. In general, we find it convenient to carry out the reaction at a temperature of from 0°C to 50°C. The time required for the reaction may also vary widely, depending on many factors, notably the reaction temperature and the nature of the reagents, catalyst and solvent employed. However, provided that the reaction is effected under the preferred conditions outlined above, a period of from 10 minutes to 18 hours will usually suffice.

Alternatively, in place of catalytic reduction, this reaction may also be carried out using a reducing agent, such as sodium borohydride or lithium borohydride.

This alternative reaction is also normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; ethers, such as tetrahydrofuran and dioxane; and a mixture of water with any one or more of the above solvents.

The reaction can take place over a wide range of temperatures, and the precise reaction temperature is not critical to the invention. In general, we find it convenient to carry out the reaction at a temperature of from -10°C to 50°C (more preferably from 0°C to 10°C). The time required for the reaction may also vary widely, depending on many factors, notably the reaction temperature and the nature of the reagents, reducing agent and solvent employed. However, provided that the reaction is effected under the preferred conditions outlined above, a period of from 10 minutes to 18 hours (more preferably from 30 minutes to 10 hours) will usually suffice.

After completion of the reaction, the desired compound of the invention can be recovered from the reaction mixture by conventional means. If necessary, the compounds obtained can be further purified by conventional means, such as recrystallisation, reprecipitation or the various chromatography techniques, notably column chromatography.

Reaction D:

In this reaction, a a compound of formula (Ie) is prepared from a compound of formula (Ic), and then this is optionally converted to a compound of formula (If). The compounds of formula (Ie) generally include those of formula (Ic) in which $R^1$, i and $R^9$ are as defined for formula (Ic).

In step D1, a compound of formula (Ic) is reduced to give the compound of formula (Ie). The reduction may be carried out using a reducing agent, such as sodium borohydride, lithium borohydride, lithium aluminium hydride, diisobutylaluminium hydride, or aluminium isopropoxide. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; ethers, such as tetrahydrofuran and dioxane; and a mixture of water and any one or more of the above organic solvents. The reaction can take place over a wide range of temperatures, and the precise reaction temperature is not critical to the invention. In general, we find it convenient to carry out the reaction at a temperature of from -10°C to 50°C (more preferably from 0°C to 10°C). The time required for the reaction may also vary widely, depending on many factors, notably the reaction temperature and the nature of the reagents, reducing agent and solvent employed. However, provided that the reaction is effected under the preferred conditions outlined above, a period of from 10 minutes to 18 hours (more preferably from 30 minutes to 10 hours) will usually suffice.

In step D2, a compound of formula (If) is prepared from the compound of formula (Ie). The compounds of formulae (Ie) and (If) generally include those of formula (Ic) in which $R^1$, i and $R^9$ are as defined for formula (Ic); and $R^3$ has the same meaning as defined for formula (I).

In step D2, the compound of formula (Ie), which may have been prepared as described in step D1, is treated with a compound of formula (VIII) in the presence of a base, to introduce a protecting group $R^3$.

The reaction is normally and preferably effected in the presence of a solvent. There is no particular restriction on the nature of the solvent to be employed, provided that it has no adverse effect on the reaction or

on the reagents involved and that it can dissolve the reagents, at least to some extent. Examples of suitable solvents include: halogenated hydrocarbons, especially halogenated aliphatic hydrocarbons, such as methylene chloride and chloroform; ethers, such as diethyl ether, diisopropyl ether and tetrahydrofuran; aromatic hydrocarbons, such as toluene; nitriles, such as acetonitrile; water; and a mixture of water and any one or more of the above organic solvents.

The nature of the compound (VIII) used will, of course, depend on the nature of the protecting group which it is wished to introduce, as is well known to the art. Examples of such reagents of formula (VIII) which may be used include: alkyl halides, such as methyl chloride, methyl iodide, ethyl iodide and propyl iodide; aralkyl halides, such as benzyl chloride, benzyl bromide and benzyl iodide; alkylsulphonyl chlorides, such as methanesulphonyl chloride and ethanesulphonyl chloride; aromatic sulphonyl halides, such as benzenesulphonyl chloride and p-toluenesulphonyl chloride; trialkylsilyl halides, such as trimethylsilyl chloride, trimethylsilyl iodide and dimethyl-t-butylsilyl chloride; aliphatic acyl halides, such as acetyl chloride and propionyl chloride; aromatic halides, such as benzoyl chloride and toluoyl chloride; alkanoyl anhydrides and substituted alkanoyl anhydrides, such as acetic anhydride, trifluoroacetic anhydride and propionic anhydride; or aromatic acid anhydrides, such as benzoic anhydride. Of these, we prefer acetyl chloride.

The reaction is normally carried out in the presence of a base, the nature of which is not critical to the invention, provided that it has no adverse effect on other parts of the molecules of the reagents. Examples of such bases which may be used include alkali metal carbonates, such as sodium carbonate and potassium carbonate; alkali metal hydrogencarbonates, such as sodium hydrogencarbonate and potassium hydrogencarbonate; alkali metal hydroxides, such as sodium hydroxide, potassium hydroxide and lithium hydroxide; tertiary lower alkylamines, such as triethylamine and ethyldiisopropylamine; pyridines, such as pyridine and 4-dimethylaminopyridine; and tertiary alicyclic amines, such as 1,8-diazabicyclo[5,4,0]undecan-7-ene (DBU) and 1,4-diazabicyclo[2,2,2]octane (DABCO). Of these, we prefer the pyridines.

The reaction can take place over a wide range of temperatures, and the precise reaction temperature is not critical to the invention. In general, we find it convenient to carry out the reaction at a temperature of from 0°C to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 15 hours will usually suffice.

After completion of the reaction, the desired compound of the invention can be recovered from the reaction mixture by conventional means. If necessary, the compounds obtained can be further purified by conventional means, such as recrystallisation, reprecipitation or the various chromatography techniques, notably column chromatography.

The compounds of the present invention can be converted to their corresponding salts by conventional salification reactions, such as are well known in the art.

The compounds of the present invention and their salts show excellent inhibitory activity against acetylchlolinesterase and are therefore very useful as novel therapeutic agents improving the condition of patients suffering from Alzheimer's disease or other forms of senile dementia, as shown by the following Experiment.

Assay of acetylcholinesterase and butyrylcholinesterase inhibitory activities

The test animals used were male ddY mice 5 to 7 weeks old (Japan SLC).

The mouse brain was homogenised by means of a 5-fold amount of a 100 mM phosphate buffer (pH 8.0) to prepare an enzyme sample. 10 μℓ of the homogenised sample and 3 ml of the same phosphate buffer were placed in a cuvette, and 10 μℓ of the compound to be tested was added. The resulting mixture was then incubated for 30 minutes at room temperature. Subsequently, 30 mM of acetylthiocholine (a product of Sigma) were added as the substrate for the assay of acetylcholinesterase inhibitory activity. Alternatively, 50 μℓ of a 30 mM preparation of butyrylthiocholine (a product of Sigma) were added as the substrate for the assay of butyrylcholinesterase inhibitory activity. The reaction was initiated by the addition of 50 μℓ of a 10 mM preparation of dithio-bis-nitrobenzoic acid (a product of Sigma).

The inhibitory activity was assayed by observing the change in absorbance of the test sample at 412 μm for 2 to 3 minutes according to Elman's method. The test compound was dissolved in 100% dimethyl sulphoxide for use in the assay.

The amount of each test compound required to inhibit the acetylcholinesterase activities by 50% ($IC_{50}$) are shown in the following Table 3. The compounds of the present invention are identified by the numbers assigned to them in the foregoing Tables 1 and 2.

Table 3

| Acetylcholinesterase inhibiting activity ($IC_{50}$) | |
|---|---|
| Example Compound No. | $IC_{50}$ (nM) |
| 1-2 | 3.3 |
| 1-4 | 10.0 |
| 1-16 | 4.0 |
| 1-28 | 4.0 |
| 1-30 | 9.0 |
| 1-40 | 4.0 |
| 1-78 | 8.0 |
| 1-85 | 6.5 |
| 1-116 | 10.0 |
| 1-304 | 7.5 |
| 1-309 | 8.2 |
| 1-313 | 2.0 |
| 1-314 | 3.4 |
| 1-315 | 2.3 |
| 2-2 | 10.0 |
| 2-3 | 12.0 |
| 2-8 | 13.0 |
| 2-9 | 14.0 |
| 2-23 | 11.0 |
| 2-24 | 10.0 |
| 2-96 | 13.9 |
| 2-99 | 31.7 |
| Tacrine | 153.0 |
| Eseline | 10.0 |

Moreover, the compounds of the present invention demonstrated a very weak butyrylcholinesterase inhibitory activity.

Since the compounds of the present invention listed above have an excellent acetylcholinesterase inhibitory action, a weak butyrylcholinesterase inhibitory activity and a low toxicity, they are useful as a therapeutic agent for dementia, cerebral ischemic injury and various kinds of nervous injury.

The compounds of the present invention may be administered by any route commonly used for compounds having this type of activity and may be formulated in admixture with conventional additives or adjuvants for this purpose. For example, for oral administration, they may be formulated as tablets, capsules, granules, powders or syrups; whilst for parentheral administration, they may be formulated as injections or suppositories.

These pharmaceutical preparations can be prepared by any conventional means using such additives as vehicles, binders, disintegrators, lubricants, stabilizers and corrigents.

Though the dosage may be vary, depending on the age, body weight and symptoms of the patient, for an adult human patient, a suitable daily dosage may be from 1 to 1000 mg/kg body weight per day, which may be adminsitered as a single dose or divided into several doses.

The following two Examples describe the preparation of capsules containing a compound according to the invention. The mesh sizes used are according to the Tyler standard.

Example A

| | |
|---|---|
| Compound of Example 1 | 25 mg |
| Lactose | 154 mg |
| Corn Starch | 100 mg |
| Magnesium Stearate | 1 mg |
| Total | 280 mg |

The above ingredients were thoroughly mixed, after which the mixture was passed through a 60-mesh screen. The screened powder (280 mg) was then packed into No. 3 gelatin capsules.

Example B

| | |
|---|---|
| Compound of Example 116 | 25 mg |
| Lactose | 154 mg |
| Corn Starch | 100 mg |
| Magnesium Stearate | 1 mg |
| Total | 280 mg |

The above ingredients were thoroughly mixed, after which the mixture was passed through a 60-mesh screen. The screened powder (280 mg) was then packed into No. 3 gelatin capsules.

The preparation of certain of the compounds of the invention is further illustrated by the following Examples. The subsequent Preparations illustrate the preparation of certain of the starting materials used in these Examples. Rf values for the compounds were determined using a silica gel plate for thin-layer chromatography (a product of Merck & Co.).

## EXAMPLE 1

1-Methyl-3-[3-(1-benzyl-4-piperidyl)acryloyl]indole (Compound No. 1-1)

1(1) 1-Methyl-3-acetylindole

A solution of 5.3 g of stannic chloride in 15 ml of benzene was added dropwise over a period of 30 minutes to a solution of 1.5 g of acetyl chloride and 1.3 g of 1-methylindole in 20 ml of benzene, whilst ice-cooling. The resulting mixture was then stirred for 30 minutes, after which it was diluted with 50 ml of water and then extracted with ethyl acetate. The ethyl acetate extract was then washed with a saturated aqueous solution of sodium chloride, and dried over anhydrous sodium sulphate. The solvent was then removed by distillation under reduced pressure, and the resulting residue was purified by silica gel column chromatography, using a gradient elution method, with mixtures of ethyl acetate and hexane in proportions of from 1 : 9 to 10 : 0 by volume as the eluent, to give 1.1 g of the title compound as an oil.

1(2) 1-Benzyl-4-methoxymethylenepiperidine

70 ml of a 15% w/v solution of butyllithium in hexane were added to a mixture of 26 g of methoxymethyltriphenylphosphonium chloride and 200 ml of dry diethyl ether, whilst stirring and ice-cooling. The resulting mixture was then stirred for 30 minutes and cooled to -10°C, after which a solution of 14 g of 1-benzyl-4-piperidinone in dry diethyl ether was added to the mixture. After this, the reaction mixture was stirred at 0°C for 2 hours and filtered. The filtrate was extracted with a 1 N aqueous solution of hydrochloric acid, the resulting acidic solution was made basic by the addition of potassium carbonate, and the resulting mixture was then extracted with ethyl acetate. The ethyl acetate extract was dried over anhydrous sodium

sulphate, and the solvent was removed by distillation under reduced pressure. The residue was purified by silica gel column chromatography, using a gradient elution method, with mixtures of ethyl acetate and hexane in proportions of from 1 : 9 to 10 : 0 by volume as the eluent, to give 6.1 g of the title compound as an oil.

1(3) 1-Benzyl-4-piperidinecarbaldehyde

The whole of the 1-benzyl-4-methoxymethylenepiperidine prepared as described in step (2) above, was dissolved in 40 ml of methanol, after which 40 ml of a 1 N aqueous solution of hydrochloric acid were added to the solution. The mixture was heated under reflux for 3 hours and then the solvent was removed by distillation under reduced pressure. The residue was then mixed with diethyl ether and with a saturated aqueous solution of potassium carbonate, after which the mixture was extracted with ethyl acetate. The ethyl acetate extract was then dried over anhydrous potassium carbonate, and the solvent was removed by distillation under reduced pressure. The residue was purified by silica gel column chromatography, using a gradient elution method, with mixtures of ethyl acetate and hexane in proportions of from 1 : 9 to 10 : 0 by volume as the eluent, to give 3.1 g of the title compound as an oil.

1(4) 1-Methyl-3-[3-(1-benzyl-4-piperidyl)acryloyl] indole

7 ml of a 15% w/v solution of butyllithium in hexane were added to a solution of 1 g of diisopropylamine in 10 ml of dry tetrahydrofuran, whilst stirring, and at a temperature of -10°C. The mixture was then stirred whilst ice-cooling for 10 minutes, after which it was cooled to -78°C. A solution of 1.8 g of 1-methyl-3-acetylindole [prepared as described in step (1)] in 10 ml of dry tetrahydrofuran was then added and the mixture was stirred for 30 minutes. A solution of 2.7 g of 1-benzyl-4-piperidinecarbaldehyde [prepared as described in step (3) above] in 20 ml of dry tetrahydrofuran was then added to the mixture. The reaction mixture was then stirred, first at 0°C for 3 hours and then at room temperature for 2 hours. At the end of this time, an aqueous solution of ammonium chloride was added to the mixture. The organic layer was then removed and the aqueous layer was extracted with ethyl acetate. The ethyl acetate extract was then combined with the organic layer removed in the previous step, and the resulting solution was washed with a saturated aqueous solution of sodium chloride and dried over anhydrous sodium sulphate. The solvent was then removed by distillation under reduced pressure. The residue was purified by silica gel column chromatography, using a gradient elution method, with mixtures of ethyl acetate and hexane in proportions of from 1 : 9 to 10 : 0 by volume as the eluent, to give 3.1 g of the title compound as an oil, with an Rf value of 0.40, using ethyl acetate as developing solvent.

EXAMPLE 2

1-Methyl-3-[3-(1-benzyl-4-piperidyl)propionyl]indole (Compound No. 1-2)

1 g of 1-methyl-3-[3-(1-benzyl-4-piperidyl)acryloyl] indole [prepared as described in Example 1] was dissolved in 30 ml of methanol, after which the mixture was hydrogenated at room temperature, in an atmosphere of hydrogen and and under atmospheric pressure, in the presence of 0.1 g of a 10% w/w palladium-on-carbon catalyst. After this, the catalyst was removed by filtration and the solvent was removed by distillation under reduced pressure. The residue was then purified by silica gel column chromatography, using a gradient elution method, with mixtures of ethyl acetate and hexane in proportions of from 1 : 9 to 10 : 0 by volume as the eluent, to give 0.9 g of the title compound as an oil, with an Rf value of 0.27, using ethyl acetate as developing solvent.

EXAMPLE 3

1-Methyl-3-[3-(1-benzyl-4-piperidyl)propionyl]indole hydrochloride Compound No. 1-3)

0.3 ml of a 4 N solution of hydrogen chloride in dioxane was added to a solution of 0.3 g of 1-methyl-3-[3-(1-benzyl-4-piperidyl)propionyl]indole [prepared as described in Example 2] in 5 ml of methylene chloride, whilst ice-cooling. The solvent was then removed by distillation under reduced pressure, and the residue was recrystallised from a mixture of methanol and diethyl ether, to give 0.21 g of the title compound, melting at 147-148°C.

EXAMPLE 4

1-Methyl-3-[3-(1-benzyl-4-piperidyl)propionyl]indole oxalate (Compound No. 1-4)

A solution of 0.9 g of oxalic acid in 10 ml of methanol was added to a solution of 0.36 g of 1-methyl-3-[3-(1-benzyl-4-piperidyl)propionyl]indole hydrochloride [prepared as described in Example 3] in 5 ml of methylene chloride, whilst ice-cooling. The solvent was then removed by distillation under reduced pressure, and the residue was recrystallised from a 1 : 1 by volume mixture of methanol and diethyl ether, to give 0.34 g of the title compound, melting at 150-151°C.

EXAMPLE 5

1,2-Dimethyl-3-[3-(1-benzyl-4-piperidyl)acryloyl]indole (Compound No. 1-5)

A procedure similar to that described in Example 1 was repeated, but using 1,2-dimethylindole instead of 1-methylindole, in relative proportions similar to those used in that Example, to obtain the title compound, melting at 101-102°C.

EXAMPLE 6

1,2-Dimethyl-3-[3-(1-benzyl-4-piperidyl)propionyl]indole (Compound No. 1-6)

A procedure similar to that described in Example 2 was repeated, but using 1,2-dimethyl-3-[3-(1-benzyl-4-piperidyl)acryloyl]indole [prepared as described in Example 5] instead of 1-methyl-3-[3-(1-benzyl-4-piperidyl)acryloyl]indole, in relative proportions similar to those used in that Example, to obtain the title compound as an oil, with an Rf value of 0.50, using a 1 : 1 by volume mixture of ethyl acetate and hexane as developing solvent.

EXAMPLE 7

1,5-Dimethyl-3-[3-(1-benzyl-4-piperidyl)acryloyl]indole (Compound No. 1-9)

A procedure similar to that described in Example 1 was repeated, but using 1,5-dimethylindole instead of 1-methylindole, in relative proportions similar to those used in that Example, to obtain the title compound, melting at 145-146°C.

EXAMPLE 8

1,5-Dimethyl-3-[3-(1-benzyl-4-piperidyl)propionyl]indole (Compound No. 1-10)

A procedure similar to that described in Example 2 was repeated, but using 1,5-dimethyl-3-[3-(1-benzyl-4-piperidyl)acryloyl]indole [prepared as described in Example 7] instead of 1-methyl-3-[3-(1-benzyl-4-piperidyl)acryloyl]indole, in relative proportions similar to those used in that Example, to obtain the title compound as an oil, with an Rf value of 0.50, using ethyl acetate as developing solvent.

EXAMPLE 9

1-Methyl-3-[3-(1-benzyl-4-piperidyl)acryloyl]-5-methoxyindole (Compound No. 1-21)

A procedure similar to that described in Example 1 was repeated, but using 1-methyl-5-methoxyindole instead of 1-methylindole, in relative proportions similar to those used in that Example, to obtain the title compound, melting at 121-122°C.

EXAMPLE 10

1-Methyl-3-[3-(1-benzyl-4-piperidyl)propionyl]-5-methoxyindole (Compound No. 1-22)

A procedure similar to that described in Example 2 was repeated, but using 1-methyl-3-[3-(1-benzyl-4-piperidyl)acryloyl]-5-methoxyindole [prepared as described in Example 9] instead of 1-methyl-3-[3-(1-benzyl-4-piperidyl)acryloyl]indole, in relative proportions similar to those used in that Example, to obtain the title compound as an oil, with an Rf value of 0.21, using ethyl acetate as developing solvent.

EXAMPLE 11

1-Methyl-3-[3-(1-benzyl-4-piperidyl)acryloyl]-5-chloroindole (Compound No. 1-27)

A procedure similar to that described in Example 1 was repeated, but using 5-chloro-1-methylindole instead of 1-methylindole, in relative proportions similar to those used in that Example, to obtain the title compound, melting at 107-108°C.

EXAMPLE 12

1-Methyl-3-[3-(1-benzyl-4-piperidyl)propionyl]-5-chloroindole (Compound No. 1-28)

A procedure similar to that described in Example 2 was repeated, but using 1-methyl-3-[3-(1-benzyl-4-piperidyl)acryloyl]-5-chloroindole [prepared as described in Example 11] instead of 1-methyl-3-[3-(1-benzyl-4-piperidyl)acryloyl]indole, in relative proportions similar to those used in that Example, to obtain the title compound as an oil, with an Rf value of 0.26, using a 9 : 1 by volume mixture of ethyl acetate and methanol as developing solvent.

EXAMPLE 13

1-Methyl-3-[3-(1-benzyl-4-piperidyl)acryloyl]-6-chloroindole (Compound No. 1-29)

A procedure similar to that described in Example 1 was repeated, but using 6-chloro-1-methylindole instead of 1-methylindole, in relative proportions similar to those used in that Example, to obtain the title compound, melting at 122-123°C.

EXAMPLE 14

1-Methyl-3-[3-(1-benzyl-4-piperidyl)propionyl]-6-chloroindole (Compound No. 1-30)

A procedure similar to that described in Example 2 was repeated, but using 1-methyl-3-[3-(1-benzyl-4-piperidyl)acryloyl]-6-chloroindole [prepared as described in Example 13] instead of 1-methyl-3-[3-(1-benzyl-4-piperidyl)acryloyl]indole, in relative proportions similar to those used in that Example, to obtain the title compound, melting at 90-91°C.

EXAMPLE 15

1-Methyl-3-[3-(1-benzyl-4-piperidyl)acryloyl]-5-fluoroindole (Compound No. 1-39)

A procedure similar to that described in Example 1 was repeated, but using 5-fluoro-1-methylindole instead of 1-methylindole, in relative proportions similar to those used in that Example, to obtain the title compound as an oil, with an Rf value of 0.23, using ethyl acetate as developing solvent.

EXAMPLE 16

1-Methyl-3-[3-(1-benzyl-4-piperidyl)propionyl]-5-fluoroindole (Compound No. 1-40)

A procedure similar to that described in Example 2 was repeated, but using 1-methyl-3-[3-(1-benzyl-4-piperidyl)acryloyl]-5-fluoroindole [prepared as described in Example 15] instead of 1-methyl-3-[3-(1-benzyl-4-piperidyl)acryloyl]indole, in relative proportions similar to those used in that Example, to obtain the title compound as an oil, with an Rf value of 0.17, using ethyl acetate as developing solvent.

EXAMPLE 17

1-Methyl-3-{3-[1-(3-fluorobenzyl)-4-piperidyl]-acryloyl}indole (Compound No. 1-77)

17(1) 8-(3-Fluorobenzyl)-1,4-dioxa-8-azaspiro[4.5]decane

An aqueous solution of 4.3 g of sodium carbonate in 20 ml of water was added to a solution of 2.86 g

of 1,4-dioxa-8-azaspiro[4.5]decane in 20 ml of toluene. A solution of 1.9 g of 3-fluorobenzyl bromide in toluene was then slowly added dropwise to the mixture at 0°C, whilst stirring. After stirring at room temperature for 40 minutes, the toluene layer was removed and washed with an aqueous solution of sodium hydrogencarbonate and a saturated aqueous solution of sodium chloride, in that order, after which the reaction mixture was dried over anhydrous sodium sulphate. The drying agent was then removed by filtration and the filtrate was separated from the solvent by distillation under reduced pressure. The residue was purified by silica gel column chromatography, using a gradient elution method, with mixtures of ethyl acetate and hexane in proportions of from 1 : 9 to 10 : 0 by volume as the eluent, to give 3.3 g of the title compound as an oil.

17(2) 1-(3-Fluorobenzyl)-4-piperidinone

1 ml of a 2 N aqueous solution of hydrochloric acid was added to a solution of 1.8 g of 8-(3-fluorobenzyl)-1,4-dioxa-8-azaspiro[4.5]decane [prepared as described in step (1), above] in 15 ml of acetone, and the resulting mixture was stirred at 35°C for 90 minutes. The reaction mixture was then diluted with 15 ml of water, after which half of the solvent in the diluted mixture was removed by distillation under reduced pressure. The concentrate was then extracted with diethyl ether, and the extract was washed with an aqueous solution of sodium hydrogencarbonate and a saturated aqueous solution of sodium chloride, in that order, after which it was dried over anhydrous sodium sulphate. The drying agent was removed by filtration, and the filtrate was removed from the solvent by distillation under reduced pressure. The residue was then purified by silica gel column chromatography, using a gradient elution method, with mixtures of ethyl acetate and hexane in proportions of from 1 : 9 to 10 : 0 by volume as the eluent, to give 1.3 g of the title compound as an oil.

17(3) 1-(3-Fluorobenzyl)-4-methoxymethylenepiperidine

A procedure similar to that described in step (2) of Example 1 was then repeated, but using 1-(3-fluorobenzyl)-4-piperidinone [prepared as described in step (2), above] instead of 1-benzyl-4-piperidinone, in relative proportions similar to those used in that Example, to obtain the title compound as an oil.

17(4) 1-(3-Fluorobenzyl)-4-piperidinecarbaldehyde

A procedure similar to that described in step (3) of Example 1 was then repeated, but using 1-(3-fluorobenzyl)-4-methoxymethylenepiperidine [prepared as described in step (3), above] instead of 1-benzyl-4-methoxymethylenepiperidine, in relative proportions similar to those used in that Example, to obtain the title compound as an oil.

17(5) 1-Methyl-3-{3-[1-(3-fluorobenzyl)-4-piperidyl]acryloyl}indole

A procedure similar to that described in step (4) of Example 1 was repeated, but using 1-(3-fluorobenzyl)-4-piperidinecarbaldehyde [prepared as described in step (4), above] instead of 1-benzyl-4-piperidine-carbaldehyde, to obtain the title compound as an oil with an Rf value of 0.31 using ethyl acetate as developing solvent.

EXAMPLE 18

1-Methyl-3-{3-[1-(3-fluorobenzyl)-4-piperidyl]propionyl}indole (Compound No. 1-78)

A procedure similar to that described in Example 2 was repeated, but using 1-methyl-3-{3-[1-(3-fluorobenzyl)-4-piperidyl]acryloyl}indole [prepared as described in Example 17] instead of 1-methyl-3-[3-(1-benzyl-4-piperidyl)acryloyl]indole, in relative proportions similar to those used in that Example, to obtain the title compound as an oil, with an Rf value of 0.30, using ethyl acetate as developing solvent.

EXAMPLE 19

1-Methyl-3-{3-[1-(2-thienylmethyl)-4-piperidyl]acryloyl}indole (Compound No. 1-115)

A procedure similar to that described in Example 17 was repeated, but using 2-thienylmethyl chloride instead of 3-fluorobenzyl bromide, in relative proportions similar to those used in that Example, to obtain the title compound as an oil, with an Rf value of 0.32, using ethyl acetate as developing solvent.

EXAMPLE 20

1-Methyl-3-{3-[1-(2-thienylmethyl)-4-piperidyl]propionyl}indole (Compound No. 1-116)

A procedure similar to that described in Example 2 was repeated, but using 1-methyl-3-{3-[1-(2-thienylmethyl)-4-piperidyl]acryloyl}indole [prepared as described in Example 19] instead of 1-methyl-3-[3-(1-benzyl-4-piperidyl)acryloyl]indole, in relative proportions similar to those used in that Example, to obtain the title compound as an oil, with an Rf value of 0.25, using ethyl acetate as developing solvent.

EXAMPLE 21

1-Methyl-3-{3-[1-(3-methoxybenzyl)-4-piperidyl]acryloyl}indole (Compound No. 1-102)

A procedure similar to that described in Example 17 was repeated, but using 3-methoxybenzyl chloride instead of 3-fluorobenzyl bromide, in relative proportions similar to those used in that Example, to obtain the title compound as an oil with an Rf value of 0.15, using ethyl acetate as developing solvent.

EXAMPLE 22

1-Methyl-3-{3-[3-(3-methoxybenzyl)-4-piperidyl]propionyl}indole (Compound No. 1-103)

A procedure similar to that described in Example 2 was repeated, but using 1-methyl-3-{3-[1-(3-methoxybenzyl)-4-piperidyl]acryloyl}indole [prepared as described in Example 21] instead of 1-methyl-3-[3-(1-benzyl-4-piperidyl)acryloyl]indole, in relative proportions similar to those used in that Example, to obtain the title compound as an oil, with an Rf value of 0.14 using ethyl acetate as developing solvent.

EXAMPLE 23

1-Methyl-3-[3-(1-methyl-4-piperidyl)acryloyl]indole (Compound No. 1-145)

A procedure similar to that described in Example 17 was repeated, but using methyl iodide instead of 3-fluorobenzyl bromide, in relative proportions similar to those used in that Example, to obtain the title compound as a glassy material with an Rf value of 0.11, using ethyl acetate as developing solvent.

EXAMPLE 24

1-Methyl-3-[3-(1-methyl-4-piperidyl)propionyl]indole (Compound No. 1-146)

A procedure similar to that described in Example 2 was repeated, but using 1-methyl-3-[3-(1-methyl-4-piperidyl)acryloyl]indole [prepared as described in Example 23] instead of 1-methyl-3-[3-(1-benzyl-4-piperidyl)acryloyl]indole, in relative proportions similar to those used in that Example, to obtain the title compound as an oil, with an Rf value of 0.27, using a 9 : 1 by volume mixture of methylene chloride and methanol as developing solvent.

EXAMPLE 25

1-Methyl-3-[3-(1-isobutyl-4-piperidyl)acryloyl]indole (Compound No. 1-152)

A procedure similar to that described in Example 17 was repeated, but using isobutyl iodide instead of 3-fluorobenzyl bromide, in relative proportions similar to those used in that Example, to obtain the title compound as an oil, with an Rf value of 0.13, using a 9 : 1 by volume mixture of methylene chloride and methanol as developing solvent.

EXAMPLE 26

1-Methyl-3-[3-(1-isobutyl-4-piperidyl)propionyl]indole (Compound No. 1-153)

A procedure similar to that described in Example 2 was repeated, but using 1-methyl-3-[3-(1-isobutyl-4-piperidyl)acryloyl]indole [prepared as described in Example 25] instead of 1-methyl-3-[3-(1-benzyl-4-piperidyl)acryloyl]indole, in relative proportions similar to those used in that Example, to obtain the title compound as an oil, with an Rf value of 0.15, using a 9 : 1 by volume mixture of methylene chloride and methanol as developing solvent.

EXAMPLE 27

1-Ethyl-3-[3-(1-benzyl-4-piperidyl)acryloyl]indole (Compound No. 1-158)

A procedure similar to that described in Example 1 was repeated, but using 1-ethylindole instead of 1-methylindole, in relative proportions similar to those used in that Example, to obtain the title compound, melting at 125-127°C.

EXAMPLE 28

1-Ethyl-3-[3-(1-benzyl-4-piperidyl)propionyl)indole (Compound No. 1-159)

A procedure similar to that described in Example 2 was repeated, but using 1-ethyl-3-[3-(1-benzyl-4-piperidyl)acryloyl]indole [prepared as described in Example 27] instead of 1-methyl-3-[3-(1-benzyl-4-piperidyl)acryloyl]indole, in relative proportions similar to those used in that Example, to obtain the title compound as an oil, with an Rf value of 0.37, using ethyl acetate as developing solvent.

EXAMPLE 29

1-Isobutyl-3-[3-(1-benzyl-4-piperidyl)acryloyl]indole (Compound No. 1-208)

A procedure similar to that described in Example 1 was repeated, but using 1-isobutylindole instead of 1-methylindole, in relative proportions similar to those used in that Example, to obtain the title compound, melting at 98-99°C.

EXAMPLE 30

Isobutyl-3-[3-(1-benzyl-4-piperidyl)propionyl]indole (Compound No. 1-209)

A procedure similar to that described in Example 2 was repeated, but using 1-isobutyl-3-[3-(1-benzyl-4-piperidyl)acryloyl]indole [prepared as described in Example 29] instead of 1-methyl-3-[3-(1-benzyl-4-piperidyl)acryloyl]indole, in relative proportions similar to those used in that Example, to obtain the title compound as an oil, with an Rf value of 0.45, using ethyl acetate as developing solvent.

EXAMPLE 31

1-Benzyloxycarbonyl-3-[3-(1-benzyl-4-piperidyl)acryloyl]indole (Compound No. 1-216)

31(1) 1-Benzyloxycarbonyl-3-acetylindole

1.5 g of sodium hydride (as a 50% dispersion in mineral oil) were added to a solution of 3.5 g of 3-acetylindole in 50 ml of dimethylformamide, whilst ice-cooling and stirring. The resulting mixture was stirred for 30 minutes, after which 5.3 g of benzyloxycarbonyl chloride were added dropwise. After the mixture had been stirred for one hour, it was poured into ice-water and extracted with diethyl ether. The diethyl ether extract was then dried over anhydrous sodium sulphate, and the solvent was removed by distillation under reduced pressure. The residue was purified by silica gel column chromatography, using a gradient elution method, with mixtures of ethyl acetate and hexane in proportions of from 1 : 9 to 10 : 0 by volume as the eluent, to give 5.1 g of the title compound as an oil.

31(2) 1-Benzyloxycarbonyl-3-[3-(1-benzyl-4-piperidyl)acryloyl]indole

A procedure similar to that described in step (4) of Example 1 was repeated, but using 1-benzyloxycarbonyl-3-acetylindole [prepared as described in step (1)] in relative proportions similar to those described in that Example, to obtain the title compound as an oil, with an Rf value of 0.52, using a 1 : 1 by volume mixture of hexane and ethyl acetate as developing solvent.

EXAMPLE 32

3-[3-(1-Benzyl-4-piperidyl)propionyl]indole (Compound No. 1-218)

A procedure similar to that described in Example 2 was repeated, but using 1-benzyloxycarbonyl-3-[3-(1-

benzyl-4-piperidyl)acryloyl]indole [prepared as described in Example 31] instead of 1-methyl-3-[3-(1- benzyl-4-piperidyl)acryloyl]indole, in relative proportions similar to those used in that Example, to obtain the title compound as an oil, melting at 129-131°C.

EXAMPLE 33

1-Methyl-3-[3-(1-benzyl-3-pyrrolidinyl)acryloyl]indole (Compound No. 1-219)

33(1) 1-Benzyl-3-pyrrolidinonecarbaldehyde

A procedure similar to that described in steps (2) and (3) of Example 1 was repeated, but using 1-benzyl-3-pyrrolidinone instead of 1-benzyl-4-piperidinone, in relative proportions similar to those used in that Example, to obtain the title compound as an oil, with an Rf value of 0.16, using a 2 : 1 by volume mixture of hexane and ethyl acetate as developing solvent.

33(2) 1-Methyl-3-[3-(1-benzyl-3-pyrrolidinyl)acryloyl]indole

A procedure similar to that described in step (4) of Example 1 was repeated, but using 1-benzyl-3-pyrrolidinonecarbaldehyde [prepared as described in step (1)] and 3-acetyl-1-methylindole as starting materials, in relative proportions similar to those used in that Example, to obtain the title compound as an oil, with an Rf value of 0.39, using a 2 : 1 by volume mixture of hexane and ethyl acetate as developing solvent.

EXAMPLE 34

1-Methyl-3-[3-(1-benzyl-3-pyrrolidyl)propionyl]indole (Compound No. 1-220)

A procedure similar to that described in Example 2 was repeated, but using 1-methyl-3-[3-(1-benzyl-3-pyrrolidinyl)acryloyl]indole [prepared as described in Example 33], instead of 1-methyl-3-[3-(1-benzyl-4-piperidyl)acryloyl]indole, in relative proportions similar to those used in that Example, to obtain the title compound as an oil, with an Rf value of 0.05, using ethyl acetate as developing solvent.

EXAMPLE 35

1-Methyl-3-[3-(1-phenethyl-4-piperidyl)acryloyl]indole (Compound No. 1-221)

A procedure similar to that described in Example 17 was repeated, but using phenethyl triflate instead of 3-fluorobenzyl bromide, in relative proportions similar to those used in that Example, to obtain the title compound as an oil, with an Rf value of 0.58, using a 9 : 1 by volume mixture of methylene chloride and methanol as developing solvent.

EXAMPLE 36

1-Methyl-3-[3-(1-phenethyl-4-piperidyl)propionyl]indole (Compound No. 1-222)

A procedure similar to that described in Example 2 was repeated, but using 1-methyl-3-[3-(1-phenethyl-4-piperidyl)acryloyl]indole [prepared as described in Example 35] instead of 1-methyl-3-[3-(1-benzyl-4-piperidyl)acryloyl]indole, in relative proportions similar to those used in that Example, to obtain the title compound as an oil, with an Rf value of 0.55, using a 9 : 1 by volume mixture of methylene chloride and methanol as developing solvent.

EXAMPLE 37

1-Methyl-3-[3-(1-phenethyl-4-piperidyl)acryloyl)-5-, chloroindole (Compound No. 1-223)

37(1) 1-Phenethyl-4-piperidinecarbaldehyde

A procedure similar to that described in steps (2) and (3) of Example 1 was repeated, but using 1-phenethyl-4-piperidinone instead of 1-benzyl-4-piperidinone, in relative proportions similar to those used in that Example, to obtain the title compound as an oil, with an Rf value of 0.27, using ethyl acetate as developing solvent.

37(2) 1-Methyl-3-[3-(1-phenethyl-4-piperidyl)acryloyl]-5-chloroindole

A procedure similar to that described in step (4) of Example 1 was repeated), but using 1-phenethyl-4-piperidinecarbaldehyde [prepared as described in step (1), above] instead of 1-benzyl-4-piperidinecarbaldehyde, in relative proportions similar to those used in that Example, to obtain the title compound as an oil, with an Rf value of 0.60, using a 9 : 1 by volume mixture of methylene chloride and methanol as developing solvent.

EXAMPLE 38

1-Methyl-3-[3-(1-phenethyl-4-piperidyl)propionyl]-5-chloroindole (Compound No. 1-224)

A procedure similar to that described in Example 2 was repeated, but using 1-methyl-3-[3-(1-phenethyl-4-piperidyl)acryloyl)-5-chloroindole [prepared as described in Example 37] instead of 1-methyl-3-[3-(1-benzyl-4-piperidyl)acryloyl]indole, in relative proportions similar to those used in that Example, to obtain the title compound, melting at 129-131°C.

EXAMPLE 39

1-Methyl-3-[3-(1-phenethyl-4-piperidyl)acryloyl]-6-chloroindole (Compound No. 1-225)

A procedure similar to that described in Example 37 was repeated, but using 6-chloro-1-methylindole instead of 3-acetyl-5-chloro-1-methylindole, in relative proportions similar to those used in that Example, to obtain the title compound as an oil, with an Rf value of 0.43, using a 9 : 1 by volume mixture of methylene chloride and methanol as developing solvent.

EXAMPLE 40

1-Methyl-3-[3-(1-phenethyl-4-piperidyl)propionyl]-6-chloroindole (Compound No. 1-226)

A procedure similar to that described in Example 2 was repeated, but using 1-methyl-3-[3-(1-phenethyl-4-piperidyl)acryloyl]-6-chloroindole [prepared as described in Example 39] instead of 1-methyl-3-[3-(1-benzyl-4-piperidyl)acryloyl]indole, in relative proportions similar to those used in that Example, to obtain the title compound, melting at 179-180°C (with decomposition).

EXAMPLE 41

1-Methyl-3-[3-(1-phenethyl-4-piperidyl)acryloyl]-5-methoxyindole (Compound No. 1-227)

A procedure similar to that described in Example 37 was repeated, but using 5-methoxy-1-methylindole instead of 3-acetyl-5-chloro-1-methylindole, in relative proportions similar to those used in that Example, to obtain the title compound as an oil, with an Rf value of 0.65, using a 9 : 1 by volume mixture of methylene chloride and methanol as developing solvent.

EXAMPLE 42

1-Methyl-3-[3-(1-phenethyl-4-piperidyl)propionyl]-5-methoxyindole (Compound No. 1-228)

A procedure similar to that described in Example 2 was repeated, but using 1-methyl-3-[3-(1-phenethyl-4-piperidyl)acryloyl]-5-methoxyindole [prepared as described in Example 41] instead of 1-methyl-3-[3-(1-benzyl-4-piperidyl)acryloyl]indole, in relative proportions similar to those used in that Example, to obtain the title compound as an oil, with an Rf value of 0.56, using a 9 : 1 by volume mixture of methylene chloride and methanol as developing solvent.

EXAMPLE 43

1-Methyl-3-[3-(1-phenethyl-4-piperidyl)acryloyl]-5-methoxyindole hydrochloride (Compound No. 1-229)

A procedure similar to that described in Example 3 was repeated, but using 1-methyl-3-[3-(1-phenethyl-4-piperidyl)acryloyl]-5-methoxyindole [prepared as described in Example 42] instead of 1-methyl-3-[3-(1-benzyl-4-piperidyl)propionyl]indole, in relative proportions similar to those used in that Example, to obtain the title

compound as an amorphous solid, with an Rf value of 0.50, using a 9 : 1 by volume mixture of methylene chloride and methanol as developing solvent.

EXAMPLE 44

1-Methyl-3-{3-[1-(3-chlorophenyl)ethyl-4-piperidyl]propionyl}indole (Compound No. 1-238)

A procedure similar to that described in Example 17 was repeated, but using 2-(3-chlorophenyl)ethyl triflate instead of 3-fluorobenzyl bromide, in relative proportions similar to those used in that Example, to obtain the title compound as an oil, with an Rf value of 0.51, using a 9 : 1 by volume mixture of methylene chloride and methanol as developing solvent.

EXAMPLE 45

1-Methyl-3-{3-[1-(3-chlorophenyl)ethyl-4-piperidyl]propionyl}indole (Compound No. 1-239)

A procedure similar to that described in Example 2 was repeated, but using 1-methyl-3-{3-[1-(3-chlorophenyl)ethyl-4-piperidyl]-propionyl}indole [prepared as described in Example 44] instead of 1-methyl-3-[3-(1-benzyl-4-piperidyl)acryloyl]indole, in relative proportions similar to those used in that Example, to obtain the title compound as an oil.

EXAMPLE 46

1-Methyl-3-{3-[1-(3-chlorophenyl)ethyl-4-piperidyl]propionyl}indole hydrochloride (Compound No. 1-243)

A procedure similar to that described in Example 3 was repeated, but using 1-methyl-3-{3-[1-(3-chlorophenyl)ethyl-4-piperidyl]-propionyl}indole [prepared as described in Example 45] instead of 1-methyl-3-[3-(1-benzyl-4-piperidyl)propionyl]indole, in relative proportions similar to those used in that Example, to obtain the title compound as an amorphous solid, with an Rf value of 0.51, using a 9 : 1 by volume mixture of methylene chloride and methanol as developing solvent.

EXAMPLE 47

1-Methyl-3-{3-[1-(3-trifluoromethylphenyl)ethyl-4-piperidyl]acryloyl}indole (Compound No. 1-244)

A procedure similar to that described in Example 17 was repeated, but using 2-(3-trifluoromethylphenyl)-ethyl triflate and 1-methylindole as starting materials, in relative proportions similar to those used in that Example, to obtain the title compound as an oil.

EXAMPLE 48

1-Methyl-3-{3-[1-(3-trifluoromethylphenyl)ethyl-4-piperidyl]propionyl}indole (Compound No. 1-245)

A procedure similar to that described in Example 17 was repeated, but using 2-(3-trifluoromethylphenyl)-ethyl triflate and 1-methylindole as starting materials, in relative proportions similar to those used in that Example, to obtain the title compound as an oil, with an Rf value of 0.54, using a 9 : 1 by volume mixture of methylene chloride and methanol as developing solvent.

EXAMPLE 49

1-Methyl-3-{3-[1-(3-trifluoromethylphenyl)ethyl-4-piperidyl]propionyl}indole hydrochloride (Compound No. 1-246)

A procedure similar to that described in Example 3 was repeated, but using 1-methyl-3-{3-[1-(3-trifluoromethylphenyl)ethyl-4-piperidyl]propionyl}indole [prepared as described in Example 48] instead of 1-methyl-3-[3-(1-benzyl-4-piperidyl)propionyl]indole, in relative proportions similar to those used in that Example, to obtain the title compound as an amorphous solid, with an Rf value of 0.54, using a 9 : 1 by volume mixture of methylene chloride and methanol as developing solvent.

EXAMPLE 50

1-Ethyl-3-[3-(1-phenethyl-4-piperidyl)acryloyl]indole (Compound No. 1-247)

A procedure similar to that described in Example 37 was repeated, but using 1-ethylindole instead of 3-acetyl-5-chloro-1-methylindole, in relative proportions similar to those used in that Example, to obtain the title compound as an oil, with an Rf value of 0.59, using a 9 : 1 by volume mixture of methylene chloride and methanol as developing solvent.

EXAMPLE 51

1-Ethyl-3-[3-(1-phenethyl-4-piperidyl)propionyl]indole (Compound No. 1-248)

A procedure similar to that described in Example 2 was repeated, but using 1-ethyl-3-[3-(1-phenethyl-4-piperidyl)acryloyl]indole [prepared as described in Example 50] instead of 1-methyl-3-[3-(1-benzyl-4-piperidyl)acryloyl]indole, in relative proportions similar to those used in that Example, to obtain the title compound as an oil, with an Rf value of 0.45, using a 9 : 1 by volume mixture of methylene chloride and methanol as developing solvent.

EXAMPLE 52

1-Ethyl-3-[3-(1-phenethyl-4-piperidyl)propionyl]indole hydrochloride (Compound No. 1-249)

A procedure similar to that described in Example 3 was repeated, but using 1-ethyl-3-[3-(1-phenethyl-4-piperidyl)propionyl]indole [prepared as described in Example 51] instead of 1-methyl-3-[3-(1-benzyl-4-piperidyl)propionyl]indole, in relative proportions similar to those used in that Example, to obtain the title compound as an oil, with an Rf value of 0.45, using a 9 : 1 by volume mixture of methylene chloride and methanol as developing solvent.

EXAMPLE 53

1-Methyl-3-[6-(N-methyl-N-benzylamino)hexanoyl]indole (Compound No. 1-250)

53(1) 1-Methyl-3-(6-bromohexanoyl)indole

A procedure similar to that described in step (1) of Example 1 was repeated, but using 6-bromohexanoyl chloride and 1-methylindole as starting materials, in relative proportions similar to those used in that Example, to obtain the title compound, melting at 63-64°C.

53(2) 1-Methyl-3-[6-(N-methyl-N-benzylamino)hexanoyl]indole

A mixture of 1 g of 1-methyl-3-(6-bromohexanoyl)-indole [prepared as described in step (1)], 0.7 g of N-methyl-N-benzylamine and 0.3 g of potassium iodide in 20 ml of tetrahydrofuran was heated under reflux for 8 hours. The solvent was then removed from the reaction mixture by distillation under reduced pressure, after which the residue was diluted with 5 ml of water and the resulting solution was extracted with ethyl acetate. The extract was then dried over anhydrous sodium sulphate, and the solvent was removed by distillation under reduced pressure. The residue was purified by silica gel column chromatography, using a gradient elution method, with mixtures of ethyl acetate and hexane in proportions of from 1 : 9 to 10 : 0 by volume as the eluent, to give 1.1 g of the title compound as an oil, with an Rf value of 0.31, using a 1 : 1 by volume mixture of ethyl acetate and hexane as developing solvent.

EXAMPLE 54

1-Methyl-3-[6-(N-ethyl-N-benzylamino)hexanoyl]indole (Compound No. 1-251)

A procedure similar to that described in Example 53 was repeated, but using 1-methyl-3-(6-bromohexanoyl)-indole and N-ethyl-N-benzylamine as starting materials, in relative proportions similar to those used in that Example, to obtain the title compound as an oil, with an Rf value of 0.30, using a 1 : 1 by volume mixture of ethyl acetate and hexane as developing solvent.

EXAMPLE 55

1-Methyl-3-[6-(2-tetrahydroisoquinolyl)hexanoyl]indole (Compound No. 1-261)

A procedure similar to that described in Example 53 was repeated, but using 1-methyl-3-(6-bromohexanoyl)-indole and tetrahydroisoquinoline as starting materials, in relative proportions similar to those used in that Example, to obtain the title compound as an oil, with an Rf value of 0.45, using a 19 : 1 by volume mixture of ethyl acetate and methanol as developing solvent.

EXAMPLE 56

1-Methyl-3-[6-(2-tetrahydroisoquinolyl)hexanoyl]indole-hydrochloride (Compound No. 1-262)

A procedure similar to that described in Example 3 was repeated, but using 1-methyl-3-[6-(2-tetrahydroisoquinolyl)hexanoyl]indole [prepared as described in Example 55] instead of 1-methyl-3-[3-(1-benzyl-4-piperidyl)propionyl]indole, in relative proportions similar to those used in that Example, to obtain the title compound as an amorphous solid, melting at 174-175°C.

EXAMPLE 57

1-Methyl-3-[6-(1-indolyl)hexanoyl]indole (Compound No. 1-265)

A procedure similar to that described in Example 53 was repeated, but using 1-methyl-3-(6-bromohexanoyl)-indole and indoline as starting materials, in relative proportions similar to those used in that Example, to obtain the title compound as an oil, with an Rf value of 0.40, with a 1 : 1 by volume mixture of ethyl acetate and hexane as developing solvent.

EXAMPLE 58

1-Methyl-3-[6-(1-indolyl)hexanoyl]indole hydrochloride (Compound No. 1-266)

A procedure similar to that described in Example 3 was repeated, but using 1-methyl-3-[6-(1-indolyl)-hexanoyl]indole [prepared as described in Example 57] instead of 1-methyl-3-[3-(1-benzyl-4-piperidyl)-propionyl]indole, in relative proportions similar to those used in that Example, to obtain the title compound as an amorphous solid, melting at 169-170°C.

EXAMPLE 59

1-Methyl-3-[6-(N-phenethylamino)hexanoyl]indole (Compound No. 1-267)

A procedure similar to that described in Example 53 was repeated, but using 1-methyl-3-(6-bromohexanoyl)-indole and N-phenethylamine as starting materials, in relative proportions similar to those used in that Example, to obtain the title compound as an oil, with an Rf value of 0.30, using a 9 : 1 by volume mixture of ethyl acetate and hexane as developing solvent.

EXAMPLE 60

1-Methyl-3-[6-(1-phenethylamino)hexanoyl]indole hydrochloride (Compound No. 1-268)

A procedure similar to that described in Example 3 was repeated, but using 1-methyl-3-[6-(1-phenethylamino)hexanoyl]indole [prepared as described in Example 59] instead of 1-methyl-3-[3-(1-benzyl-4-piperidyl)-propionyl]indole, in relative proportions similar to those used in that Example, to obtain the title compound as an amorphous solid, melting at 172-173°C.

EXAMPLE 61

1-Methyl-3-[6-(3,4-dimethoxyphenethyl)hexanoyl]indole (Compound No. 1-269)

A procedure similar to that described in Example 53 was repeated, but using 1-methyl-3-(6-bromohexanoyl)-indole and 3,4-dimethoxyphenethylamine as starting materials, in relative proportions similar to those used in that Example, to obtain the title compound as an oil, with an Rf value of 0.50, using a 9 : 1 by volume mixture of ethyl acetate and methanol as developing solvent.

EXAMPLE 62

1-Methyl-3-[6-(3,4-dimethoxyphenethyl)hexanoyl]indole hydrochloride (Compound No. 1-270)

A procedure similar to that described in Example 3 was repeated, but using 1-methyl-3-[6-(3,4-dimethoxyphenethyl)hexanoyl]indole [prepared as described in Example 61] instead of 1-methyl-3-[3-(1-benzyl-4-piperidyl)propionyl]indole, in relative proportions similar to those used in that Example, to obtain the title compound as an amorphous solid, melting at 226-227°C.

EXAMPLE 63

1-Methyl-3-[6-(2,4-dichlorobenzyl)hexanoyl]indole (Compound No. 1-295)

A procedure similar to that described in Example 53 was repeated, but using 1-methyl-3-(6-bromohexanoyl)indole and 2,4-dichlorobenzylamine as starting materials, in relative proportions similar to those used in that Example, to obtain the title compound as an oil, with an Rf value of 0.33, using a 9 : 1 by volume mixture of ethyl acetate and methanol as developing solvent.

EXAMPLE 64

1-Methyl-3-[6-(2,4-dichlorobenzyl)hexanoyl]indole hydrochloride (Compound No. 1-296)

A procedure similar to that described in Example 3 was repeated, but using 1-methyl-3-[6-(2,4-dichlorobenzyl)hexanoyl]indole [prepared as described in Example 63] instead of 1-methyl-3-[3-(1-benzyl-4-piperidyl)propionyl]indole, in relative proportions similar to those used in that Example, to obtain the title compound as an amorphous solid, melting at 182-183°C.

EXAMPLE 65

1-Methyl-3-[4-(N-methyl-N-benzylamino)phenylacetyl]indole (Compound No. 1-271)

65(1) 1-Methyl-3-(4-nitrophenylacetyl)indole

A procedure similar to that of step (1) of Example 1 was repeated, but using 4-nitrophenylacetyl chloride and 1-methylindole as starting materials, in relative proportions similar to those used in that Example, to obtain the title compound as an oil, with an Rf value of 0.50, using a 1 : 1 by volume mixture of ethyl acetate and hexane as developing solvent.

65(2) 1-Methyl-3-(4-aminophenylacetyl)indole

A procedure similar to that described in Example 2 was repeated, but using 1-methyl-3-(4-nitrophenylacetyl)indole [prepared as described in step (1)] instead of 1-methyl-3-[3-(1-benzyl-4-piperidyl)acryloyl]indole, in relative proportions similar to those used in that Example, to obtain the title compound as an oil, with an Rf value of 0.50, using ethyl acetate as developing solvent.

65(3) 1-Methyl-3-[4-(benzylamino)phenylacetyl]indole

1 g of benzaldehyde and 0.5 g of sodium borohydride were added to a solution of 1 g of 1-methyl-3-(4-aminophenylacetyl)indole [prepared as described in step (2)] in 20 ml of methanol, and the resulting mixture was stirred overnight at room temperature. The solvent was then removed from the reaction mixture by distillation under reduced pressure. After this, the residue was mixed with 5 ml of water, and the aqueous mixture was extracted with ethyl acetate. The extract was then dried over anhydrous sodium sulphate, and the solvent was removed by distillation under reduced pressure. The residue was purified by silica gel column chromatography, using a gradient elution method, with mixtures of ethyl acetate and hexane in proportions of from 1 : 9 to 10 : 0 by volume as the eluent, to give 0.7 g of the title compound as an oil, with an Rf value of 0.50, using a 1 : 1 by volume mixture of ethyl acetate and hexane as developing solvent.

65(4) 1-Methyl-3-[4-(N-methyl-N-benzylamino)phenylacetyl)indole

0.3 g of methyl iodide and 1 g of silver carbonate were added to a solution of 0.3 g of 1-methyl-3-[4-(benzylamino)phenylacetyl]indole [prepared as described in step (3)] in 5 ml of dimethylformamide, and the resulting mixture was stirred overnight at room temperature, after which it was filtered. The filtrate was mixed with 15 ml of water, and the aqueous mixture was extracted with ethyl acetate. The extract was then dried over anhydrous sodium sulphate, and the solvent was removed by distillation under reduced pressure. The residue was purified by silica gel column chromatography, using a gradient elution method, with mixtures of ethyl acetate and hexane in proportions of from 1 : 9 to 10 : 0 by volume as the eluent, to give 0.15 g of the title compound as an oil, with an Rf value of 0.48, using a 1 : 1 by volume mixture of ethyl acetate and hexane as developing solvent.

## EXAMPLE 66

1-Methyl-3-[4-(N-methyl-N-benzylamino)phenylacetyl]indole hydrochloride (Compound No. 1-272)

A procedure similar to that described in Example 3 was repeated, but using 1-methyl-3-[4-(N-methyl-N-benzylamino)phenylacetyl]indole [prepared as described in Example 65] instead of 1-methyl-3-[3-(1-benzyl-4-piperidyl)propionyl]indole, in relative proportions similar to those used in that Example, to obtain the title compound as an amorphous solid.

## EXAMPLE 67

1-Methyl-3-[3-(4-piperidyl)propionyl]indole hydrobromide (Compound No. 1-274)

67(1) 8-Benzyloxycarbonyl-1,4-dioxa-8-azaspiro[4.5]decane

A procedure similar to that described in step (1) of Example 17 was repeated, but using benzyl chloroformate and 1,4-dioxa-8-azaspiro[4.5]decane as starting materials, in relative proportions similar to those used in that Example, to obtain the title compound as an oil, with an Rf value of 0.59, using a 1 : 1 by volume mixture of ethyl acetate and hexane as developing solvent.

67(2) 1-Benzyloxycarbonyl-4-piperidinone

A procedure similar to that described in step (2) of Example 17 was repeated, but using 8-benzyloxycarbonyl-1,4-dioxa-8-azaspiro[4.5]decane [prepared as described in step (1)], instead of 8-(3-fluorobenzyl)-1,4-dioxa-8-azaspiro[4.5]decane, in relative proportions similar to those used in that Example, to obtain the title compound as an oil, with an Rf value of 0.51, using a 1 : 1 mixture of ethyl acetate and hexane as developing solvent.

67(3) 1-Benzyloxycarbonyl-4-methoxymethylenepiperidine

A procedure similar to that described in step (3) of Example 17 was repeated, but using 1-benzyloxycarbonyl-4-piperidinone [prepared as described in step (2)] instead of 1-(3-fluorobenzyl)-4-piperidinone, in relative proportions similar to those used in that Example, to obtain the title compound as an oil.

67(4) 1-Benzyloxycarbonyl-4-piperidinecarbaldehyde

A procedure similar to that described in step (4) of Example 17 was repeated, but using 1-benzyloxycarbonyl-4-methoxymethylenepiperidine [prepared as described in step (3)] instead of 1-(3-fluorobenzyl)-4-methoxymethylenepiperidine, in relative proportions similar to those used in that Example, to obtain the title compound as an oil.

67(5) 1-Methyl-3-[4-(1-benzyloxycarbonyl-4-piperidyl)-propionyl]indole

A procedure similar to that described in step (5) of Example 17 was repeated, but using 1-benzyloxycarbonyl-4-piperidinecarbaldehyde [prepared as described in step (4)] and 1-methylindole as starting materials, in relative proportions similar to those used in that Example, to obtain the title compound as an oil.

67(6) 1-Methyl-3-[4-(1-benzyloxycarbonyl-4-piperidyl)propionyl]indole

A procedure similar to that described in Example 2 was repeated, but using 1-methyl-3-[4-(1-benzyloxycarbonyl-4-piperidyl)propionyl]indole [prepared as described in step (5)] instead of 1-methyl-3-[3-(1-benzyl-4-piperidyl)acryloyl]indole, in relative proportions similar to those used in that Example, to obtain the title compound as an oil.

67(7) 1-Methyl-3-[3-(4-piperidyl)propionyl]indole

4 g of 1-methyl-3-[4-(1-benzyloxycarbonyl-4-piperidyl)propionyl]indole [prepared as described in step

(6)] were dissolved, under an atmosphere of nitrogen, in 5 ml of a 30% solution of hydrogen bromide in acetic acid, and the solution was stirred at room temperature for 30 minutes. At the end of this time, the solvent was removed by distillation under reduced pressure to obtain 3.4 g of the title compound as an oil, having an Rf value of 0.08, using a 9 : 1 by volume mixture of methylene chloride and methanol as developing solvent.

EXAMPLE 68

1-Methyl-3-[3-(1-benzyl-4-piperidyl)-2-methylacryloyl]indole (Compound No. 1-327)

68(1) 1-Methyl-3-propionylindole

A procedure similar to that described in step (1) of Example 1 was repeated, but using 1-methylindole and propionyl chloride as starting materials, in relative proportions similar to those used in that Example, to obtain the title compound as an oil.

68(2) 1-Methyl-3-[3-(1-benzyl-4-piperidyl)-2-methylacryloyl]indole

A procedure similar to that described in step (4) of Example 1 was repeated, but using 1-methyl-3-propionylindole [prepared as described in step (1)] and 1-benzyl-4-piperidinecarbaldehyde as starting materials, in relative proportions similar to those used in that Example, to obtain the title compound as an oil.

EXAMPLE 69

1-Methyl-3-[3-(1-benzyl-4-piperidyl)-2-methylpropionyl]indole (Compound No. 1-328)

A procedure similar to that described in Example 2 was repeated, but using 1-methyl-3-[3-(1-benzyl-4-piperidyl)-2-methylacryloyl]indole [prepared as described in Example 68] instead of 1-methyl-3-[3-(1-benzyl-4-piperidyl)acryloyl]indole in relative proportions similar to those used in that Example, to obtain the title compound as an oil, with an Rf value of 0.45, using a 9 : 1 by volume mixture of methylene chloride and methanol as developing solvent.

EXAMPLE 70

1-Methyl-3-[3-(1-benzyl-4-piperidyl)-1-hydroxypropyl]indole (Compound No. 1-275)

0.3 g of sodium borohydride was added to a solution of 1 g of 1-methyl-3-[3-(1-benzyl-4-piperidyl)propionyl]indole [prepared as described in Example 2] in 10 ml of methanol, at room temperature and whilst stirring. The resulting mixture was stirred for 3 hours, after which 3 ml of water were added to the mixture. The methanol was removed from the mixture by distillation under reduced pressure, after which the residue was extracted with ethyl acetate. The extract was then dried over anhydrous sodium sulphate, and the solvent was removed by distillation under reduced pressure. The residue was then purified by silica gel column chromatography, using a gradient elution method, with mixtures of ethyl acetate and methanol in proportions of from 1 : 9 to 10 : 0 by volume as the eluent, to give 0.71 g of the title compound, having an Rf value of 0.21, using a 9 : 1 by volume mixture of methylene chloride and methanol as developing solvent.

EXAMPLE 71

1-Methyl-3-[6-(N-ethyl-N-benzylamino)hexanoyl]indole-hydrochloride (Compound No. 1-286)

A procedure similar to that described in Example 3 was repeated, but using 1-methyl-3-[6-(N-ethyl-N-benzylamino)hexanoyl]indole [prepared as described in Example 54] instead of 1-methyl-3-[3-(1-benzyl-4-piperidyl)propionyl]indole, in relative proportions similar to those used in that Example, to obtain the title compound, melting at 125-126°C.

EXAMPLE 72

1-Methyl-3-{6-[N-methyl-N-phenethylamino]hexanoyl}indole (Compound No. 1-287)

A procedure similar to that described in Example 53 was repeated, but using 1-methyl-3-(6-bromohexanoyl)indole and N-methyl-N-phenethylamine as starting materials, in relative proportions similar to those used

in that Example, to obtain the title compound as an oil, with an Rf value of 0.21, using ethyl acetate as developing solvent.

EXAMPLE 73

1-Methyl-3-{6-[N-methyl-N-phenethylamino]hexanoyl}indole hydrochloride (Compound No. 1-288)

A procedure similar to that described in Example 3 was repeated, but using 1-methyl-3-{6-[N-methyl-N-phenethyl)amino]hexanoyl}indole [prepared as described in Example 72] instead of 1-methyl-3-[3-(1-benzyl-4-piperidyl)propionyl]indole, in relative proportions similar to those used in that Example, to obtain the title compound as an amorphous solid, with an Rf value of 0.23, using a 9 : 1 by volume mixture of ethyl acetate and methanol as developing solvent.

EXAMPLE 74

1-Methyl-3-[6-(N-adamantyl-N-methylamino)hexanoyl]indole (Compound No. 1-289)

A procedure similar to that described in Example 53 was repeated, but using 1-methyl-3-(6-bromohexanoyl)indole and N-adamantyl-N-methylamine as starting materials, in relative proportions similar to those used in that Example, to obtain the title compound as an oil, with an Rf value of 0.24, using ethyl acetate as developing solvent.

EXAMPLE 75

1-Methyl-3-[6-(N-adamantyl-N-methylamino)hexanoyl]indole hydrochloride (Compound No. 1-290)

A procedure similar to that described in Example 3 was repeated, but using 1-methyl-3-[6-(N-adamantyl-N-methylamino)hexanoyl]indole [prepared as described in Example 74] instead of 1-methyl-3-[3-(1-benzyl-4-piperidyl)propionyl]indole, in relative proportions similar to those used in that Example, to obtain the title compound, melting at 144-145°C.

EXAMPLE 76

1-Methyl-3-[6-(3,4-dimethoxybenzylamino)hexanoyl]indole (Compound No. 1-291)

A procedure similar to that described in Example 53 was repeated, but using 1-methyl-3-(6-bromohexanoyl)indole and 3,4-dimethoxybenzylamine as starting materials, in relative proportions similar to those used in that Example, to obtain the title compound as an oil, with an Rf value of 0.27, using ethyl acetate as developing solvent.

EXAMPLE 77

1-Methyl-3-[6-(3.4-dimethoxybenzylamino)hexanoyl]indole hydrochloride (Compound No. 1-292)

A procedure similar to that described in Example 3 was repeated, but using 1-methyl-3-[6-(3,4-dimethoxybenzylamino)hexanoyl]indole [prepared as described in Example 76] instead of 1-methyl-3-[3-(1-benzyl-4-piperidyl)propionyl]indole, in relative proportions similar to those used in that Example, to obtain the title compound, melting at 99-100°C.

EXAMPLE 78

1-Methyl-3-{6-[N-methyl-N-(3,4-dimethoxybenzyl)amino]hexanoyl}indole (Compound No. 1-293)

A procedure similar to that described in Example 53 was repeated, but using 1-methyl-3-(6-bromohexanoyl)indole and N-methyl-N-(3,4-dimethoxybenzyl)amine as starting materials, in relative proportions similar to those used in that Example, to obtain the title compound as an oil, with an Rf value of 0.30, using ethyl acetate as developing solvent.

EXAMPLE 79

1-Methyl-3-{6-[N-methyl-N-(3,4-dimethoxybenzyl)amino]hexanoyl}indole hydrochloride (Compound No. 1-294)

A procedure similar to that described in Example 3 was repeated, but using 1-methyl-3-{6-[N-methyl-N-(3,4-dimethoxybenzyl)amino]hexanoyl}indole [prepared as described in Example 78] instead of 1-methyl-3-[3-(1-benzyl-4-piperidyl)propionyl]indole, in relative proportions similar to those used in that Example, to obtain the title compound as an oil, with an Rf value of 0.20, using ethyl acetate as developing solvent.

EXAMPLE 80

1-Methyl-3-{6-[N-methyl-N-(2,4-dichlorobenzyl)amino]hexanoyl}indole (Compound No. 1-295)

A procedure similar to that described in Example 53 was repeated, but using 1-methyl-3-(6-bromohexanoyl)indole and N-methyl-N-(2,4-dichlorobenzyl)amine as starting materials, in relative proportions similar to those used in that Example, to obtain the title compound as an oil, with an Rf value of 0.28, using ethyl acetate as developing solvent.

EXAMPLE 81

1-Methyl-3-{6-[N-methyl-N-(2,4-dichlorobenzyl)amino]hexanoyl}indole hydrochloride (Compound No. 1-296)

A procedure similar to that described in Example 3 was repeated, but using 1-methyl-3-{6-[N-methyl-N-(2,4-dichlorobenzyl)amino]hexanoyl}indole [prepared as described in Example 80] instead of 1-methyl-3-[3-(1-benzyl-4-piperidyl)propionyl]indole, in relative proportions similar to those used in that Example, to obtain the title compound as an oil, with an Rf value of 0.22, using a 9 : 1 by volume mixture of ethyl acetate and methanol as developing solvent.

EXAMPLE 82

1-Methyl-3-[3-(1-benzyl-4-piperidyl)propionyl]-5-chloroindole hydrochloride (Compound No. 1-329)

A procedure similar to that described in Example 3 was repeated, but using 1-methyl-3-[3-(1-benzyl-4-piperidyl)propionyl]-5-chloroindole [prepared as described in Example 12] instead of 1-methyl-3-[3-(1-benzyl-4-piperidyl)propionyl]indole, in relative proportions similar to those used in that Example, to obtain the title compound, melting at 149-150°C.

EXAMPLE 83

1-Methyl-3-{3-[1-(3-trifluoromethylbenzyl)-4-piperidyl]acryloyl}indole (Compound No. 1-108)

A procedure similar to that described in Example 17 was repeated, but using 3-trifluoromethylbenzyl bromide instead of 3-fluorobenzyl bromide, in relative proportions similar to those used in that Example, to obtain the title compound as an oil, with an Rf value of 0.30, using ethyl acetate as developing solvent.

EXAMPLE 84

1-Methyl-3-{3-[1-(3-trifluoromethylbenzyl)-4-piperidyl]propionyl}indole (Compound No. 1-109)

A procedure similar to that described in Example 2 was repeated, but using 1-methyl-3-{3-[1-(3-trifluoromethylbenzyl)-4-piperidyl]acryloyl}indole [prepared as described in Example 83] instead of 1-methyl-3-[3-(1-benzyl-4-piperidyl)acryloyl]indole, in relative proportions similar to those used in that Example, to obtain the title compound as an oil, with an Rf value of 0.22, using a 9 : 1 by volume mixture of ethyl acetate and methanol as developing solvent.

EXAMPLE 85

1-Methyl-3-{3-[1-(3-trifluoromethylbenzyl)-4-piperidyl]propionyl}indole hydrochloride (Compound No. 1-110)

A procedure similar to that described in Example 3 was repeated, but using 1-methyl-3-{3-[1-(3-trifluoromethylbenzyl)-4-piperidyl]propionyl}indole [prepared as described in Example 84] instead of 1-methyl-3-[3-(1-benzyl-4-piperidyl)propionyl]indole, in relative proportions similar to those used in that Example, to obtain the title compound, melting at 202-204°C.

EXAMPLE 86

1-Methyl-3-{3-[1-(3-chlorobenzyl)-4-piperidyl]acryloyl}indole (Compound No. 1-83)

A procedure similar to that described in Example 17 was repeated, but using 3-chlorobenzyl bromide instead of 3-fluorobenzyl bromide, in relative proportions similar to those used in that Example, to obtain the title compound as an oil, with an Rf value of 0.30, using ethyl acetate as developing solvent.

EXAMPLE 87

1-Methyl-3-{3-[1-(3-chlorobenzyl)-4-piperidyl]propionyl}indole (Compound No. 1-84)

A procedure similar to that described in Example 2 was repeated, but using 1-methyl-3-{3-[1-(3-chlorobenzyl)-4-piperidyl]acryloyl}indole [prepared as described in Example 86] instead of 1-methyl-3-[3-(1-benzyl-4-piperidyl)acryloyl]indole, in relative proportions similar to those used in that Example, to obtain the title compound as an oil, with an Rf value of 0.14, using ethyl acetate as developing solvent.

EXAMPLE 88

1-Methyl-3-{3-[1-(3-chlorobenzyl)-4-piperidyl] propionyl}indole hydrochloride (Compound No. 1-85)

A procedure similar to that described in Example 3 was repeated, but using 1-methyl-3-{3-[1-(3-chlorobenzyl)-4-piperidyl]propionyl}indole [prepared as described in Example 87] instead of 1-methyl-3-[3-(1-benzyl-4-piperidyl)propionyl]indole, in relative proportions similar to those used in that Example, to obtain the title compound, melting at 128-129°C.

EXAMPLE 89

1-Methyl-3-{3-[1-(2-chlorobenzyl)-4-piperidyl] acryloyl}-5-chloroindole (Compound No. 1-300)

A procedure similar to that described in Example 17 was repeated, but using 2-chlorobenzyl bromide instead of 3-fluorobenzyl bromide, in relative proportions similar to those used in that Example, to obtain the title compound as an oil, with an Rf value of 0.21, using ethyl acetate as developing solvent.

EXAMPLE 90

1-Methyl-3-{3-[1-(2-chlorobenzyl)-4-piperidyl] propionyl}-5-chloroindole (Compound No. 1-299)

A procedure similar to that described in Example 2 was repeated, but using 1-methyl-3-{3-[1-(2-chlorobenzyl)-4-piperidyl]acryloyl}-5-chloroindole [prepared as described in Example 89] instead of 1-methyl-3-[3-(1-benzyl-4-piperidyl)acryloyl]indole, in relative proportions similar to those used in that Example, to obtain the title compound as an oil, with an Rf value of 0.23, using ethyl acetate as developing solvent.

EXAMPLE 91

1-Methyl-3-{3-[1-(2-chlorobenzyl)-4-piperidyl] propionyl}-5-chloroindole hydrochloride (Compound No. 1-301)

A procedure similar to that described in Example 3 was repeated, but using 1-methyl-3-{3-[1-(2-chlorobenzyl)-4-piperidyl]propionyl}-5-chloroindole [prepared as described in Example 90] instead of 1-methyl-3-[3-(1-benzyl-4-piperidyl)propionyl]indole, in relative proportions similar to those used in that Example, to obtain the title compound, melting at 164-165°C.

EXAMPLE 92

1-Methyl-3-{3-[1-(3-chlorobenzyl)-4-piperidyl]acryloyl}-5-chloroindole (Compound No. 1-303)

A procedure similar to that described in Example 17 was repeated, but using 3-chlorobenzyl bromide instead of 3-fluorobenzyl bromide, in relative proportions similar to those used in that Example, to obtain the title compound as an oil, with an Rf value of 0.24, using ethyl acetate as developing solvent.

EXAMPLE 93

1-Methyl-3-{3-[1-(3-chlorobenzyl)-4-piperidyl] propionyl}-5-chloroindole (Compound No. 1-302)

A procedure similar to that described in Example 2 was repeated, but using 1-methyl-3-{3-[1-(3-chlorobenzyl)-4-piperidyl]acryloyl}-5-chloroindole [prepared as described in Example 92] instead of 1-methyl-3-[3-(1-benzyl-4-piperidyl)acryloyl]indole, in relative proportions similar to those used in that Example, to obtain the title compound as an oil, with an Rf value of 0.25, using ethyl acetate as developing solvent.

EXAMPLE 94

1-Methyl-3-{3-[1-(3-chlorobenzyl)-4-piperidyl] propionyl}-5-chloroindole hydrochloride (Compound No. 1-304)

A procedure similar to that described in Example 3 was repeated, but using 1-methyl-3-{3-[1-(3-chlorobenzyl)-4-piperidyl]propionyl}-5-chloroindole [prepared as described in Example 93] instead of 1-methyl-3-[3-(1-benzyl-4-piperidyl)propionyl]indole, in relative proportions similar to those used in that Example, to obtain the title compound, melting at 164-165°C.

EXAMPLE 95

1-Methyl-3-{3-[1-(4-chlorobenzyl)-4-piperidyl]-acryloyl]-5-chloroindole (Compound No. 1-306)

A procedure similar to that described in Example 17 was repeated, but using 4-chlorobenzyl bromide instead of 3-fluorobenzyl bromide, in relative proportions similar to those used in that Example, to obtain the title compound as an oil, with an Rf value of 0.09, using ethyl acetate as developing solvent.

EXAMPLE 96

1-Methyl-3-{3-[1-(4-chlorobenzyl)-4-piperidyl] propionyl}-5-chloroindole (Compound No. 1-305)

A procedure similar to that described in Example 2 was repeated, but using 1-methyl-3-{3-[1-(4-chlorobenzyl)-4-piperidyl]acryloyl}-5-chloroindole [prepared as described in Example 95] instead of 1-methyl-3-[3-(1-benzyl-4-piperidyl)acryloyl]indole, in relative proportions similar to those used in that Example, to obtain the title compound as an oil, with an Rf value of 0.06, using ethyl acetate as developing solvent.

EXAMPLE 97

1-Methyl-3-{3-[1-(4-chlorobenzyl)-4-piperidyl] propionyl}-5-chloroindole hydrochloride (Compound No. 1-307)

A procedure similar to that described in Example 3 was repeated, but using 1-methyl-3-{3-[1-(4-chlorobenzyl)-4-piperidyl]propionyl}-5-chloroindole [prepared as described in Example 96] instead of 1-methyl-3-[3-(1-benzyl-4-piperidyl)propionyl]indole, in relative proportions similar to those used in that Example, to obtain the title compound, melting at 159-160°C.

EXAMPLE 98

1-Methyl-3-[3-(1-benzyl-4-piperidyl)acryloyl]-5-trifluoromethylindole (Compound No. 1-59)

A procedure similar to that described in Example 1 was repeated, but using 5-trifluoromethylindole instead of 1-methylindole, in relative proportions similar to those used in that Example, to obtain the title compound as an oil, with an Rf value of 0.22, using ethyl acetate as developing solvent.

EXAMPLE 99

1-Methyl-3-[3-(1-benzyl-4-piperidyl)propionyl]-5-trifluoromethylindole (Compound No. 1-60)

A procedure similar to that described in Example 2 was repeated, but using 1-methyl-3-[3-(1-benzyl-4-piperidyl)acryloyl]-5-trifluoromethylindole [prepared as described in Example 98] instead of 1-methyl-3-[3-(1-benzyl-4-piperidyl)acryloyl]indole, in relative proportions similar to those used in that Example, to obtain the title compound as an oil, with an Rf value of 0.14, using ethyl acetate as developing solvent.

EXAMPLE 100

1-Methyl-3-{3-[1-(3-fluorobenzyl)-4-piperidyl]acryloyl}-5-chloroindole (Compound No. 1-308)

A procedure similar to that described in Example 17 was repeated, but using 3-acetyl-1-methyl-5-chloroindole instead of 3-acetyl-1-methylindole in proportions similar to those used in that Example, to obtain the title compound as an oil, with an Rf value of 0.12, using ethyl acetate as developing solvent.

EXAMPLE 101

1-Methyl-3-{3-[1-(3-fluorobenzyl)-4-piperidyl] propionyl}-5-chloroindole (Compound No. 1-309)

A procedure similar to that described in Example 2 was repeated, but using 1-methyl-3-{3-[1-(3-fluorobenzyl)-4-piperidyl]acryloyl}-5-chloroindole [prepared as described in Example 100] instead of 1-methyl-3-[3-(1-benzyl-4-piperidyl)acryloyl]indole, in relative proportions similar to those used in that Example, to obtain the title compound as an oil, with an Rf value of 0.13, using ethyl acetate as developing solvent.

EXAMPLE 102

1-Methyl-3-{3-[1-(2-fluorobenzyl)-4-piperidyl]acryloyl}-5-fluoroindole (Compound No. 1-310)

A procedure similar to that described in Example 17 was repeated, but using 2-fluorobenzyl bromide instead of 3-fluorobenzyl bromide in proportions similar to those used in that Example, to obtain the title compound as an oil, with an Rf value of 0.14, using ethyl acetate as developing solvent.

EXAMPLE 103

1-Methyl-3-{3-[1-[2-fluorobenzyl)-4-piperidyl] propionyl}-5-fluoroindole (Compound No. 1-311)

A procedure similar to that described in Example 2 was repeated, but using 1-methyl-3-{3-[1-(2-fluorobenzyl)-4-piperidyl]acryloyl}-5-fluoroindole [prepared as described in Example 102] instead of 1-methyl-3-[3-(1-benzyl-4-piperidyl)acryloyl]indole, in relative proportions similar to those used in that Example, to obtain the title compound as an oil, with an Rf value of 0.59, using a 9 : 1 by volume mixture of methylene chloride and methanol as developing solvent.

EXAMPLE 104

1-Methyl-3-{3-[1-(3-fluorobenzyl)-4-piperidyl]acryloyl}-5-fluoroindole (Compound No. 1-312)

A procedure similar to that described in Example 17 was repeated, but using 3-acetyl-1-methyl-5-fluoroindole instead of 3-acetyl-1-methylindole in proportions similar to those used in that Example, to obtain the

title compound as an oil, with an Rf value of 0.30, using a 1 : 1 by volume mixture of ethyl acetate and hexane as developing solvent.

EXAMPLE 105

1-Methyl-3-{3-[1-(3-fluorobenzyl)-4-piperidyl] propionyl}-5-fluoroindole (Compound No. 1-313)

A procedure similar to that described in Example 2 was repeated, but using 1-methyl-3-{3-[1-(3-fluorobenzyl)-4-piperidyl]acryloyl}-5-fluoroindole [prepared as described in Example 104] instead of 1-methyl-3-[3-(1-benzyl-4-piperidyl)acryloyl]indole, in relative proportions similar to those used in that Example, to obtain the title compound, melting at 108-109°C.

EXAMPLE 106

1-Methyl-3-{3-[1-(3-fluorobenzyl)-4-piperidyl] propionyl}-5-fluoroindole hydrochloride (Compound No. 1-314)

A procedure similar to that described in Example 3 was repeated, but using 1-methyl-3-{3-[1-(3-fluorobenzyl)-4-piperidyl]propionyl}-5-fluoroindole [prepared as described in Example 105] instead of 1-methyl-3-[3-(1-benzyl-4-piperidyl)propionyl]indole, in relative proportions similar to those used in that Example to obtain the title compound, melting at 195-196°C.

EXAMPLE 107

1-Methyl-3-{3-[1-(4-fluorobenzyl)-4-piperidyl]acryloyl}-5-fluoroindole (Compound No. 1-317)

A procedure similar to that described in Example 17 was repeated, but using 4-fluorobenzyl bromide instead of 3-fluorobenzyl bromide in proportions similar to those used in that Example, to obtain the title compound as an oil, with an Rf value of 0.30, using ethyl acetate as developing solvent.

EXAMPLE 108

1-Methyl-3-{3-[1-(4-fluorobenzyl)-4-piperidyl] propionyl}-5-fluoroindole (Compound No. 1-318)

A procedure similar to that described in Example 2 was repeated, but using 1-methyl-3-{3-[1-(4-fluorobenzyl)-4-piperidyl]acryloyl}-5-fluoroindole [prepared as described in Example 107] instead of 1-methyl-3-[3-(1-benzyl-4-piperidyl)acryloyl]indole, in relative proportions similar to those used in that Example, to obtain the title compound as an oil, with an Rf value of 0.45, using a 9 : 1 by volume mixture of methylene chloride and methanol as developing solvent.

EXAMPLE 109

1-Methyl-3-{3-[1-(4-methoxybenzyl)-4-piperidyl] acryloyl}indole (Compound No. 1-104)

A procedure similar to that described in Example 17 was repeated, but using 4-methoxybenzyl bromide instead of 3-fluorobenzyl bromide in proportions similar to those used in that Example, to obtain the title compound as an oil, with an Rf value of 0.12, using ethyl acetate as developing solvent.

EXAMPLE 110

1-Methyl-3-{3-[1-(4-methoxybenzyl)-4-piperidyl]propionyl}indole (Compound No. 1-105)

A procedure similar to that described in Example 2 was repeated, but using 1-methyl-3-{3-[1-(4-methoxybenzyl)-4-piperidyl]acryloyl}indole [prepared as described in Example 109] instead of 1-methyl-3-[3-(1-benzyl-4-piperidyl)acryloyl]indole, in relative proportions similar to those used in that Example, to obtain the title compound as an oil, with an Rf value of 0.61, using a 10 : 1 by volume mixture of methylene chloride and methanol as developing solvent.

EXAMPLE 111

1-Methyl-3-{3-[1-(3,4-dimethoxybenzyl)-4-piperidyl] acryloyl}indole (Compound No. 1-330)

A procedure similar to that described in Example 17 was repeated, but using 3,4-dimethoxybenzyl bromide instead of 3-fluorobenzyl bromide in proportions similar to those used in that Example, to obtain the title compound as an oil, with an Rf value of 0.11, using ethyl acetate as developing solvent.

EXAMPLE 112

1-Methyl-3-{3-[1-(3,4-dimethoxybenzyl)-4-piperidyl] propionyl}indole (Compound No. 1-331)

A procedure similar to that described in Example 2 was repeated, but using 1-methyl-3-{3-[1-(3,4-dimethoxybenzyl)-4-piperidyl]acryloyl}indole [prepared as described in Example 111] instead of 1-methyl-3-[3-(1-benzyl-4-piperidyl)acryloyl]indole, in relative proportions similar to those used in that Example, to obtain the title compound as an oil, with an Rf value of 0.37, using a 10 : 1 by volume mixture of methylene chloride and methanol as developing solvent.

EXAMPLE 113

1-Methyl-3-{3-[1-(3-fluorophenethyl)-4-piperidyl] acryloyl}-5-fluoroindole (Compound No. 1-322)

A procedure similar to that described in Example 17 was repeated, but using 3-fluorophenethyl bromide instead of 3-fluorobenzyl bromide in proportions similar to those used in that Example, to obtain the title compound as an oil, with an Rf value of 0.13, using ethyl acetate as developing solvent.

EXAMPLE 114

1-Methyl-3-{3-[1-(3-fluorophenylethyl)-4-piperidyl] propionyl}-5-fluoroindole (Compound No. 1-323)

A procedure similar to that described in Example 2 was repeated, but using 1-methyl-3-{3-[1-(3-fluorophenylethyl)-4-piperidyl]acryloyl}-5-fluoroindole [prepared as described in Example 113] instead of 1-methyl-3-[3-(1-benzyl-4-piperidyl)acryloyl]indole, in relative proportions similar to those used in that Example, to obtain the title compound as an amorphous solid, with an Rf value of 0.44, using a 9 : 1 by volume mixture of methylene chloride and methanol as developing solvent.

EXAMPLE 115

1-Methyl-3-[3-(1-phenethyl-4-piperidyl)propionyl]indole hydrochloride (Compound No. 1-324)

A procedure similar to that described in Example 3 was repeated, but using 1-methyl-3-[3-(1-phenethyl-4-piperidyl)propionyl]indole [prepared as described in Example 36] instead of 1-methyl-3-[3-(1-benzyl-4-piperidyl)propionyl]indole, in relative proportions similar to those used in that Example, to obtain the title compound, melting at 207-208°C.

EXAMPLE 116

1-Methyl-3-[3-(1-benzyl-4-piperidyl)acryloyl]indazole (Compound No. 2-1)

A procedure similar to that described in step (4) of Example 1 was repeated, but using 3-acetyl-1-methylindazole [prepared as described in step (c) of Preparation 1] instead of 1-methyl-3-acetylindole, in relative proportions similar to those used in that Example, to give 3.1 g of the title compound as an oil, with an Rf value of 0.65, using ethyl acetate as developing solvent.

EXAMPLE 117

1-Methyl-3-[3-(1-benzyl-4-piperidyl)propionyl]indazole (Compound No. 2-2)

A procedure similar to that described in Example 2 was repeated, but using 1-methyl-3-[3-(1-benzyl-4-piperidyl)acryloyl]indazole [prepared as described in Example 116] instead of 1-methyl-3-[3-(1-benzyl-4-piperidyl)acryloyl]indole, in relative proportions similar to those used in that Example, to give 0.9 g of the title compound as an oil, with an Rf value of 0.40, using ethyl acetate as developing solvent.

EXAMPLE 118

1-Methyl-3-[3-(1-benzyl-4-piperidyl)propionyl]indazole hydrochloride (Compound No. 2-3)

A procedure similar to that described in Example 3 was repeated, but using 1-methyl-3-[3-(1-benzyl-4-piperidyl)propionyl]indazole [prepared as described in Example 117] instead of 1-methyl-3-[3-(1-benzyl-4-piperidyl)propionyl]indole, in relative proportions similar to those used in that Example, to obtain 0.21 g of the title compound.

EXAMPLE 119

1-Methyl-3-{3-[1-(2-chlorobenzyl)-4-piperidyl]-acryloyl}indazole (Compound No. 2-94)

A procedure similar to that described in Example 17 was repeated, but using 2-chlorobenzyl bromide instead of 3-fluorobenzyl bromide in step (1) of that Example, and 1-methyl-3-acetylindazole [prepared as described in Preparation 3] instead of 1-methyl-3-acetylindole in step (5) of that Example, in relative proportions similar to those used in that Example, to obtain the title compound as an oil, with an Rf value of 0.3, using ethyl acetate as developing solvent.

EXAMPLE 120

1-Methyl-3-{3-[1-(2-chlorobenzyl)-4-piperidyl]propionyl}indazole (Compound No. 2-95)

A procedure similar to that described in Example 2 was repeated, but using 1-methyl-3-{3-[1-(2-chlorobenzyl)-4-piperidyl]acryloyl}indazole [prepared as described in Example 119] instead of 1-methyl-3-[3-(1-benzyl-4-piperidyl)acryloyl]indole, in relative proportions similar to those used in that Example, to give 0.9 g of the title compound as an oil, with an Rf value of 0.65, using a 1 : 1 by volume mixture of ethyl acetate and hexane as developing solvent.

EXAMPLE 121

1-Methyl-3-{3-[1-(2-chlorobenzyl)-4-piperidyl]propionyl}indazole hydrochloride (Compound No. 2-96)

A procedure similar to that described in Example 3 was repeated, but using 1-methyl-3-{3-[1-(2-chlorobenzyl)-4-piperidyl]propionyl}indazole [prepared as described in Example 120] instead of 1-methyl-3-[3-(1-benzyl-4-piperidyl)propionyl]indole, in relative proportions similar to those used in that Example, to obtain the title compound, melting at 207-208°C.

EXAMPLE 122

1-Methyl-3-{3-[1-(3-chlorobenzyl)-4-piperidyl]acryloyl}indazole (Compound No. 2-97)

A procedure similar to that described in Example 17 was repeated, but using 3-chlorobenzyl bromide instead of 3-fluorobenzyl bromide in step (1) of that Example, and 1-methyl-3-acetylindazole [prepared as described in Preparation 3] instead of 1-methyl-3-acetylindole in step (5) of that Example, in relative proportions similar to those used in that Example, to obtain the title compound as an oil, with an Rf value of 0.32, using ethyl acetate as developing solvent.

EXAMPLE 123

1-Methyl-3-{3-[1-(3-chlorobenzyl)-4-piperidyl]propionyl}indazole (Compound No. 2-98)

A procedure similar to that described in Example 2 was repeated, but using 1-methyl-3-{3-[1-(3-chlorobenzyl)-4-piperidyl]acryloyl}indazole [prepared as described in Example 122] instead of 1-methyl-3-[3-(1-benzyl-4-piperidyl)acryloyl]indole, in relative proportions similar to those used in that Example, to obtain the title compound as an oil, with an Rf value of 0.40, using a 1 : 1 by volume mixture of ethyl acetate and hexane as developing solvent.

EXAMPLE 124

1-Methyl-3-{3-[1-(3-chlorobenzyl)-4-piperidyl]-propionyl}indazole hydrochloride (Compound No. 2-99)

A procedure similar to that described in Example 3 was repeated, but using 1-methyl-3-{3-[1-(3-chlorobenzyl)-4-piperidyl]propionyl}indazole [prepared as described in Example 123] instead of 1-methyl-3-[3-(1-benzyl-4-piperidyl)propionyl]indole, in relative proportions similar to those used in that Example, to obtain the title compound, melting at 186-188°C.

EXAMPLE 125

1-Methyl-3-{3-[1-(4-chlorobenzyl)-4-piperidyl]acryloyl}indazole (Compound No. 2-100)

A procedure similar to that described in Example 17 was repeated, but using 4-chlorobenzyl bromide instead of 3-fluorobenzyl bromide in step (1) of that Example, and 1-methyl-3-acetylindazole [prepared as described in Preparation 3] instead of 1-methyl-3-acetylindole in step (5) of that Example, in relative proportions similar to those used in that Example, to obtain the title compound as an oil, with an Rf value of 0.32, using ethyl acetate as developing solvent.

EXAMPLE 126

1-Methyl-3-{3-[1-(4-chlorobenzyl)-4-piperidyl]propionyl}indazole (Compound No. 2-101)

A procedure similar to that described in Example 2 was repeated, but using 1-methyl-3-{3-[1-(4-chlorobenzyl)-4-piperidyl]acryloyl}indazole [prepared as described in Example 125] instead of 1-methyl-3-[3-(1-benzyl-4-piperidyl)acryloyl]indole, in relative proportions similar to those used in that Example, to obtain the title compound as an oil, with an Rf value of 0.40, using a 1 : 1 by volume mixture of ethyl acetate and hexane as developing solvent.

EXAMPLE 127

1-Methyl-3-{3-[1-(4-chorobenzyl)-4-piperidyl]propionyl}indazole hydrochloride (Compound No. 2-102)

A procedure similar to that described in Example 3 was repeated, but using 1-methyl-3-{3-[1-(4-chlorobenzyl)-4-piperidyl]propionyl}indazole [prepared as described in Example 126] instead of 1-methyl-3-[3-(1-benzyl-4-piperidyl)propionyl]indole, in relative proportions similar to those used in that Example, to obtain the title compound, melting at 220-222°C.

EXAMPLE 128

1-Methyl-3-{3-[1-(2-methoxybenzyl)-4-piperidyl]acryloyl}indazole (Compound No. 2-164)

A procedure similar to that described in Example 17 was repeated, but using 2-methoxybenzyl chloride instead of 3-fluorobenzyl bromide in step (1) of that Example, and 1-methyl-3-acetylindazole [prepared as described in Preparation 3] instead of 1-methyl-3-acetylindole in step (5) of that Example, in relative proportions similar to those used in that Example, to obtain the title compound as an oil, with an Rf value of 0.32, using ethyl acetate as developing solvent.

EXAMPLE 129

1-Methyl-3-{3-[1-(2-methoxybenzyl)-4-piperidyl]propionyl}indazole (Compound No. 2-165)

A procedure similar to that described in Example 2 was repeated, but using 1-methyl-3-{3-[1-(2-methoxybenzyl)-4-piperidyl]acryloyl}indazole [prepared as described in Example 128] instead of 1-methyl-3-[3-(1-benzyl-4-piperidyl)acryloyl]indole, in relative proportions similar to those used in that Example, to obtain the title compound as an oil, with an Rf value of 0.30, using ethyl acetate as developing solvent.

EXAMPLE 130

1-Methyl-3-{3-[1-(2-methoxybenzyl)-4-piperidyl]propionyl}indazole hydrochloride (Compound No. 2-166)

A procedure similar to that described in Example 3 was repeated, but using 1-methyl-3-{3-[1-(2-methoxybenzyl)-4-piperidyl]propionyl}indazole [prepared as described in Example 129] instead of 1-methyl-3-[3-(1-benzyl-4-piperidyl)propionyl]indole, in relative proportions similar to those used in that Example, to obtain the title compound as an oil.

EXAMPLE 131

1-Methyl-3-{3-[1-(3-methoxybenzyl)-4-piperidyl]acryloyl}indazole (Compound No. 2-167)

A procedure similar to that described in Example 17 was repeated, but using 3-methoxybenzyl chloride instead of 3-fluorobenzyl bromide in step (1) of that Example, and 1-methyl-3-acetylindazole [prepared as described in Preparation 3] instead of 1-methyl-3-acetylindole in step (5) of that Example, in relative proportions similar to those used in that Example, to obtain the title compound as an oil, with an Rf value of 0.32, using ethyl acetate as developing solvent.

EXAMPLE 132

1-Methyl-3-{3-[1-(3-methoxybenzyl)-4-piperidyl]propionyl}indazole (Compound No. 2-168)

A procedure similar to that described in Example 2 was repeated, but using 1-methyl-3-{3-[1-(3-methoxybenzyl)-4-piperidyl]acryloyl}indazole [prepared as described in Example 131] instead of 1-methyl-3-[3-(1-benzyl-4-piperidyl)acryloyl]indole, in relative proportions similar to those used in that Example, to obtain the title compound as an oil, with an Rf value of 0.48, using a 1 : 1 by volume mixture of ethyl acetate and hexane as developing solvent.

EXAMPLE 133

1-Methyl-3-{3-[1-(3-methoxybenzyl)-4-piperidyl]propionyl}indazole hydrochloride (Compound No. 2-169)

A procedure similar to that described in Example 3 was repeated, but using 1-methyl-3-{3-[1-(3-methoxybenzyl)-4-piperidyl]propionyl}indazole [prepared as described in Example 132] instead of 1-methyl-3-[3-(1-benzyl-4-piperidyl)propionyl]indole, in relative proportions similar to those used in that Example, to obtain the title compound, melting at 125-127°C.

EXAMPLE 134

1-Methyl-3-{3-[1-(4-methoxybenzyl)-4-piperidyl]acryloyl}indazole (Compound No. 2-170)

A procedure similar to that described in Example 17 was repeated, but using 4-methoxybenzyl chloride instead of 3-fluorobenzyl bromide in step (1) of that Example, and 1-methyl-3-acetylindazole [prepared as described in Preparation 3] instead of 1-methyl-3-acetylindole in step (5) of that Example, in relative proportions similar to those used in that Example, to obtain the title compound as an oil, with an Rf value of 0.43, using a 1 : 1 by volume mixture of ethyl acetate and hexane as developing solvent.

EXAMPLE 135

1-Methyl-3-{3-[1-(4-methoxybenzyl)-4-piperidyl]propionyl}indazole (Compound No. 2-171)

A procedure similar to that described in Example 2 was repeated, but using 1-methyl-3-{3-[1-(4-methoxybenzyl)-4-piperidyl]acryloyl}indazole [prepared as described in Example 134] instead of 1-methyl-3-[3-(1-benzyl-4-piperidyl)acryloyl]indole, in relative proportions similar to those used in that Example, to obtain the title compound, melting at 212-214°C.

EXAMPLE 136

1-Methyl-3-{3-[1-(4-methoxybenzyl)-4-piperidyl]propionyl}indazole hydrochloride (Compound No. 2-172)

A procedure similar to that described in Example 3 was repeated, but using 1-methyl-3-{3-[1-(4-methoxybenzyl)-4-piperidyl]propionyl}indazole [prepared as described in Example 135] instead of 1-methyl-3-[3-(1-benzyl-4-piperidyl)propionyl]indole, in relative proportions similar to those used in that Example, to obtain the title compound.

EXAMPLE 137

1-Methyl-3-{3-[1-(3-fluorobenzyl)-4-piperidyl]acryloyl}indazole (Compound No. 2-71)

A procedure similar to that described in Example 17 was repeated, but using 3-fluorobenzyl chloride instead of 3-fluorobenzyl bromide in step (1) of that Example, and 1-methyl-3-acetylindazole [prepared as described in Preparation 3] instead of 1-methyl-3-acetylindole in step (5) of that Example, in relative proportions similar to those used in that Example, to obtain the title compound as an oil, with an Rf value of 0.32, using ethyl acetate as developing solvent.

EXAMPLE 138

1-Methyl-3-{3-[1-(3-fluorobenzyl)-4-piperidyl]propioyl}indazole (Compound No. 2-72)

A procedure similar to that described in Example 2 was repeated, but using 1-methyl-3-{3-[1-(3-fluorobenzyl)-4-piperidyl]acryloyl}indazole [prepared as described in Example 137] instead of 1-methyl-3-[3-(1-benzyl-4-piperidyl)acryloyl]indole, in relative proportions similar to those used in that Example, to obtain the title compound as an oil, with an Rf value of 0.30, using ethyl acetate as developing solvent.

EXAMPLE 139

1-Methyl-3-{3-[1-(3-fluorobenzyl)-4-piperidyl]propionyl}indazole hydrochloride (Compound No. 2-283)

A procedure similar to that described in Example 3 was repeated, but using 1-methyl-3-{3-[1-(3-fluorobenzyl)-4-piperidyl]propionyl}indazole [prepared as described in Example 138] instead of 1-methyl-3-[3-(1-benzyl-4-piperidyl)propionyl]indole, in relative proportions similar to those used in that Example, to obtain the title compound.

EXAMPLE 140

1-Methyl-3-[3-(1-phenethyl-4-piperidyl)acryloyl]indazole (Compound No. 2-224)

A procedure similar to that described in Example 17 was repeated, but using phenethyl chloride instead of 3-fluorobenzyl bromide in step (1) of that Example, and 1-methyl-3-acetylindazole [prepared as described in Preparation 1] instead of 1-methyl-3-acetylindole in step (5) of that Example, in relative proportions similar to those used in that Example, to obtain the title compound as an oil, with an Rf value of 0.32, using ethyl acetate as developing solvent.

EXAMPLE 141

1-Methyl-3-[3-(1-phenethyl-4-piperidyl)propionyl]indazole (Compound No. 2-225)

A procedure similar to that described in Example 2 was repeated, but using 1-methyl-3-[3-(1-phenethyl-4-piperidyl)acryloyl]indazole [prepared as described in Example 140] instead of 1-methyl-3-[3-(1-benzyl-4-piperidyl)acryloyl]indole, in relative proportions similar to those used in that Example, to obtain the title compound as an oil, with an Rf value of 0.33, using ethyl acetate as developing solvent.

EXAMPLE 142

1-Methyl-3-[3-(1-phenethyl-4-piperidyl)propionyl]indazole hydrochloride (Compound No. 2-226)

A procedure similar to that described in Example 3 was repeated, but using 1-methyl-3-[3-(3-phenethyl-4-piperidyl)propionyl]indazole [prepared as described in Example 141] instead of 1-methyl-3-[3-(1-benzyl-4-piperidyl)propionyl]indole, in relative proportions similar to those used in that Example, to obtain the title compound, melting at 215°C (with decomposition).

EXAMPLE 143

1-Methyl-5-[3-(1-benzyl-4-piperidyl)acryloyl]indole (Compound No. 1-362)

A procedure similar to that described in Example 1 was repeated, but using 1-methyl-5-acetylindole instead of 1-methyl-3-acetylindole, in relative proportions similar to those used in that Example, to obtain the title compound as an oil, with an Rf value of 0.15, using ethyl acetate as developing solvent.

EXAMPLE 144

1-Methyl-5-[3-(1-benzyl-4-piperidyl)propionyl]indole (Compound No. 1-363)

A procedure similar to that described in Example 2 was repeated, but using 1-methyl-5-[3-(1-benzyl-4-piperidyl)acryloyl]indole [prepared as described in Example 143] instead of 1-methyl-3-[3-(1-benzyl-4-piperidyl)acryloyl]indole, in relative proportions similar to those used in that Example, to obtain the title compound as an amorphous substance, with an Rf value of 0.46, using a 9 : 1 by volume mixture of methylene chloride and methanol as developing solvent.

EXAMPLE 145

1-Methyl-5-[N-methyl-N-benzyloxycarbonylaminomethyl]-3-[3-(1-benzyl-4-piperidyl)acryloyl]indole (Compound No. 1-364)

A procedure similar to that described in Example 1 was repeated, but using 1-methyl-5-[N-methyl-N-benzyloxycarbonylaminomethyl]-3-acetylindole [prepared as described in Preparation 2] instead of 1-methyl-3-acetylindole, in relative proportions similar to those used in that Example, to obtain the title compound as an oil, with an Rf value of 0.09, using ethyl acetate as developing solvent.

EXAMPLE 146

1-Methyl-5-(methylaminomethyl)-3-[3-(1-benzyl-4-piperidyl)propionyl]indole (Compound No. 1-365)

A procedure similar to that described in Example 2 was repeated, but using 1-methyl-5-[N-methyl-N-benzyloxycarbonylaminomethyl-3-[3-(1-benzyl-4-piperidyl)acryloyl]indole [prepared as described in Example 145] instead of 1-methyl-3-[3-(1-benzyl-4-piperidyl)-acryloyl]indole, in relative proportions similar to those used in that Example, to obtain the title compound as an amorphous substance, with an Rf value of 0.18, using a 9 : 1 by volume mixture of methylene chloride and methanol as developing solvent.

EXAMPLE 147

1-Methy-5-[N-methyl-N-propargylaminomethyl]-3-[3-(1-benzyl-4-piperidyl)propionyl]indole (Compound No. 1-366)

1.0 g of t-butylamine were added to a solution of 1.0 g of 1-methyl-5-[methylaminomethyl]-3-[3-(1-benzyl-4-piperidyl)propionyl]indole [prepared as described in Example 146] in 10 ml of tetrahydrofuran. 0.4 g of propargylamine was added to the resulting mixture, whilst ice-cooling, and the mixture was stirred for 2 hours at room temperature. The solvent was then removed by distillation under reduced pressure, water was added to the residue and the mixture was extracted with ethyl acetate. The ethyl acetate extract was then dried over anhydrous sodium sulphate, the solvent was removed by distillation under reduced pressure, and the residue was purified by alumina column chromatography using a gradient elution method, with mixtures of ethyl acetate and hexane in proportions of from 1 : 9 to 10 : 0 by volume as the eluent, to give 0.61 g of the title compound as an amorphous substance, with an Rf value of 0.22, using a 9 : 1 by volume mixture of methylene chloride and methanol as developing solvent.

PREPARATION 1

1(a) Indazole-3-carboxylic acid

11.3 g of caustic soda and then 40 g of isatine were dissolved in 180 ml of water. The resulting solution was cooled to 0°C, after which 70 ml of a solution of 18.8 g of sodium nitrite in water was added to the mixture. A solution of 50 g of concentrated sulphuric acid in 520 ml of water was then rapidly added dropwise to the reaction mixture, and the mixture was stirred for 15 minutes. At the end of this time, a solution of 123 g of stannous chloride in 230 ml of concentrated hydrochloric acid was added to the mixture. The mixture was then stirred for 1 hour, heated gradually to room temperature, and then stirred for a further 1 hour. The solution was then filtered, and the precipitate collected. This precipitate was dissolved in water, and, after removal of insoluble matter, the precipitate was recrystalised from the water, to give 15.7 g of the title compound, melting at 265-266°C.

1(b) 1-Methyl-indazole-3-carboxylic acid

A solution of 15.7 g of indazole-3-carboxylic acid [prepared as described in step (a)] in 200 ml of dimethylsulphoxide was added to a suspension of 11 g of sodium hydrogencarbonate (a 55% dispersion in mineral oil) in 70 ml of dimethylsulphoxide. The resulting mixture was stirred at room temperature for 1 hour. 16.6 g of methyl iodide were then added to the mixture, and the mixture was stirred for a further 2 hours at room temperature. At the end of this time, the reaction mixture was poured into ice water, and then made acidic by the addition of a 1 N aqueous solution of hydrochloric acid. The mixture was then extracted with ethyl acetate and, after the ethyl acetate layer had been washed with a saturated aqueous solution of sodium chloride, it was dried over anhydrous sodium sulphate. The solvent was then removed by distillation under reduced pressure, and the residue was recrystallised from ethanol to give 11.9 g of the title compound, melting at 212-213°C.

1(c) 3-Acetyl-1-methylindazole

11.9 g of 1-methylindazole-3-carboxylic acid [prepared as described in step (b)] were dissolved in 200 ml of tetrahydrofuran, and 45 ml of 1.5 M methyl iodide were added to the resulting solution. The mixture was then gradually heated to 0°C and, after the addition of a further 45 ml of 1.5 M methyl iodide, the resulting mixture was heated to room temperature. After the mixture had been stirred for 1 hour, 150 ml of a 1 N aqueous solution of hydrochloric acid were added to the solution to make it acidic, and the organic layer was then removed. The aqueous layer was extracted with diethyl ether, and the diethyl ether layer was then combined with the organic layer removed in the previous step. The resulting mixture was washed with a saturated aqueous solution of sodium chloride, and then dried over anhydrous sodium sulphate. The solvent was removed by distillation under reduced pressure, and the residue was purified over silica gel column chromatography using a gradient elution method with mixtures of ethyl acetate and hexane of from 1 : 9 to 10 : 0 by volume as the eluent, to give 8.8 g of the title compound, with an Rf value of 0.55, using a 1 : 1 by volume mixture of ethyl acetate and hexane as developing solvent.

PREPARATION 2

2(a) (N-Methyl)indole-4-carboxamide

A mixture of 6.8 g of N-methylamine hydrochloride and 10 g of triethylamine in 40 ml of dimethylformamide was stirred at room temperature for 30 minutes. 8.0 g of indole-5-carboxylic acid and 11 g of dicylohexylcarbodiimide were added to the mixture, whilst ice-cooling, and the resulting mixture was stirred for 3 hours, at room temperature. At the end of this time, the solvent was removed by distillation under reduced pressure, and the residue was purified by silica gel column chromatography, using a gradient elution method with mixtures of ethyl acetate and hexane in proportions of from 1 : 9 to 10 : 0 by volume as the eluent, to give 5.2 g of the title compound. This compound was used in the next step without further purification.

2(b) 5-(N-methylaminomethyl)indole

5.2 g of (N-methyl)indole-4-carboxamide [prepared as described in step (a)] and 1 g of lithium aluminium hydride were added to 50 ml of tetrahydrofuan, and the resulting mixture was heated under reflux for 2 hours. At the end of this time, 0.5 ml of a 4 N aqueous solution of sodium hydroxide was added to the solution, whilst ice-cooling, and the mixture was stirred for one hour. The reaction mixture was then filtered over Celite (a trade mark). The filtrate was then concentrated by evaporation under reduced pressure, and the concentrate was used in the next step without further purification.

2(c) 5-(N-methyl-N-benzyloxycarbonylmethylaminomethyl)indole

3.0 g of triethylamine were added to a solution of 5-(N-methylaminomethyl)indole [prepared as described in step (b)] in 20 ml of methylene chloride, after which 5.5 g of benzyloxychloride were added, whilst ice-cooling. The mixture was then stirred for 2 hours at room temperature, after which the mixture was diluted with water and then extracted with ethyl acetate. The extract was then dried over anhydrous sodium sulphate, and the solvent was removed by distillation under reduced pressure. The residue was purified by silica gel column chromatography, using a gradient elution method with mixtures of ethyl acetate and hexane in proportions of from 1 : 9 to 10 : 1 by volume as the eluent, to give 5.1 g of the title compound, as an amorphous solid, with an Rf value of 0.81, using ethyl acetate as developing solvent.

2(d) 1-Acetyl-5-(N-methyl-N-benzyloxycarbonylaminomethyl)indole

1.5 g of sodium hydride (as a 55% suspension in mineral oil) were added to a solution of 5.0 g of 5-(N-methyl-N-benzyloxycarbonylaminomethyl)indole [prepared as described in step (c)] in 30 ml of dimethylformamide, whilst ice-cooling. The mixture was then stirred for 30 minutes, after which 1.0 g of acetyl chloride was added. The resulting mixture was then stirred at room temperature for a further one hour, after which iced water was added. The mixture was then extracted with ethyl acetate, the extract was dried over anhydrous sodium sulphate, and the solvent was removed by distillation under reduced pressure. The resulting residue was purified by silica gel column chromatography, using a gradient elution method with mixtures of ethyl acetate and hexane in proportions of from 1 : 9 to 10 : 0 by volume as the eluent, to give 3.1 g of the title compound as an oil, with an Rf value of 0.52, using ethyl acetate as developing solvent.

2(e) 3-Acetyl-5-(N-methyl-N-benzyloxycarbonylaminomethyl)indole

A solution of 3.0 g of 1-acetyl-5-(N-methyl-N-benzyloxycarbonylaminomethyl)indole [prepared as described in step (d)] in 50 ml of methanol was irradiated for 3 hours under a medium pressure mercury lamp, whilst ice-cooling. At the end of this time, the solvent was removed by distillation under reduced pressure and the residue was purified by silica gel column chromatography, using a gradient elution method with mixtures of ethyl acetate and hexane in proportions of from 1 : 9 to 10 : 0 by volume as the eluent, to give 1.1 g of the title compound as an oil, with an Rf value of 0.41, using ethyl acetate as developing solvent.

2(f) 1-Methyl-5-(N-methyl-N-benzyloxycarbonylaminomethyl)-3-acetylindole

0.15 g of sodium hydride (as a 55% suspension in mineral oil) was added to a solution of 1.0 g of 3-acetyl-5-(N-methyl-N-benzyloxycarbonylaminomethyl)indole [prepared as described in step (e)] in 10 ml of dimethylformamide, whilst ice-cooling and stirring. The mixture was then stirred for 30 minutes, after which 0.45 g of methyl iodide was added to the mixture. The resulting mixture was then stirred for a further one hour, at room temperature, and then iced water was added and the mixture was extracted with ethyl acetate. The extract was dried over anhydrous sodium sulphate, and the solvent was removed by distillation under reduced pressure. The residue was then purified by silica gel column chromatography, using a gradient elution method with mixtures of ethyl acetate and hexane in proportions of from 1 : 9 to 10 : 0 by volume as the eluent, to give 0.56 g of the title compound as an oil, with an Rf value of 0.51, using ethyl acetate as developing solvent.

## Claims

1. Compounds of formula (I):

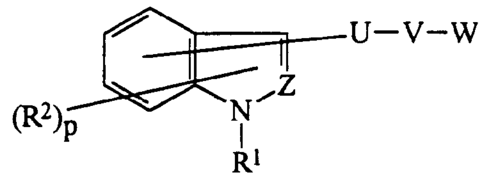

(I)

in which:

Z represents a group of formula -N= or -CH= or a group of formula -CH= in which the hydrogen atom is replaced by the group -U-V-W shown in formula (I);

p is 0, 1, 2 or 3;

$R^1$ represents a hydrogen atom or any of the groups or atoms represented by $R^2$;

$R^2$ represents:

a hydrogen atom;

any of the groups and atoms represented by $R^a$;

an aryl group, as defined below;

an aralkyl group, as defined below;

aralkyloxycarbonyl group, in which the aralkyl part is as defined below;

an arylamino group, in which the aryl part is as defined below;

an arylaminoalkyl group, in which the aryl part is as defined below, and the alkyl part has from 1 to 6 carbon atoms;

a heterocyclic group having from 3 to 8 ring atoms of which from 1 to 4 are nitrogen and/or oxygen and/or sulphur hetero-atoms, said group being unsubstituted or being substituted by at least one substituent selected from the groups and atoms represented by $R^a$;

an alkyl group which has from 1 to 6 carbon atoms and which is substituted by a single heterocyclic group, said heterocyclic group having from 3 to 8 ring atoms of which from 1 to 4 are nitrogen and/or oxygen and/or sulphur hetero-atoms, and being unsubstituted or being substituted by at least one substituent selected from the groups and atoms represented by $R^a$; or

an alkylamino group which has from 1 to 6 carbon atoms and which is substituted by a single heterocyclic group, said heterocyclic group having from 3 to 8 ring atoms of which from 1 to 4 are nitrogen and/or oxygen and/or sulphur hetero-atoms, and being unsubstituted or being substituted by at least one substituent selected from the groups and atoms represented by $R^a$;

U represents a group of formula -CO- or -CH($OR^3$)-, where $R^3$ represents a hydrogen atom or a hydroxy-protecting group;

V represents a group of formula

$$-(CR^e{=}CR^f)_m-(CR^gR^h)_n-$$

where m is 0, 1 or 2, n is 0 or an integer from 1 to 7, provided that (m + n) > 0, and $R^e$, $R^f$, $R^g$ and $R^h$ are the same or different and each represents a hydrogen atom or an alkyl group having from 1 to 4 carbon atoms;

W represents:

a heterocyclic group having from 3 to 14 ring atoms, of which one is a nitrogen atom through which the heterocyclic group is attached to the group represented by V, from 1 to 3 are additional nitrogen and/or oxygen and/or sulphur hetero-atoms, and the remainder are carbon atoms, said group being unsubstituted or being substituted by at least one substituent selected from oxygen atoms and the groups and atoms represented by $R^a$;

a group of formula (II):

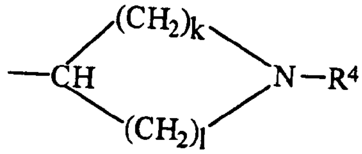

(II)

or

said group of formula (II) in which the ring is condensed with one or two benzene rings

or

a group of formula:

$$-(Ar)_j-(CH_2)_i-NR^4R^5 \qquad (III)$$

where

k and l are the same or different and each represents 0 or an integer from 1 to 4, provided that (k + l) is at least 1;

i is 0 or an integer from 1 to 4;

j is 0 or 1;

Ar represents an aryl group as defined below or an aromatic heterocyclic group which has from 5 to 7 ring atoms, of which from 1 to 3 are hetero-atoms, from 0 to 3 of said hetero-atoms being nitrogen atoms and 0 or 1 of said hetero-atoms being an oxygen or a sulphur atom, said aromatic heterocyclic group being unsubstituted or being substituted by at least one substituent selected from the groups and atoms represented by $R^a$;

$R^4$ and $R^5$ are the same or different and each represents:

a hydrogen atom;

any of the groups and atoms represented by $R^a$;

an aryl group, as defined below;

an aralkyl group, as defined below;

an arylcarbonyl group, in which the aryl part is as defined below; and

a heterocyclic group having from 3 to 8 ring atoms of which from 1 to 4 are nitrogen and/or oxygen and/or sulphur hetero-atoms, said group being unsubstituted or being substituted by at least one substituent selected from the groups and atoms represented by $R^a$;

said aryl groups are carbocyclic aromatic groups which have from 6 to 14 ring carbon atoms and which are unsubstituted or which are substituted by at least one substituent selected from the groups and atoms represented by $R^a$;

said aralkyl group is an alkyl group having from 1 to 6 carbon atoms which group is substituted by from 1 to 3 aryl groups as defined above;

$R^a$ represents:

an alkyl group having from 1 to 6 carbon atoms;

an aryl group which is a carbocyclic aromatic group having from 6 to 14 ring carbon atoms, which group is unsubstituted or is substituted by at least one substituent selected from the groups and atoms represented by $R^c$;

a cycloalkyl group having from 3 to 14 ring carbon atoms in one or more rings;

a cycloalkylalkyl group in which the cycloalkyl part has from 3 to 14 ring carbon atoms in one or more rings, and the alkyl part has from 1 to 4 carbon atoms;

a heterocyclic group having from 3 to 14 ring atoms of which from 1 to 4 are nitrogen and/or oxygen and/or sulphur hetero-atoms, said group being unsubstituted or being substituted by at least one substituent selected from the groups and atoms represented by $R^b$;

an aralkyl group in which the alkyl part has from 1 to 6 carbon atoms and the aryl part is a carbocyclic aromatic group having from 6 to 14 ring carbon atoms and being unsubstituted or being substituted by at least one substituent selected from the groups and atoms represented by $R^c$;

a halogen atom;

an amino group;

an alkylamino group in which the alkyl part has from 1 to 6 carbon atoms;

a dialkylamino group in which each alkyl part is an alkyl group having from 1 to 6 carbon atoms;

a mono- or di- arylamino group in which the or each aryl part is a carbocyclic aromatic group which has from 6 to 14 ring carbon atoms and which is unsubstituted or substituted by at least one substituent selected from the groups and atoms represented by $R^c$;

an aminoalkyl group in which the alkyl part has from 1 to 6 carbon atoms and in which the amino part is unsubstituted or is substituted by a single acyl group represented by $R^d$;

an alkylaminoalkyl group in which each alkyl part is an alkyl group having from 1 to 6 carbon atoms;

an alkynylaminoalkyl group in which the alkyl part has from 1 to 6 carbon atoms and the alkynyl part has from 2 to 6 carbon atoms;

a nitro group;

a cyano group;

a sulphonyl group;

an alkylsulphonyl group in which the alkyl part has from 1 to 6 carbon atoms;

a haloalkylsulphonyl group in which the alkyl part has from 1 to 6 carbon atoms;

an alkanoyl group having from 1 to 6 carbon atoms;

an arylcarbonyl group in which the aryl part is a carbocyclic aromatic group which has from 6 to 14 ring carbon atoms and which is unsubstituted or is substituted by at least one substituent selected from the groups and atoms represented by $R^c$;

an arylalkanoyl group in which the aryl part is a carbocyclic aromatic group which has from 6 to 14 ring carbon atoms and which is unsubstituted or is substituted by at least one substituent selected from the groups and atoms represented by $R^c$, and the alkanoyl part has from 2 to 6 carbon atoms;

an alkoxy group having from 1 to 6 carbon atoms;

an alkoxycarbonyl group having from 2 to 7 carbon atoms;

a haloalkyl group having from 1 to 6 carbon atoms;

and

in the case of groups attached to nitrogen atoms, a cyanoamino group;

$R^b$ represents:

an alkyl group having from 1 to 6 carbon atoms;

an aryl group which is a carbocyclic aromatic group having from 6 to 14 ring carbon atoms and which is unsubstituted or is substituted by at least one substituent selected from the groups and atoms represented by $R^c$;

an aralkyl group in which the alkyl part has from 1 to 6 carbon atoms and the aryl part is a carbocyclic aromatic group which has from 6 to 14 ring carbon atoms and which is unsubstituted or is substituted by at least one substituent selected from the groups and atoms represented by $R^c$;

a halogen atom;

an amino group;

an alkylamino group in which the alkyl part has from 1 to 6 carbon atoms;

a dialkylamino group in which each alkyl part is an alkyl group having from 1 to 6 carbon atoms;

an arylamino group in which the aryl part is a carbocyclic aromatic group which has from 6 to 14 ring carbon atoms and which is unsubstituted or is substituted by at least one substituent selected from the groups and atoms represented by $R^c$;

an aminoalkyl group in which the alkyl part has from 1 to 6 carbon atoms;

an alkylaminoalkyl group in which each alkyl part is an alkyl group having from 1 to 6 carbon atoms;

a nitro group;

a cyano group;

an alkanoyl group having from 1 to 6 carbon atoms;

an arylcarbonyl group in which the aryl part is a carbocyclic aromatic group which has from 6 to 14 ring carbon atoms and which is unsubstituted or is substituted by at least one substituent selected from the groups and atoms represented by $R^c$;

an arylalkanoyl group in which the aryl part is a carbocyclic aromatic group which has from 6 to 14 ring carbon atoms and which is unsubstituted or is substituted by at least one substituent selected from the groups and atoms represented by $R^c$;

an alkoxy group having from 1 to 6 carbon atoms;

an alkoxycarbonyl group having from 2 to 7 carbon atoms; or

a haloalkyl group having from 1 to 6 carbon atoms;

$R^c$ represents:

an alkyl group having from 1 to 6 carbon atoms;

an aryl group which is an unsubstituted carbocyclic aromatic group having from 6 to 10 ring carbon atoms; an aralkyl group in which the alkyl part has from 1 to 6 carbon atoms and the aryl part is an unsubstituted carbocyclic aromatic group having from 6 to 10 ring carbon atoms;

a halogen atom;

an amino group;

an alkylamino group in which the alkyl part has from 1 to 6 carbon atoms;

a dialkylamino group in which each alkyl part is an alkyl group having from 1 to 6 carbon atoms;

an arylamino group in which the aryl part is an unsubstituted carbocyclic aromatic group having from 6 to 10 ring carbon atoms;

an aminoalkyl group in which the alkyl part has from 1 to 6 carbon atoms;

an alkylaminoalkyl group in which each alkyl part is an alkyl group having from 1 to 6 carbon atoms;

a nitro group;

a cyano group;

an alkanoyl group having from 1 to 6 carbon atoms;

an arylcarbonyl group in which the aryl part is an unsubstituted carbocyclic aromatic group having from 6 to 10 ring carbon atoms;

an alkoxy group having from 1 to 6 carbon atoms; an alkoxycarbonyl group having from 2 to 7 carbon atoms; or

a haloalkyl group having from 1 to 6 carbon atoms;

$R^d$ represents:

an alkanoyl group having from 1 to 6 carbon atoms;

an alkenoyl or alkynoyl group having from 3 to 6 carbon atoms;

an aromatic acyl group in which the aromatic part is a carbocyclic aromatic group which has from 6 to 14 ring carbon atoms and which is unsubstituted or is substituted by at least one substituent selected from the groups and atoms represented by $R^c$;

a heterocyclic acyl group in which the heterocyclic part has from 3 to 8 ring atoms, of which from 1 to 3 are nitrogen and/or oxygen and/or sulphur hetero-atoms, said heterocylic group being unsubstituted or being substituted by at least one substituent selected from the groups and atoms represented by $R^c$;

an alkoxycarbonyl group in which the alkoxy part has from 1 to 6 carbon atoms;

an aryloxycarbonyl group in which the aryl part has from 6 to 10 ring carbon atoms and is unsubstituted or is substituted by at least one substituent selected from the groups and atoms represented by $R^c$; or

an aralkyloxycarbonyl group in which the aralkyl part is an alkyl group having from 1 to 6 carbon atoms which is substituted by from 1 to 3 aryl groups having from 6 to 10 ring carbon atoms and being unsubstituted or substituted by at least one substituent selected from the groups and atoms represented by $R^c$;

and pharmaceutically acceptable salts thereof.

**2.** A compound according to Claim 1, in which $R^1$ represents a hydrogen atom, an alkyl group having from 1 to 6 carbon atoms, a cycloalkyl group having from 3 to 10 ring carbon atoms, an aryl group as defined in Claim 1, an aralkyl group as defined in Claim 1, an alkanoyl group as defined in Claim 1, an arylcarbonyl group as defined in Claim 1, an alkoxy group having from 1 to 6 carbon atoms, an alkoxycarbonyl group having from 1 to 6 carbon atoms or a haloalkyl group having from 1 to 6 carbon atoms.

**3.** A compound according to Claim 1, in which $R^2$ represents a hydrogen atom, an alkyl group having from 1 to 6 carbon atoms, a cycloalkyl group having from 3 to 10 ring carbon atoms, an aryl group as defined in Claim 1, an aralkyl group as defined in Claim 1, a halogen atom, an amino group, a mono- or di- alkylamino group as defined in Claim 1, an alkylaminoalkyl groups in which each alkyl part has from 1 to 4 carbon atoms, an alkynylaminoalkyl group in which the alkynyl part has from 2 to 4 carbon atoms and the alkyl part has from 1 to 4 carbon atoms, an arylamino group as defined in Claim 1, a nitro group, a cyano group, a sulphonyl group, an alkylsulphonyl group as defined in Claim 1, a haloalkylsulphonyl group as defined in Claim 1, an alkanoyl group as defined in Claim 1, an arylcarbonyl group as defined in Claim 1, an alkoxy group having from 1 to 6 carbon atoms, an alkoxycarbonyl as defined in Claim 1 or a haloalkyl group having from 1 to 6 carbon atoms.

**4.** A compound according to Claim 1, in which U represents a group of formula: $-CO-$ or $-CH(OR^3)-$, in which $R^3$ represents a hydrogen atom, an alkanoyl group as defined in Claim 1, an arylcarbonyl group as defined in Claim 1, an alkylsulphonyl group having from 1 to 6 carbon atoms or an arylsulphonyl group as defined in Claim 1.

**5.** A compound according to Claim 1, in which $R^3$ represents a hydrogen atom or a hydroxy protecting group for a pro-drug.

**6.** A compound according to Claim 1, in which V represents a group of formula: $-(CH=CH)_m-(CH_2)_n-$ (in which $\underline{m}$ is 0, 1 or 2, and $\underline{n}$ is 0 or an integer of from 1 to 7).

**7.** A compound according to Claim 1, in which W represents a nitrogen-containing heterocyclic group as defined in Claim 1, or a group of formula (II) as defined in Claim 1, in which $\underline{k}$ and $\underline{l}$ are the same or different and each is 0 or an integer of from 1 to 4; $R^4$ represents a hydrogen atom, an alkyl group having from 1 to 6 carbon atoms, a cycloalkyl group as defined in Claim 1, an aryl group as defined in Claim 1, an aralkyl group as defined in Claim 1, an alkanoyl group as defined in Claim 1, an arylcarbonyl group as defined in Claim 1, an alkoxy group having from 1 to 6 carbon atoms, an alkoxycarbonyl group having from 2 to 7 carbon atoms or a haloalkyl group having from 1 to 6 carbon atoms.

**8.** A compound according to Claim 1, in which W represents a group of formula: $-NR^4R^5$, in which $R^4$ and $R^5$ are the same or different and each represents a hydrogen atom, an alkyl group having from 1 to 6 carbon atoms, a cycloalkyl group as defined in Claim 1, an aryl group as defined in Claim 1, an aralkyl group as defined in Claim 1, an alkanoyl group as defined in Claim 1, an arylcarbonyl group as defined in Claim 1, an alkoxy group having from 1 to 6 carbon atoms, an alkoxycarbonyl group having from 2 to 7 carbon atoms or a haloalkyl group having from 1 to 6 carbon atoms, or a saturated or unsaturated heterocyclic group containing a nitrogen atom or atoms as defined in Claim 1.

**9.** A compound according to Claim 1, in which $R^1$ and $R^2$ are the same or different from each other and each represents:

a hydrogen atom;
an alkyl group having from 1 to 4 carbon atoms;
a cycloalkyl group having from 3 to 10 ring carbon atoms;
a halogen atom;
a sulphonyl group;
an amino group;
a mono- or di- alkylamino group in which the or each alkyl part has from 1 to 4 carbon atoms;
an alkylaminoalkyl group in which each alkyl part has from 1 to 4 carbon atoms;
an alkynylaminoalkyl group in which the alkynyl part has from 2 to 4 carbon atoms and the alkyl part has from 1 to 4 carbon atoms;
a nitro group, a cyano group;
an alkanoyl group having from 1 to 4 carbon atoms;
an alkoxy group having from 1 to 4 carbon atoms;
an alkoxycarbonyl group having from 2 to 5 carbon atoms;
a haloalkyl group having from 1 to 4 carbon atoms;
an aryl group having from 6 to 10 ring carbon atoms, which group may be unsubstituted or may be substituted by 1 or 2 of substituents A:

substituents A are selected from alkyl groups having from 1 to 4 carbon atoms, cycloalkyl groups having from 3 to 6 ring carbon atoms, halogen atoms, mono- or di- alkylamino groups in which the or each alkyl part has from 1 to 4 carbon atoms, alkylsulphonyl groups having from 1 to 4 carbon atoms, haloalkylsulphonyl groups having from 1 to 4 carbon atoms, alkanoyl groups having from 2 to 4 carbon atoms, alkoxy groups having from 1 to 4 carbon atoms, alkoxycarbonyl groups having from 2 to 5 carbon atoms, haloalkyl groups having from 1 to 4 carbon atoms, halogen atoms, amino groups, nitro groups, cyano groups and sulphonyl groups;

an aralkyl group having from 1 to 3 carbon atoms in the alkyl part and from 6 to 10 ring carbon atoms in the aryl part, said group being unsubstituted or substituted by at least one of substituents A;
a mono- or di- arylamino group, in which the or each aryl part has from 6 to 10 ring carbon atoms and is unsubstituted or substituted by at least one of substituents A;
an arylcarbonyl group, in which the aryl part has from 6 to 10 ring carbon atoms and is unsubstituted or substituted by at least one of substituents A; or
an arylalkanoyl group having from 2 to 4 carbon atoms in the alkanoyl part and from 6 to 10 ring carbon atoms in the aryl part, said group being unsubstituted or substituted by at least one of substituents A;

**10.** A compound according to Claim 1, in which U represents a group of formula: -CO- or $-CH(OR^3)-$, in which $R^3$ represents:

a hydrogen atom;
an alkanoyl group having from 2 to 4 carbon atoms;
an alkylsulphonyl group having from 1 to 4 carbon atoms;
an arylcarbonyl group having from 6 to 10 ring carbon atoms in the aryl part, which group may be unsubstituted or may be substituted by 1 or 2 of substituents B:

substituents B are selected from alkyl groups having from 1 to 4 carbon atoms, alkoxy groups having from 1 to 4 carbon atoms, halogen atoms and nitro groups;

an arylsulphonyl group, in which the aryl part has from 6 to 10 ring carbon atoms and is unsubstituted or substituted by at least one of substituents B; or
a hydroxy-protecting group for a pro-drug.

**11.** A compound according to Claim 1, in which V represents a group of formula:

$$-(CH=CH)_m-(CH_2)_n-$$

in which $\underline{m}$ is 0, 1 or 2, and $\underline{n}$ is 0 or an integer of from 1 to 5, provided that ($\underline{m}$ + $\underline{n}$) > 0.

**12.** A compound according to Claim 1, in which W represents a partially or completely saturated nitrogen-containing heterocyclic group having from 5 to 10 ring atoms including a nitrogen atom through which it is attached to the group represented by V.

**13.** A compound according to Claim 1, in which V represents a 1-piperidinyl, 1-piperazinyl, 1-morpholinyl, 1-tetrahydroquinolyl or 2-tetrahydroisoquinolyl group.

**14.** A compound according to Claim 1, in which W represents a group of formula (II) as defined in Claim 1, in which $\underline{k}$ and $\underline{l}$ are the same or different from each other and each is an integer from 1 to 3, and the ring nitrogen atom is substituted with $R^4$ as defined in Claim 1.

**15.** A compound according to Claim 1, in which W represents a group of formula (II) as defined in Claim 1 having a 5- or 6-membered ring, in which an ethylene group in the 5- or 6-membered ring is condensed with 1 or 2 benzene rings, and the ring nitrogen atom is substituted with $R^4$ as defined in Claim 1.

**16.** A compound according to Claim 14, in which $R^4$ represents:

a hydrogen atom;

an alkyl group having from 1 to 4 carbon atoms;

a cycloalkyl group having from 3 to 10 ring carbon atoms;

an alkanoyl group having from 1 to 4 carbon atoms;

an alkoxy group having from 1 to 4 carbon atoms;

an alkoxycarbonyl group having from 2 to 5 carbon atoms;

an aryl group which has from 6 to 10 ring carbon atoms and which is unsubstituted or is substituted by 1 or 2 of substituents C:

substituents C are selected from alkyl groups having from 1 to 4 carbon atoms, halogen atoms, haloalkyl groups having from 1 to 4 carbon atoms, alkanoyl groups having 2 or 3 carbon atoms, alkoxy groups having from 1 to 4 carbon atoms, mono- or di- alkylamino groups in which the or each alkyl part has from 1 to 4 carbon atoms, nitro groups and cyano groups;

an arylalkanoyl group having from 2 to 4 carbon atoms in the alkanoyl part and from 6 to 10 ring carbon atoms in the aryl part, said group being unsubstituted or substituted by at least one of substituents C;

an arylcarbonyl group, in which the aryl part has from 6 to 10 ring carbon atoms and is unsubstituted or substituted by at least one of substituents C;

an aralkyl group having from 1 to 3 carbon atoms in the alkyl part and from 6 to 10 ring carbon atoms in the aryl part, said group being unsubstituted or substituted by at least one of substituents C;

a nitrogen-containing heterocyclic group having from 5 to 10 ring atoms, said group being unsubstituted or substituted by at least one of substituents C;

and a nitrogen-containing heterocyclic alkyl group, in which the heterocyclic part has from 5 to 10 ring atoms, said group being unsubstituted or substituted by at least one of substituents C, and the alkyl part has from 1 to 4 carbon atoms.

**17.** A compound according to Claim 15, in which $R^4$ represents:

a hydrogen atom;

an alkyl group having from 1 to 4 carbon atoms;

a cycloalkyl group having from 3 to 10 ring carbon atoms;

an alkanoyl group having from 1 to 4 carbon atoms;

an alkoxy group having from 1 to 4 carbon atoms;

an alkoxycarbonyl group having from 2 to 5 carbon atoms;

an aryl group having from 6 to 10 ring carbon atoms and which is unsubstituted or is substituted by 1 or 2 of substituents C:

substituents C are selected from alkyl groups having from 1 to 4 carbon atoms, halogen atoms, haloalkyl groups having from 1 to 4 carbon atoms, alkanoyl groups having 2 or 3 carbon atoms, alkoxy groups having from 1 to 4 carbon atoms, mono- or di- alkylamino groups in which the or each alkyl part has from 1 to 4 carbon atoms, nitro groups and cyano groups;

an arylalkanoyl group having from 2 to 4 carbon atoms in the alkanoyl part and from 6 to 10 ring carbon atoms in the aryl part, said group being unsubstituted or substituted by at least one of substituents

C;

an arylcarbonyl group, in which the aryl part has from 6 to 10 ring carbon atoms and is unsubstituted or substituted by at least one of substituents C;

an aralkyl group having from 1 to 3 carbon atoms in the alkyl part and from 6 to 10 ring carbon atoms in the aryl part, said group being unsubstituted or substituted by at least one of substituents C;

a nitrogen-containing heterocyclic group having from 5 to 10 ring atoms, said group being unsubstituted or substituted by at least one of substituents C; or

a nitrogen-containing heterocyclic alkyl group, in which the heterocyclic part has from 5 to 10 ring atoms, said group being unsubstituted or substituted by at least one of substituents C, and the alkyl part has from 1 to 4 carbon atoms.

**18.** A compound according to Claim 1, in which W represents a group of formula $-NR^5R^6$, in which $R^5$ and $R^6$ may be the same or different from each other and each represents:

a hydrogen atom;

an alkyl group having from 1 to 4 carbon atoms;

a cycloalkyl group having from 3 to 10 ring carbon atoms;

an alkanoyl group having from 1 to 4 carbon atoms;

an alkoxy group having from 1 to 4 carbon atoms;

an aryl group having from 6 to 10 ring carbon atoms which is unsubstituted or is substituted by 1 or 2 of substituents D:

substituents D are selected from alkyl groups having from 1 to 4 carbon atoms, halogen atoms, haloalkyl groups having from 1 to 4 carbon atoms, alkanoyl groups having 2 or 3 carbon atoms, alkoxy groups having from 1 to 4 carbon atoms, alkoxylalkyl groups in which the alkyl and alkoxy parts each have from 1 to 4 carbon atoms, nitro groups and cyano groups;

an arylalkanoyl group having from 2 to 4 carbon atoms in the alkanoyl part and from 6 to 10 ring carbon atoms in the aryl part, said group being unsubstituted or substituted by at least one of substituents D;

an arylcarbonyl group, in which the aryl part has from 6 to 10 ring carbon atoms and is unsubstituted or substituted by at least one of substituents D;

an aralkyl group having from 1 to 3 carbon atoms in the alkyl part and from 6 to 10 ring carbon atoms in the aryl part, said group being unsubstituted or substituted by at least one of substituents D;

a nitrogen-containing heterocyclic group having from 5 to 10 ring atoms, said group being unsubstituted or substituted by at least one of substituents D;

a nitrogen-containing heterocyclic alkyl group, in which the heterocyclic part has from 5 to 10 ring atoms, said group being unsubstituted or substituted by at least one of substituents D, and the alkyl part has from 1 to 4 carbon atoms;

an aryloxy group, in which the aryl part has from 6 to 10 ring carbon atoms and is unsubstituted or substituted by at least one of substituents D; or

a saturated or unsaturated heterocyclic group having from 5 to 10 ring atoms, such as a piperazinyl, quinolyl, thienyl or furyl group.

**19.** A compound according to Claim 1, in which $R^1$ represents:

a hydrogen atom;

a methyl, ethyl, isopropyl or isobutyl group;

a cyclopropyl, cyclobutyl, cyclopentyl or cyclohexyl group;

a trifluoromethyl or 2,2,2-trifluoroethyl group;

an acetyl or propionyl group;

a methoxy, ethoxy or isopropyloxy group;

a methoxycarbonyl or ethoxycarbonyl group;

a phenyl group which is unsubstituted or is substituted by 1 or 2 of substituents E:

substituents E are selected from methyl, ethyl, isopropyl, cyclopropyl, chlorine, fluorine, bromine, trifluoromethyl, methylamino, dimethylamino, acetyl, propionyl, methoxy, ethoxy, isopropoxy, nitro and cyano groups;

a benzyl group which is unsubstituted or is substituted by 1 or 2 of substituents E;

a benzoyl group which is unsubstituted or is substituted by 1 or 2 of substituents E;

or a phenylacetyl group which is unsubstituted or is substituted by 1 or 2 of substituents E;

**20.** A compound according to Claim 1, in which $R^2$ represents:

a hydrogen atom;
a methyl, ethyl, isopropyl or isobutyl group;
a cyclopropyl, cyclobutyl, cyclopentyl or cyclohexyl group;
a fluorine, chlorine or bromine atom;
a sulphonyl group;
a trifluoromethyl or 2,2,2-trifluoroethyl group;
a methylamino, ethylamino, isopropylamino, dimethylamino, diethylamino or diisopropylamino group;
an alkylaminoalkyl group in which each alkyl part has from 1 to 3 carbon atoms;
an alkynylaminoalkyl group in which the alkynyl part has 3 or 4 carbon atoms and the alkyl part has from 1 to 3 carbon atoms;
an acetyl or propionyl group;
a methoxy, ethoxy or isopropyloxy group;
a methoxycarbonyl or ethoxycarbonyl group;
an amino group;
a nitro group;
a cyano group;
a phenyl group which is unsubstituted or is substituted by 1 or 2 of substituents F:
substituents F are selected from methyl, ethyl, isopropyl, cyclopropyl, chlorine, fluorine, bromine, trifluoromethyl, methylamino, dimethylamino, acetyl, propionyl, methoxy, ethoxy, isopropoxy, nitro and cyano groups;
or a benzyl group which is unsubstituted or is substituted by 1 or 2 of substituents F.

**21.** A compound according to Claim 1, in which U represents a group of formula: -CO- or -CH($OR^3$)-, in which $R^3$ represents:
a hydrogen atom;
an acetyl, propionyl, methylsulphonyl, ethylsulphonyl, propylsulphonyl, trimethylsilyl, triethylsilyl, methoxycarbonyl or ethoxycarbonyl group;
a benzoyl group which is unsubstituted or is substituted by 1 or 2 of substituents G:
substituents G are selected from methyl, ethyl, fluorine, chlorine, bromine, acetyl, methoxy, ethoxy, nitro and cyano groups;
or a phenylsulphonyl group which is unsubstituted or is substituted by 1 or 2 of substituents G.

**22.** A compound according to Claim 1, in which V represents a group of formula: $-(CH=CH)_m-(CH_2)_n-$ (in which $\underline{m}$ is 0 or 1, and $\underline{n}$ is 0 or an integer from 1 to 3, provided that ($\underline{m}$ + $\underline{n}$) > 0.

**23.** A compound according to Claim 1, in which W represents a partially or completely saturated nitrogen-containing heterocyclic group having from 6 to 10 ring atoms including a nitrogen atom through which it is attached to the group represented by V.

**24.** A compound according to Claim 1, in which W represents a 2-tetrahydroisoquinolyl group.

**25.** A compound according to Claim 1, in which W represents a group of formula (II) as defined in Claim 1, in which $\underline{k}$ and $\underline{l}$ are the same or different from each other and each is 1 or 2, and $R^4$ is as defined in Claim 1, such as a pyrrolidinyl or piperidyl which is unsubstituted or substituted by a group $R^4$.

**26.** A compound according to Claim 1, in which W represents a group of formula (II) having a 6-membered ring formed by a cyclic alkylene group and a nitrogen atom, in which the ethylene group in the 6-membered ring is condensed with 1 or 2 benzene rings, such as a 4-(1-tetrahydroquinolyl) group which is unsubstituted or substituted by a group $R^4$.

**27.** A compound according to Claim 25, in which $R^4$ represents:
a hydrogen atom;
a methyl, ethyl or isopropyl group;
an adamantyl group;
an acetyl, propionyl or butyryl group;
a methoxy or ethoxy group;
a methoxycarbonyl or ethoxycarbonyl group;
a phenyl group which is unsubstituted or is substituted by 1 or 2 of substituents H:

substituents H are selected from methyl, ethyl, fluorine, chlorine, bromine, acetyl, methoxy, ethoxy, dimethylamino, nitro and cyano groups;

an aralkyl group having from 1 to 3 carbon atoms in the alkyl part and from 6 to 10 ring carbon atoms in the aryl part, said group being unsubstituted or substituted by at least one of substituents H;

a benzoyl group which is unsubstituted or is substituted by 1 or 2 of substituents H;

a 2-thienyl, 3-thienyl, 2-furyl, 3-furyl, 2-indolinyl, 2-imidazolyl or 2-(4,5-dihydro)imidazolyl group which is unsubstituted or is substituted by 1 or 2 of substituents H; or

a 2-thenyl, 3-thenyl, 2-furylmethyl, 3-furylmethyl, 2-imidazolylmethyl or 4,5-dihydroimidazol-2-ylmethyl group which is unsubstituted or is substituted by 1 or 2 of substituents H.

**28.** A compound according to Claim 26, in which $R^4$ represents:

a hydrogen atom;

a methyl, ethyl or isopropyl group;

an adamantyl group;

an acetyl, propionyl or butyryl group;

a methoxy or ethoxy group;

a methoxycarbonyl or ethoxycarbonyl group;

a phenyl group which is unsubstituted or is substituted by 1 or 2 of substituents H:

substituents H are selected from methyl, ethyl, fluorine, chlorine, bromine, acetyl, methoxy, ethoxy, dimethylamino, nitro and cyano groups;

an aralkyl group having from 1 to 3 carbon atoms in the alkyl part and from 6 to 10 ring carbon atoms in the aryl part, said group being unsubstituted or substituted by at least one of substituents H;

a benzoyl group which is unsubstituted or is substituted by 1 or 2 of substituents H;

a 2-thienyl, 3-thienyl, 2-furyl, 3-furyl, 2-indolinyl, 2-imidazolyl or 2-(4,5-dihydro)imidazolyl group which is unsubstituted or is substituted by 1 or 2 of substituents H; or

a 2-thenyl, 3-thenyl, 2-furylmethyl, 3-furylmethyl, 2-imidazolylmethyl or 4,5-dihydroimidazol-2-ylmethyl group which is unsubstituted or is substituted by 1 or 2 of substituents H.

**29.** A compound according to Claim 1, in which W represents a group of formula $-NR^5R^6$, in which $R^5$ and $R^6$ may be the same or different from each other and each represents:

a hydrogen atom;

a methyl, ethyl or isopropyl group;

an adamantyl group;

an acetyl or propionyl group;

a methoxy or ethoxy group;

a methoxycarbonyl or ethoxycarbonyl group;

a phenyl group which is unsubstituted or is substituted by 1 or 2 of substituents I:

substituents I are selected from methyl, ethyl, fluorine, chlorine, bromine, acetyl, methoxy, ethoxy, nitro, cyano and dimethylamino groups;

an aralkyl group having from 1 to 3 carbon atoms in the alkyl part and from 6 to 10 ring carbon atoms in the aryl part, said group being unsubstituted or substituted by at least one of substituents I;

a benzoyl group which is unsubstituted or is substituted by 1 or 2 of substituents I;

a 2-thienyl, 3-thienyl, 2-furyl, 3-furyl, 2-indolinyl, 2-imidazolyl or 2-(4,5-dihydro)imidazolyl group which is unsubstituted or is substituted by 1 or 2 of substituents I; or

a 2-thenyl, 3-thenyl, 2-furylmethyl, 3-furylmethyl, 2-imidazolylmethyl or 4,5-dihydroimidazol-2-ylmethyl group which is unsubstituted or is substituted by 1 or 2 of substituents I.

**30.** A compound according to Claim 1, in which $R^1$ represents:

a hydrogen atom;

a methyl, ethyl, isopropyl or isobutyl group;

an adamantyl group;

a trifluoromethyl, or 2,2,2-trifluoroethyl group;

a phenyl group;

a phenoxy group;

or a benzyl group.

**31.** A compound according to Claim 1, in which $R^2$ represents:

a hydrogen atom;

a methyl, ethyl or isopropyl group;
a cyclopropyl group;
an acetyl group;
a methoxy or ethoxy group;
a fluorine, chlorine or bromine atom;
a trifluoromethyl or 2,2,2-trifluoroethyl group;
an amino group;
a methylamino or dimethylamino group;
a methylaminomethyl or ethylaminomethyl group;
a propargylaminomethyl group or a [(2-butynyl)amino]methyl group;
a nitro group;
a cyano group;
a phenyl group or a benzyl group.

**32.** A compound according to Claim 1, in which U represents a group of formula: -CO- or -CH($OR^3$)-, in which $R^3$ represents a hydrogen atom, an acetyl group, a propionyl group or a benzoyl group.

**33.** A compound according to Claim 1, in which V represents a vinyl group, ethylene or a trimethylene group.

**34.** A compound according to Claim 1, in which W represents a 3-pyrrolidinyl or 4-piperidinyl group having a group $R^4$ at the 1-position, in which $R^4$ represents:
a hydrogen atom;
a methyl, ethyl or isopropyl group;
a cyclopropyl or adamantyl group;
a phenyl group which is unsubstituted or is substituted by 1 or 2 of substituents J:
substituents J are selected from methyl, fluorine, chlorine, acetyl, methoxy, ethoxy, nitro, cyano and dimethylamino groups at the 2-to 5-position thereof;
a benzyl, 2-phenylethyl, 3-propylphenyl or 4-butylphenyl group which is unsubstituted or is substituted by 1 or 2 of substituents J;
a benzoyl group which is unsubstituted or is substituted by 14 or 2 of substituents J;
a thienyl group which is unsubstituted or is substituted by 1 or 2 substituents selected from methyl, fluorine, chlorine and methoxy groups; or
a 2-indolinyl group which is unsubstituted or is substituted by 1 or 2 substituents selected from methyl, fluorine, chlorine and methoxy groups.

**35.** A compound according to Claim 1, in which W represents:
a methylamino, ethylamino or propylamino group;
a dimethylamino or diisopropylamino group;
an anilino group which is unsubstituted or is substituted by 1 or 2 of substituents K:
substituents K are selected from methyl, fluorine, chlorine, acetyl, methoxy, ethoxy, nitro, cyano and dimethylamino groups at the 2-to 5-positions thereof;
a phenylmethylamino group which is unsubstituted or is substituted by 1 or 2 of substituents K;
a benzylamino group which is unsubstituted or is substituted by 1 or 2 of substituents K;
a benzylmethylamino group which is unsubstituted or is substituted by 1 or 2 of substituents K;
a benzylethylamino group which is unsubstituted or is substituted by 1 or 2 of substituents K;
a benzylisopropylamino group which is unsubstituted or is substituted by 1 or 2 of substituents K;
or a 2-thenyl group which is unsubstituted or is substituted by 1 or 2 substituents selected from methyl, fluorine, chlorine and methoxy groups.

**36.** A compound according to Claim 1, in which $R^1$ represents a hydrogen atom, a methyl, ethyl, isopropyl or isobutyl group, or a trifluoromethyl group.

**37.** A compound according to Claim 1, in which $R^2$ represents a hydrogen atom, a methyl or ethyl group, a fluorine or chlorine atom, a methoxy or ethoxyamino group, a dimethylamino group, or a trifluoromethyl or 2,2,2-trifluoroethyl group.

**38.** A compound according to Claim 1, in which U represents a -CO- group.

**39.** A compound according to Claim 1, in which V represents a vinyl group or an ethylene group.

**40.** A compound according to Claim 1, in which W represents a 1-benzyl-3-pyrrolidinyl, 1-phenylmethyl-3-pyrrolidinyl, 1-benzyl-4-piperidinyl or 1-phenylmethyl-4-piperidinyl group, and in which said benzyl or 1-phenylmethyl groups are unsubstituted or are substituted by 1 or 2 substituents selected from methyl, fluorine, chlorine, methoxy, dimethylamino and trifluoromethyl groups at the 2- to 5-positions thereof.

**41.** The following compounds according to Claim 1:
1-methyl-3-[3-(1-benzyl-4-piperidyl)acryloyl]indole;
1-methyl-3-[3-(1-benzyl-4-piperidyl)propionyl]indole;
1-methyl-3-[3-(1-benzyl-4-piperidyl)propionyl]indole hydrochloride;
1-methyl-3-[3-(1-benzyl-4-piperidyl)propionyl]indole oxalate;
1-methyl-3-[3-(1-benzyl-4-piperidyl)acryloyl]-5-chloroindole;
1-methyl-3-[3-(1-benzyl-4-piperidyl)propionyl]-5-chloroindole;
1-methyl-3-[3-(1-benzyl-4-piperidyl)propionyl]-6-chloroindole;
1-methyl-3-[3-(1-benzyl-4-piperidyl)acryloyl]-5-fluoroindole;
1-methyl-3-[3-(1-benzyl-4-piperidyl)propionyl]-5-fluoroindole;
1-methyl-3-{3-[1-(3-fluorobenzyl)-4-piperidyl]acryloyl}indole;
1-methyl-3-{3-[1-(3-fluorobenzyl)-4-piperidyl]propionyl}indole;
1-methyl-3-{3-[1-(3-chlorobenzyl)-4-piperidyl]propionyl}indole;
1-methyl-3-{3-[1-(3-chlorobenzyl)-4-piperidyl]propionyl}indole hydrochloride;
1-methyl-3-{3-[1-[2-(3-fluorophenyl)ethyl]-4-piperidyl]propionyl}indole;
1-methyl-3-[1-acetoxy-3-(1-benzyl-4-piperidyl)propyl] indole;
1-methyl-3-{3-[1-(3-chlorobenzyl)-4-piperidyl]acryloyl}-5-chloroindole;
1-methyl-3-{3-[1-(3-chlorobenzyl)-4-piperidyl] propionyl}-5-chloroindole hydrochloride;
1-methyl-3-{3-[1-(3-fluorobenzyl)-4-piperidyl]acryloyl}-5-chloroindole;
1-methyl-3-{3-[1-(3-fluorobenzyl)-4-piperidyl]propionyl}-5-chloroindole;
1-methyl-3-{3-[1-(3-fluorobenzyl)-4-piperidyl]acryloyl}-5-fluoroindole;
1-methyl-3-{3-[1-(3-fluorobenzyl)-4-piperidyl]propionyl}-5-fluoroindole;
1-methyl-3-{3-[1-(3-fluorobenzyl)-4-piperidyl]propionyl}-5-fluoroindole hydrochloride;
1-methyl-3-{3-[1-(3-fluorobenzyl)-4-piperidyl]propionyl}-5-fluoroindole oxalate;
1-methyl-3-{3-[1-(3-fluorobenzyl)-4-piperidylw]propionyl}-5-fluoroindole trifluoromethanesulphonate;
1-methyl-3-[3-(1-phenethyl-4-piperidyl]propionyl]-5-fluoroindole hydrochloride;
1-methyl-5-[3-(1-benzyl-4-piperidyl)propionyl]indole;
1-methyl-5-[N-methyl-N-Propargylaminomethyl]-3-[3-(1-benzyl-4-piperidyl)propionyl]indole;
1-methyl-3-[3-(1-benzyl-4-piperidyl)propionyl]indazole;
1-methyl-3-[3-(1-benzyl-4-piperidyl)propionyl]indazole hydrochloride;
1-methyl-3-[3-(1-benzyl-4-piperidyl)propionyl]indazole oxalate;
1-methyl-3-{3-[1-(2-chlorobenzyl)-4-piperidyl]propionyl}indazole;
1-methyl-3-{3-[1-(2-chlorobenzyl)-4-piperidyl]propionyl}indazole hydrochloride;
1-methyl-3-{3-[1-(3-chlorobenzyl)-4-piperidyl] propionyl}indazole;
1-methyl-3-{3-[1-(3-chlorobenzyl)-4-piperidyl]propionyl}indazole hydrochloride;
and pharmaceutically acceptable salts thereof.

**42.** A pharmaceutical composition for the treatment or prophylaxis of cerebral impairment, which composition comprises an effective amount of an acetylcholinesterase inhibitor in admixture with a pharmaceutically acceptable carrier or diluent, in which said acetylcholinesterase inhibitor is at least one compound of formula (I) or a pharmaceutically acceptable salt thereof, as claimed in any one of Claims 1 to 41.

**43.** The use of a compound of formula (I), as claimed in any one of Claims 1 to 41, or a pharmaceutically acceptable salt thereof, as a pharmaceutical.

**44.** Use of a compound, according to claim 43, as an acetylcholinesterase inhibitor.

**45.** Use of a compound of formula (I), as claimed in any one of Claims 1 to 41, in the preparation of a composition for the treatment or prophylaxis of dementia.

European Patent Office

# PARTIAL EUROPEAN SEARCH REPORT

which under Rule 45 of the European Patent Convention shall be considered, for the purposes of subsequent proceedings, as the European search report

Application Number
EP 93 30 2220
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | US-A-4 064 255 (MAR-PHA SOCIETÉ D'ETUDES ET D'EXPLOITATION DE MARQUES) 20 December 1977 *see general formula and examples* --- | 1-39,43 | C07D209/12<br>C07D409/14<br>C07D401/06<br>C07D231/56<br>A61K31/445<br>A61K31/40<br>A61K31/41 |
| X | EP-A-0 035 925 (PHARMINDUSTRIE) 16 September 1981 *see example 10, page 15,lines 16-17* --- | 1-39 | |
| X | CHEMICAL ABSTRACTS, vol. 88, 1978, Columbus, Ohio, US; abstract no. 22523s, 'cytotoxic and antineoplastic compounds' page 601 ; * abstract * & IZV. SIB. OTD. AKAD. NAUK SSSR, SER. KHIM. NAUK vol. 5, 1977, pages 148 - 152 --- | 1-39,43 | |
| | -/-- | | TECHNICAL FIELDS SEARCHED (Int. Cl. 5)<br>C07D<br>A61K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| MUNICH | 08 JUNE 1993 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent Office

# PARTIAL EUROPEAN SEARCH REPORT

Application Number

EP 93 30 2220
Page 2

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| X | CHEMICAL ABSTRACTS, vol. 100, 1984, Columbus, Ohio, US; abstract no. 209687g, 'synthesis of heterocyclic phthalimidoalkyl ketones' page 585 ; * abstract * & KHIM. GETEROTSIKL. SOEDIN. vol. 1, 1984, pages 71 - 81 *1h-ISOINDOLE-1,3(2h)-DIONE, 2-[6-(1h-INDAZOL-3-YL)-6-OXYHEXYL], CA RN = 89587-17-7 --- | 1-39 | |
| X | CHEMICAL ABSTRACTS, vol. 117, 1992, Columbus, Ohio, US; abstract no. 90076r, 'synthesis of some derivatives of 3-acetylindole of potential biological activity' page 761 ; * abstract * & BULL. FAC. PHARM. (CAIRO UNIV.) vol. 28, no. 2, 1990, pages 47 - 51 --- | 1-39,43 | TECHNICAL FIELDS SEARCHED (Int. Cl. 5) |
| X | WILLIAM J. HOULIHAN 'indoles part three' , INTERSCIENCE, JOHN WILEY AND SONS *see page 405, formula 350, page 477 and page 484* --- | 1-39 | |
| D,Y | PATENT ABSTRACTS OF JAPAN vol. 13, no. 283 (C-612)28 June 1989 & JP-A-10 79 151 ( SUGIMOTO HACHIRO ) 24 March 1989 * abstract * --- | 1-45 | |
| D,Y | PATENT ABSTRACTS OF JAPAN vol. 14, no. 435 (C-0760)18 September 1990 & JP-A-21 69 569 ( SUGIMOTO HACHIRO ) 29 June 1990 * abstract * ----- | 1-45 | |

EPO FORM 1503 03.82 (P04E10)

EP 93 30 2220
-C-

Reasons for limitation of the Search

Claim 1 of the present application covers a wide range of indole and indazole derivatives, represented by formula I.
The point of attachment of the group -U-V-W is undefined, and the definitions of W allow a large range of heterocyclic or linear substituents.
All of the examples actually prepared (Nos 1-147), with the exception of example 61 which does not fall within the scope of W in the claims, it not containing a N moiety, have the following structure

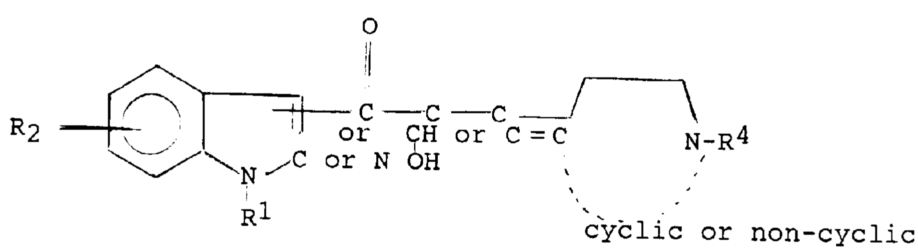

with the U-V-W attached in the 3- or 5-positions.

The application does not comply with the requirements of the Convention, in that it would appear that essential to the solution of the problem underlying the present application is a structural element as shown above, which is not an essential feature according to formula I of claim 1.

For these reasons, the Search was limited to compounds containing this essential structural element (Rule 45, EPC), as a meaningful Search was not possible for the other subject matter.